(19) Europäisches Patentamt / European Patent Office / Office européen des brevets

(11) **EP 2 688 962 B1**

(12) **EUROPÄISCHE PATENTSCHRIFT**

(45) Veröffentlichungstag und Bekanntmachung des Hinweises auf die Patenterteilung:
**01.08.2018 Patentblatt 2018/31**

(21) Anmeldenummer: **12710919.7**

(22) Anmeldetag: **21.03.2012**

(51) Int Cl.:
*C09C 1/62* (2006.01)     *C09C 1/66* (2006.01)
*C09C 1/00* (2006.01)     *A61K 8/02* (2006.01)
*A61K 8/19* (2006.01)     *A61K 8/81* (2006.01)
*A61Q 1/04* (2006.01)     *A61Q 1/10* (2006.01)
*A61Q 1/12* (2006.01)     *A61Q 19/00* (2006.01)
*A61Q 1/06* (2006.01)     *A61Q 19/10* (2006.01)
*A61Q 3/02* (2006.01)     *A61Q 5/06* (2006.01)
*C08K 3/08* (2006.01)     *C08K 9/02* (2006.01)
*C08K 9/10* (2006.01)     *C09D 7/62* (2018.01)
*C09D 7/40* (2018.01)

(86) Internationale Anmeldenummer:
**PCT/EP2012/054961**

(87) Internationale Veröffentlichungsnummer:
**WO 2012/130680 (04.10.2012 Gazette 2012/40)**

(54) **KUPFERHALTIGE METALLPIGMENTE MIT METALLOXIDSCHICHT UND KUNSTSTOFFSCHICHT, VERFAHREN ZU DEREN HERSTELLUNG, BESCHICHTUNGSMITTEL UND BESCHICHTETER GEGENSTAND**

COPPER-CONTAINING METAL PIGMENTS COMPRISING A METAL OXIDE LAYER AND A PLASTIC LAYER, METHOD FOR THE PRODUCTION THEREOF, COATING AGENT AND COATED OBJECT

PIGMENTS MÉTALLIQUES CONTENANT DU CUIVRE COMPORTANT UNE COUCHE D'OXYDE MÉTALLIQUE ET UNE COUCHE DE PLASTIQUE, PROCÉDÉ POUR LA PRODUCTION DESDITS PIGMENTS MÉTALLIQUES, AGENT DE REVÊTEMENT ET OBJET REVÊTU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.03.2011 DE 102011001575**
          **27.05.2011 DE 102011103882**

(43) Veröffentlichungstag der Anmeldung:
**29.01.2014 Patentblatt 2014/05**

(73) Patentinhaber: **Eckart GmbH**
**91235 Hartenstein (DE)**

(72) Erfinder:
• **STRUCK, Oliver**
  **91239 Henfenfeld (DE)**
• **NGUYEN, Phu Qui**
  **41238 Mönchengladbach (DE)**
• **SCHUMACHER, Dirk**
  **91257 Pegnitz (DE)**
• **HÖFENER, Sebastian**
  **90482 Nürnberg (DE)**

(74) Vertreter: **Louis Pöhlau Lohrentz**
**Patentanwälte**
**Postfach 30 55**
**90014 Nürnberg (DE)**

(56) Entgegenhaltungen:
WO-A1-2009/135784     DE-A1- 10 238 090
DE-A1-102005 061 684

**Beschreibung**

Die Erfindung betrifft kupferhaltige Metallpigmente mit wenigstens einer umhüllenden Metalloxidschicht und wenigstens einer umhüllenden Kunststoffschicht sowie ein Verfahren zu deren Herstellung. Die Erfindung betrifft weiterhin ein Beschichtungsmittel sowie einen beschichteten Gegenstand.

Kupferhaltige Metallpigmente, insbesondere Metalleffektpigmente, wie Kupferpigmente oder Messingpigmente, die auch als Goldbronzepigmente bezeichnet werden, werden häufig in der graphischen Industrie beispielsweise in Druckfarben, eingesetzt.

Kupferhaltige Metallpigmente sind aufgrund ihres Gehaltes an Kupfer in der Anwendung problematisch. Kupferhaltige Pigmente, beispielsweise kupferhaltige Metalleffektpigmente, geben leicht Cu(I)-Ionen an die Umgebung, beispielsweise einen Lack oder eine Farbe, ab. Die Cu(I)-Ionen gehen unter Einfluss von Feuchtigkeit leicht in Cu(II) über. Mit dem Wechsel der Oxidationszahl von Cu(I) zu Cu(II) geht zwangsläufig eine Reduktion von Komponenten der Umgebung, beispielsweise von Bindemittelkomponenten eines Lackes oder eine Farbe, einher. Darüber hinaus bilden insbesondere Cu(II)-Ionen farbige Komplexe, die sich störend auf den Farbton der entsprechenden Anwendung auswirken können. Beispielsweise bilden Cu(II)-Ionen unter alkalischen Bedingungen, insbesondere in Gegenwart von Aminen, intensiv blaue Kupferkomplexe.

Vor allem jedoch können die Cu(II)-Ionen, und im Fall von Goldbronzepigmenten auch von Zn(II)-Ionen, eine Gelierung des Bindemittels bewirken, was die entsprechende Anwendung unbrauchbar macht.

Aus diesem Grund können beispielsweise Kupfer- oder Goldbronzepigmente, die gemäß der DE 102 38 090 A1 mit $SiO_2$ beschichtet sind, nicht zuverlässig in Nagellacken eingesetzt werden.

Die vorgenannten Effekte sind speziell mit kupferhaltigen Metallpigmenten verbunden, finden mithin kein Pendant bei anderen Metallpigmenten beispielsweise bei Metallpigmenten aus Aluminium, Eisen, Zinn, etc..

Gemäß der Lehre der WO 2009/149834 A2 werden kupferhaltige Metalleffektpigmente sowie Beschichtungsmittel mit kupferhaltigen Metalleffektpigmenten bereitgestellt, in denen die vorgenannten kupferspezifischen Probleme dadurch gelöst werden, dass als weitere Komponente ein Cellulosederivat, ausgewählt aus der Gruppe bestehend aus Alkylcellulose, Hydroxyalkylcellulose, Alkyl(hydroxyalkyl)cellulose und Mischungen davon vorhanden ist.

Diese speziellen Cellulosederivate können theoretisch flüssigen Beschichtungsmitteln wie Druckfarben, Lacken oder Farben zugegeben werden. Jedoch verhindern beispielsweise Inkompatibilitäten oft den Einsatz derartiger Cellulosederivate.

Die WO 2007/017195 A2 offenbart allgemein Metalleffektpigmente mit einer Beschichtung. Die Beschichtung ist dadurch gekennzeichnet, dass sie eine anorganisch/organische Mischschicht umfasst. Die Mischschicht enthält dabei ein anorganisches Netzwerk und wenigstens eine organische Komponente, wobei die organische Komponente ein organisches Oligomer und/oder Polymer ist, das wenigstens teilweise mit dem anorganischen Netzwerk über einen oder mehrere organische Netzwerkbildner kovalent verbunden ist.

Es hat sich herausgestellt, dass die Herstellung von anorganisch/organischen Mischschichten prozesstechnisch aufwendig ist.

Aus der WO 2005/063897 A2 sind ebenfalls allgemein Metalleffektpigmente mit Beschichtung bekannt. Diese Metalleffektpigmente sind mit chemisch vernetzbaren und/oder unter Einwirkung von Wärme, IR-Strahlung, UV-Strahlung und/oder Elektronenstrahlen vernetzbarem oligomeren und/oder polymeren Bindemittel beschichtet. Diese chemisch noch reaktive Bindemittelbeschichtung erlaubt nach Applikation des Metallpigmentes mit dem Bindemittel eines Lackes oder einer Druckfarbe eine Umsetzung. Unter der Bindemittelbeschichtung kann eine Vorbeschichtung mit $SiO_2$ angeordnet sein.

Es hat sich gezeigt, dass bei kupferhaltigen Metallpigmenten mit der aus der WO 2005/063897 bekannten Beschichtung mit noch nicht vernetzter Bindemittelbeschichtung die Abgabe von Kupferionen an die Umgebung nicht zuverlässig verhindert wird. Zudem müssen relativ große Mengen an Bindemittel zur Beschichtung verwendet werden, was z.B. zu einer verringerten Deckfähigkeit führt.

Die DE 198 20 112 A1 offenbart mit reaktiven Orientierungshilfsmitteln beschichtete Effektpigmente. Gegenstand dieser Anmeldung sind im wesentlichen Aluminiumeffektpigmente, die mit einer Beschichtung aus Metalloxiden oder Polymeren beschichtet sein können. Auf dieser Beschichtung sind sodann Orientierungshilfsmittel angeordnet, die eine kovalente Anbindung an das Bindemittel einer Farbe oder eines Lackes ermöglichen.

Schließlich sind aus der DE 40 30 727 A1 kunstharzbeschichtete Metalleffektpigmente, insbesondere Aluminiumeffektpigmente, bekannt.

Aus der US 2009/0117281 A1 ist eine Doppelbeschichtung aus $SiO_2$ und einem Kunstharz von Metalleffektpigmenten bekannt. Diese Beschichtung ermöglicht den Einsatz der Metallpigmente in wässrigen Formulierungen und bewirkt eine verbesserte Chemikalienstabilität und Wasserbeständigkeit der damit applizierten Filme. Die US 2009/0117281 A1 ist eindeutig auf den Einsatz von Aluminiumeffektpigmenten ausgerichtet.

Metalleffektpigmente mit verbesserter Chemikalienbeständigkeit werden ebenfalls in der WO 2008/095697 A1 beschrieben.

Mit Ausnahme der WO 2009/149834 A2 und der WO 2008/095697 A1 betrifft der vorstehend angeführte Stand der Technik grundsätzlich die Beschichtung von Aluminiumeffektpigmenten, um korrosionsstabile Aluminiumeffektpigmente bereitzustellen.

Abgesehen von der WO 2009/149834 A2 wird in keinem der vorgenannten Dokumente aus dem Stand der Technik das Problem angesprochen, dass kupferhaltige Pigmente Kupferionen an die Umgebung abgeben, die sodann zu unerwünschten Effekten führen können.

Die WO 2009/135784 A1 betrifft Perlglanzpigmente, die mit einer Metalloxid/Hydroxidschicht und einem Acrylcopolymer beschichtet sind.

Aufgabe der vorliegenden Erfindung ist es mithin, kupferhaltige Metallpigmente, insbesondere Metalleffektpigmente, bereitzustellen, die so stabilisiert sind, dass keine nachteiligen Mengen an Kupferionen an die Umgebung abgegeben werden. Beispielsweise färben derartige nachteilige Mengen an Kupferionen Nagellack grün, lassen ihn gelieren oder machen das Redispergieren mittels Aufschütteln unmöglich.

Weiterhin sollen die erfindungsgemäßen kupferhaltigen Metallpigmente möglichst geringe, vorzugsweise keine, Einbußen anderer anwendungstechnischer Eigenschaften wie beispielsweise Deckfähigkeit, Farbe, etc. z.B. beim Einbrennvorgang einer Pulverlackbeschichtung oder eines Coil-Coatings erleiden.

Ferner ist es eine weitere Aufgabe, Beschichtungsmittel bereitzustellen, die die erfindungsgemäßen kupferhaltigen Metallpigmente enthalten und möglichst geringe, vorzugsweise keine, der oben genannten Einbußen von anwendungstechnischen Eigenschaften erleiden.

Zudem ist es Aufgabe der vorliegenden Erfindung, einen beschichteten Gegenstand bereitzustellen, der die erfindungsgemäßen plättchenförmigen kupferhaltigen Metallpigmente enthält oder aufweist oder der das vorgenannte erfindungsgemäße Beschichtungsmittel enthält oder aufweist und möglichst geringe, vorzugsweise keine, der oben genannten Einbußen von anwendungstechnischen Eigenschaften erleidet.

Eine weitere Aufgabe der Erfindung ist es, kupferhaltige Metallpigmente, insbesondere Metalleffektpigmente, bereitzustellen, die prozesstechnisch einfach hergestellt werden können.

Die der Erfindung zugrundeliegende Aufgabe wird durch Bereitstellung von plättchenförmigen kupferhaltigen Metallpigmenten gelöst, wobei die plättchenförmigen kupferhaltigen Pigmente einen Gehalt an elementarem Kupfer von wenigstens 50 Gew.-%, bezogen auf das Gewicht des unbeschichteten kupferhaltigen Metallpigmentes, aufweisen und wobei die kupferhaltigen Metallpigmente wenigstens eine umhüllende Metalloxidschicht und wenigstens eine umhüllende chemisch nichtreaktive Kunststoffschicht aufweisen und die Summe des Gehalts der wenigstens einen chemisch nichtreaktiven Kunststoffschicht und der wenigstens einen chemisch nichtreaktiven Metalloxidschicht in einem Bereich von 10 bis 50 Gew.-% , bezogen auf das Gewicht des unbeschichteten Metallpigments, liegt und das Gewichtsverhältnis der wenigstens einen Metalloxidschicht zur wenigstens einen chemisch nichtreaktiven Kunststoffschicht in einem Bereich von 1:2 bis 1:20 liegt.

Bevorzugte Weiterbildungen der erfindungsgemäßen plättchenförmigen kupferhaltigen Metallpigmente sind in den Unteransprüchen 2 bis 12 angegeben.

Ferner wird die der Erfindung zugrundeliegende Aufgabe durch ein Beschichtungsmittel gelöst, das die erfindungsgemäßen plättchenförmigen kupferhaltigen Metallpigmente enthält.

Zudem wird die der Erfindung zugrundeliegende Aufgabe durch einen beschichteten Gegenstand gelöst, der die erfindungsgemäßen plättchenförmigen kupferhaltigen Metallpigmente enthält oder aufweist oder der das vorgenannte erfindungsgemäße Beschichtungsmittel enthält oder aufweist.

Die der Erfindung zugrundeliegende Aufgabe wird zudem durch die Verwendung der erfindungsgemäßen plättchenförmigen kupferhaltigen Metallpigmenten in einem Beschichtungsmittel gelöst.

Die der Erfindung zugrundeliegende Aufgabe wird ferner durch Bereitstellung eines Verfahrens zur Herstellung eines der erfindungsgemäßen plättchenförmigen kupferhaltigen Metallpigmente gelöst, das die folgenden Schritte umfasst:

(1a) Beschichten von plättchenförmigen kupferhaltigen Metallpigmenten mit Metalloxid,
(1b) Beschichten der in Schritt (1a) erhaltenen metalloxidbeschichteten plättchenförmigen kupferhaltigen Metallpigmenten mit dem (den) Edukt(en) der chemisch nichtreaktiven Kunststoffschicht,
(1c) Aushärten oder Auspolymerisieren der in Schritt (1b) mit dem (den) Edukt(en) der chemisch nichtreaktiven Kunststoffschicht beschichteten kupferhaltigen Metallpigmente,
oder
(2a) Beschichten der plättchenförmigen kupferhaltigen Metallpigmenten mit dem (den) Edukt(en) der chemisch nichtreaktiven Kunststoffschicht,
(2b) Aushärten oder Auspolymerisieren der in Schritt (2a) mit dem (den) Edukt(en) der chemisch nichtreaktiven Kunststoffschicht beschichteten plättchenförmigen kupferhaltigen Metallpigmente,
(2c) Beschichten der in Schritt (2b) erhaltenen chemisch nichtreaktiven Kunststoffschicht beschichteten plättchenförmigen kupferhaltigen Metallpigmente mit Metalloxid.

Die Summe des Gehalts der wenigstens einen chemisch nichtreaktiven Kunststoffschicht und der wenigstens einen chemisch nichtreaktiven Metalloxidschicht liegt zwingend in einem Bereich von 10 bis 50 Gew.-%, bezogen auf das Gewicht des unbeschichteten Metallpigments. Innerhalb dieses Bereiches liegt das Gewichtsverhältnis der wenigstens einen Metalloxidschicht zur wenigstens einen chemisch nichtreaktiven Kunststoffschicht in einem Bereich von 1:2 bis 1:20. Insoweit sind Gewichtsverhältnisse, die innerhalb des Bereiches von 1:2 bis 1:20, aber außerhalb des Bereichs von 10 bis 50 Gew.-% liegen, nicht erfindungsgemäße Ausführungsformen.

**Plättchenförmige kupferhaltige Metallpigmente**

Die erfindungsgemäßen plättchenförmigen kupferhaltigen Metallpigmente weisen einen Gehalt an elementarem Kupfer von wenigstens 50 Gew.-%, bevorzugt von wenigstens 60 Gew.-%, weiter bevorzugt von wenigstens 70 Gew.-%, noch weiter bevorzugt von wenigstens 80 Gew.-%, noch weiter bevorzugt von wenigstens 90 Gew.-%, jeweils bezogen auf das Gewicht des unbeschichteten kupferhaltigen Metallpigmentes auf. Im Sinne der Erfindung wird unter dem vorgenannten Gehalt an elementarem Kupfer auch der in einer Legierung enthaltene Anteil an Kupfer verstanden.

Gemäß einer Ausführungsform der Erfindung weisen die plättchenförmigen Kupferpigmente, auch Kupfereffektpigmente genannt, vorzugsweise einen Kupfergehalt von 98 bis 100 Gew.-%, bevorzugt von 99 bis 99,999 Gew.-%, jeweils bezogen auf das Gewicht der unbeschichteten kupferhaltigen Metallpigmente auf. Es versteht sich von selbst, dass der Fachmann bei der Angabe 100 Gew.-% Kupfer auch gegebenenfalls in Spurenmengen enthaltene übliche Fremdmetalle mitliest. Der Begriff "Spurenmenge" im Sinne der vorliegenden Erfindung bezeichnet Mengen von höchstens 0,01 Gew.-%.

Gemäß einer weiteren bevorzugten Ausführungsform handelt es sich bei den plättchenförmigen kupferhaltigen Metallpigmenten um Zink und Kupfer enthaltende Messingpigmente, die auch als Goldbronzen bezeichnet werden.

Bei weiter bevorzugten Ausführungsformen handelt es sich bei den plättchenförmigen kupferhaltigen Metallpigmenten um oxidierte Kupferpigmente oder oxidierte Messingpigmente. Derartige Effektpigmente werden durch die sogenannte "Feuerbronzierung" erhalten. Die Metalleffektpigmente werden hierbei gezielt unter Hitzeeinwirkung oxidiert. Der sich bildende Metalloxidfilm führt zu Interferenzeffekten sowie, durch die rötliche Eigenfärbung von Kupferoxid, zu einer entsprechend modifizierten Körperfarbe.

Messingeffektpigmente, üblicherweise als "Goldbronze" bezeichnet, weisen bevorzugt einen Kupfergehalt von 70 bis weniger als 98 Gew.-%, vorzugsweise 75 bis 90 Gew.-%, auf, jeweils bezogen auf das Gewicht der unbeschichteten kupferhaltigen Metallpigmente auf. Der Zinkgehalt liegt vorzugsweise entsprechend zwischen 30 und mehr als 2 Gew.-%, vorzugsweise bei 25 bis 10 Gew.-%, beispielsweise bei 25 Gew.-%, wobei gegebenenfalls bis zu 2 Gew.-%, bevorzugt unter 1 Gew.-%, des Kupfers durch Verunreinigungen anderer Metalle ersetzt sein können, jeweils bezogen auf das Gewicht des unbeschichteten kupferhaltigen Metallpigments.

Bei Messingeffektpigmenten oder Goldbronze-Effektpigmenten wird der Farbton durch das Kupfer-Zink-Verhältnis der Legierung bestimmt.

Goldbronze-Effektpigmente werden in charakteristischen Naturfarbtönen als "Bleichgold" mit einem Kupferanteil von ca. 90 % und einem Rest von ca. 10 Gew.-% Zink, als "Reichbleichgold" mit einem Kupferanteil von ca. 85 Gew.-% und einem Rest von ca. 15 Gew.-% Zink sowie als "Reichgold" mit einem Kupferanteil von ca. 70 Gew.-% und einem Rest von ca. 30 Gew.-% Zink, kommerziell gehandelt. Die Angabe in Gew.-% bezieht sich dabei jeweils auf das unbeschichtete kupferhaltige Metallpigment.

Bei einer bevorzugten Ausführungsform enthalten die unbeschichteten Messingeffektpigmente eine "Verunreinigung" mit beispielsweise 0,1 bis 2 Gew.-%, vorzugsweise 0,5 bis 1,8 Gew.-%, Aluminium, jeweils bezogen auf den Gewicht des unbeschichteten kupferhaltigen Metalleffektpigmentes. Die Legierungen, die einen solchen Anteil an Aluminium haben, haben sich als korrosionsstabiler, verglichen mit ausschließlich Kupfer und Zink enthaltenden Messingeffektpigmenten, erwiesen.

Bei bestimmten bevorzugten Ausführungsformen werden die plättchenförmigen kupferhaltigen unbeschichteten Metallpigmente aus der Gruppe, bestehend aus Kupferpigmenten, Messingpigmenten, oxidierten Kupferpigmenten, oxidierten Messingpigmenten und Mischungen davon, ausgewählt.

An den Flächen der Plättchenform dieser Metalleffektpigmente wird einfallendes Licht wie von einem Spiegel gerichtet reflektiert, wodurch der metallische Effekt, vorliegend der Effekt von kupferhaltigen Metalleffektpigmenten, bei einem Betrachter hervorgerufen wird.

Die plättchenförmigen kupferhaltigen unbeschichteten Metallpigmente, die gemäß der vorliegenden Erfindung verwendet werden, weisen einen mittleren Pigmentdurchmesser ($D_{50}$) aus einem Bereich von etwa 1 $\mu$m bis etwa 200 $\mu$m, vorzugsweise von etwa 3 $\mu$m bis 120 $\mu$m, noch weiter bevorzugt von etwa 5 $\mu$m bis etwa 80 $\mu$m, auf. Als sehr geeignet haben sich auch Pigmentdurchmesser aus einem Bereich von etwa 10 $\mu$m bis etwa 50 $\mu$m, vorzugsweise von etwa 15 $\mu$m bis etwa 40 $\mu$m, erwiesen.

Die Bestimmung der Größenverteilung der Partikel erfolgt vorzugsweise mittels Lasergranulometrie. Bei dieser Methode können die Partikel in Form eines Pulvers vermessen werden. Die Streuung des eingestrahlten Laserlichts wird in verschiedene Raumrichtungen erfasst und gemäß der Fraunhofer Beugungstheorie ausgewertet. Dabei werden die Partikel rechnerisch als Kugeln behandelt. Somit beziehen sich die ermittelten Durchmesser stets auf den über alle Raumrichtungen ermittelten Äquivalentkugeldurchmesser, unabhängig von der tatsächlichen Form der Partikel. Der Auswertung der Beugungsdaten liegt ein Modell zugrunde, welches auf den Durchmesser einer Äquivalentkugel abzielt. Daher werden keine Absolutwerte erhalten, jedoch haben sich die gemessenen Durchmesser als verlässliche Relativwerte in der Beschreibung der Grössencharakteristik von plättchenförmigen Metallpigmenten durchgesetzt. Es wird die Größenverteilung ermittelt, die in Form eines Volumenmittels, bezogen auf den Äquivalentkugeldurchmesser berechnet wird. Diese volumengemittelte Größenverteilung kann als Summenhäufigkeitsverteilung dargestellt werden. Die Summenhäufigkeitsverteilung wird vereinfachend durch verschiedene Kennwerte charakterisiert, beispielsweise den $D_{50}$-Wert. Der Begriff "mittlerer Pigmentdurchmesser" oder "$D_{50}$" im Sinne der vorliegenden Erfindung bezeichnet die Partikelgröße, bei der 50 % der vorgenannten mittels Lasergranulometrie volumengemittelten Partikelgrößenverteilung unter und 50 % der vorgenannten mittels Lasergranulometrie volumengemittelten Partikelgrößenverteilung oberhalb des angegebenen Wertes liegen. Die Messungen können beispielsweise mit dem Partikelgrößenanalysator HELOS der Fa. Sympatec GmbH, Clausthal-Zellerfeld, Deutschland, durchgeführt werden.

Die mittlere Dicke ($h_{50}$) der bei der vorliegenden Erfindung verwendeten kupferhaltigen unbeschichteten Metalleffektpigmente liegt vorzugsweise in einem Bereich von 25 nm bis etwa 2 $\mu$m, vorzugsweise von etwa 40 nm bis etwa 1,5 $\mu$m, noch weiter bevorzugt von etwa 70 nm bis 1,1 $\mu$m, noch weiter bevorzugt von etwa 80 nm bis etwa 1 $\mu$m. Der Begriff "mittlere Dicke" oder "$h_{50}$" im Sinne der Erfindung bezieht sich auf das arithmetische Mittel der Dicken von mindestens 100 Metalleffektpigmenten mittels der Rasterelektronenmikroskopie (REM). Hierbei ist auf eine möglichst gute Orientierung der Plättchen im Anwendungsmedium zu achten. Hierzu können die Metalleffektpigmente zuvor durch geeignete Additive vorbehandelt werden. Anschließend wird der ausgehärtete Lack angeschliffen und nach üblicher Probenpräparation der Querschliff im REM betrachtet. Für die Zählung werden nur Teilchen ausgewählt, die eine gute Orientierung aufweisen. Die mittlere Dicke oder der $h_{50}$-Wert bezieht sich dabei auf das unbeschichtete kupferhaltige Metallpigment oder Metalleffektpigment.

Als sehr geeignet hat sich auch eine mittlere Dicke ($h_{50}$) von etwa 90 nm bis etwa 600 nm, weiter bevorzugt von etwa 110 nm bis etwa 450 nm, erwiesen.

Das Größen-Dicken-Verhältnis, das auch als Aspektverhältnis bezeichnet wird, liegt dabei vorzugsweise in einem Bereich von etwa 1000:1 bis 3:1, weiter bevorzugt von etwa 700:1 bis etwa 10:1, noch weiter bevorzugt bis etwa 500:1 bis 20:1. Der Begriff "Größen-Dicken-Verhältnis" oder "Aspektverhältnis" im Sinne der Erfindung bezieht sich auf das Verhältnis von $D_{50}$ zu $h_{50}$.

Bei bestimmten Ausführungsformen hat sich ferner ein Größen-Dicken-Verhältnis von etwa 450:1 bis 10:1, noch weiter bevorzugt von etwa 400:1 bis 15:1, als vorteilhaft erwiesen. Ferner wurde in bestimmten Varianten der Erfindung gefunden, dass ein sehr geeignetes Größen-Dicken-Verhältnis im Bereich von etwa 80:1 bis 3:1, weiter bevorzugt von etwa 50:1 bis 5:1, noch weiter bevorzugt von etwa 40:1 bis 10:1 liegt.

Die Erfinder haben überraschend herausgefunden, dass eine wirksame Einkapselung von kupferhaltigen Metallpigmenten erreicht werden kann, wenn wenigstens eine umhüllende Metalloxidschicht und wenigstens eine umhüllende chemisch nichtreaktive Kunststoffschicht auf die kupferhaltigen Pigmente aufgebracht ist.

Bei bestimmten besonders bevorzugten Ausführungsformen ist die wenigstens eine umhüllende Metalloxidschicht zwischen plättchenförmigen kupferhaltigem Metallpigment und der wenigstens einen umhüllenden chemisch nichtreaktiven Kunststoffschicht angeordnet. Ein derartiger Schichtaufbau hat sich als besonders effektiv hinsichtlich beispielsweise der Chemikalienbeständigkeit und insbesondere dem Verhindern des Austritts von Kupferionen herausgestellt.

Bei wieder anderen Ausführungsformen ist es hingegen bevorzugt, dass die wenigstens eine chemisch nichtreaktive Kunststoffschicht zwischen dem kupferhaltigem Metallpigment und der wenigstens einen umhüllenden Metalloxidschicht angeordnet ist. Metallpigmente mit einem derartigen Schichtaufbau sind beispielsweise durch eine besondere Härte gekennzeichnet. Es hat sich darüber hinaus überraschenderweise gezeigt, dass Metallpigmente mit einem wenigstens zweischichtigen Beschichtungsaufbau mit mindestens einer umhüllenden Metalloxidschicht und mindestens einer umhüllenden chemisch nichtreaktiven Kunststoffschicht sich durch eine besondere Stabilität auch gegenüber mechanischen Einflüssen, beispielsweise abrasiven Einflüssen, auszeichnen.

Ohne dass es als Einschränkung der vorliegenden Erfindung verstanden werden soll, wird vermutet, dass die mechanische Stabilität beispielsweise der erfindungsgemäßen kupferhaltigen Metallpigmente mit außenliegender chemisch nichtreaktiver Kunststoffschicht darauf zurückzuführen ist, dass die vorgenannte chemisch nichtreaktive Kunststoffschicht über eine gewisse Elastizität verfügt, d.h. nicht spröde ist. Somit können mechanische Kräfte, die auf die erfindungsgemäßen kupferhaltigen Metallpigmente einwirken, durch die äußere umhüllende chemisch nichtreaktive Kunststoffschicht abgefedert werden.

**Chemisch nichtreaktive Kunststoffschicht:**

Unter einer "chemisch nichtreaktiven Kunststoffschicht" wird erfindungsgemäß verstanden, dass die Kunststoffschicht im Wesentlichen vollständig, vorzugsweise vollständig, ausgehärtet ist. Diese ausgehärtete Kunststoffschicht reagiert mithin nicht wesentlich mit dem Bindemittel eines Beschichtungsmittels, wie zum Beispiel eines Lackes, beispielsweise eines Pulverlackes, oder einer Farbe. Gemäß einer bevorzugten Variante findet keine Reaktion zwischen der ausgehärteten Kunststoffschicht und dem Bindemittel eines Beschichtungsmittels statt.

Keinesfalls handelt es sich somit bei der "chemisch nichtreaktiven Kunststoffschicht" um eine Beschichtung aus noch nicht ausgehärtetem Bindemittel, wie sie in der WO 2005/063897 A2 offenbart sind. Ein Bindemittel ist dadurch charakterisiert, dass es erst später in der Anwendung, beispielsweise als Harz/Härtersystem oder durch radikalische Polymerisation, aushärtet.

In diesem Fall jedoch sind die Metallpigmente irreversibel in den ausgehärteten Pulverlack eingebunden. Somit ermöglicht die Erfindung die Bereitstellung eines Ensembles von vereinzelten kupferhaltigen Metallpigmenten, die wenigstens eine umhüllende Metalloxidschicht und eine umhüllende chemisch nichtreaktive Kunststoffschicht aufweisen. Inbesondere ermöglicht die vorliegende Erfindung die Bereitstellung von Pulvern und Pasten enthaltend die erfindungsgemäßen kupferhaltigen Metallpigmente.

Bei herkömmlichen Metalleffektpigmenten werden Schutzschichten aufgebracht, die die Metalleffektpigmente, üblicherweise Aluminium- und/oder Eiseneffektpigmente, vor korrosiven Einflüssen aus der Umgebung schützen sollen.

Vorliegend hat sich gezeigt, dass kupferhaltige Pigmente effektiv eingekapselt werden können, so dass keine merklichen Mengen an Kupferionen, vorzugsweise keine Kupferionen, von den kupferhaltigen Pigmenten in die Umgebung, beispielsweise eines Lackes, einer Farbe, eines Kunststoffes oder eines Kosmetikums abgegeben werden.

Die erfindungsgemäßen kupferhaltigen Metallpigmente weisen eine mittlere Dicke der Kunststoffschicht in einem Bereich von 100 nm bis 300 nm, bevorzugt von 120 nm bis 250 nm und besonders bevorzugt von 150 nm bis 230 nm auf. Unterhalb einer mittleren Dicke der Kunststoffschicht von 100 nm wird ein deutlicher Abfall der vorteilhaften Eigenschaften beobachtet. Oberhalb einer mittleren Dicke von 300 nm wiederum wird die Deckfähigkeit und/oder der Glanz der erfindungsgemäßen Metallpigmente in ihren Applikationen negativ beeinträchtigt.

Es wird vermutet, das diese verhältnismäßig dicken Kunststoffschichten vor allem als Barriereschicht gegenüber Wasser und andere korrosiven Chemikalien wirken. Ohne dass es als Einschränkung der vorliegenden Erfindung verstanden werden soll, ist es die Ansicht der Erfinder, dass die Schicht jedoch auch Kupfer- und/oder Zinkionen in einem gewissen Ausmaß in der Beschichtung zurückhält, so dass diese Ionen nicht in das umgebende Anwendungsmedium gelangen können.

Bei bestimmten Ausführungsformen ist es insbesondere bevorzugt, dass der Gewichtsanteil der wenigstens einen chemisch nichtreaktiven Kunststoffschicht mindestens 8 Gew.-%, vorzugsweise mindestens 9 Gew.-%, weiter bevorzugt mindestens 9,5 Gew.-%, mehr bevorzugt mindestens 10 Gew.-% und noch mehr bevorzugt mindestens 11 Gew.-% beträgt, jeweils bezogen auf das Gewicht des unbeschichteten kupferhaltigen Metallpigmentes.

Der Gewichtsanteil der Kunststoffschichten, bezogen auf das Gewicht des unbeschichteten kupferhaltigen Metallpigmentes, hängt wesentlich von der spezifischen Oberfläche des unbeschichteten Metallpigments ab. Er liegt gemäß weiteren bevorzugten Ausführungsformen in einem Bereich von 8 bis 40 Gew.-%, vorzugsweise im Bereich von 9 bis 35 Gew.-%, weiter bevorzugt im Bereich von 9,5 bis 30 Gew.-%, mehr bevorzugt im Bereich von 10 bis 23 Gew.-% und noch mehr bevorzugt im Bereich von 11 bis 18 Gew.-%, jeweils bezogen auf das Gewicht des unbeschichteten kupferhaltigen Metallpigmentes.

Gemäß weiteren bevorzugten Ausführungsformen besteht die wenigstens eine Kunststoffschicht im wesentlichen aus einem Kunststoff, der aus der Gruppe, bestehend aus Polyacrylat, Polymethacrylat, Polyacrylamid, Polyacrylnitril, Polyvinylchlorid, Polyvinylacetat, Polyamid, Polyalken, Polydien, Polyalkin, Polyalkylenglycol, Epoxidharz, Polyester, Polyether, Polyol, Polyurethan, Polycarbonat, Polyethylenterephthalat und Mischungen hiervon, ausgewählt wird.

Gemäß einer bevorzugten Ausführungsform besteht die wenigstens eine Kunststoffschicht im wesentlichen aus einem Kunststoff, der aus der Gruppe, bestehend aus Polyacrylat, Polymethacrylat, Polyurethan, Polyester und Mischungen davon, ausgewählt wird. Kupferhaltige Metalleffektpigmente mit mindestens einer derartigen Kunststoffschicht zeichnen sich beispielsweise durch eine erhöhte UV-Beständigkeit aus. Als besonders geeignete Kunststoffe zur Herstellung von Kunststoffschichten mit erhöhter UV-Beständigkeit haben sich beispielsweise Polyacrylate, Polymethacrylate oder deren Mischungen erwiesen. Bei bestimmten Ausführungsformen der Erfindung besteht die mindestens eine Kunststoffschicht.daher im wesentlichen aus Polyacrylaten und/oder Polymethacrylaten.

Als Monomere für die Herstellung von Polyacrylaten und Polymethacrylaten werden beispielsweise Isoamylacrylat, Laurylacrylat, Stearylacrylat, Butoxyethylacrylat, Ethoxy-diethylenglycolacrylat, Methoxy-triethylenglycolacrylat, Methoxy-polyethyleneglycolacrylat, Methoxydipropylenglycolacrylat, Phenoxyethylacrylat, Phenoxy-polyethylenglycolacrylat, Tetrahydrofurfurylacrylat, Isobornylacrylate, 2-Hydroxyethylacrylat, 2-Hydroxypropylacrylat, 2-Hydroxy-3-phenoxypropylacrylat, 2-Acryloyloxyethylsuccinsäure, 2-Acryloyloxyethylphthalsäure, 2-Acryloyloxyethyl-2-hydroxyethylphthalsäure, Triethylenglycoldiacrylat, Neopentylglycoldiacrylat, 1,6-Hexandioldiacrylat, 1,9-Nonandioldiacrylat, Dimethyloltricyclodecanediacrylat, Trimethylolpropantriacrylat, Pentaerythritoltriacrylat, Pentaerythritoltetraacrylat, Dipentaerythritolhexaacrylat, 2-Hydroxy-3-acryloyloxypropylmethacrylat, Isooctylacrylat, Isomyristylacrylat, Isostearylacrylat, 2-Ethylhexyldiglycolacrylat, 2-Hydroxybutylacrylat, 2-Acryloyloxyethylhexahydrophthalsäure, Hydroxypivalinsäure Neopentylglycoldiacrylat, Polytetraethylenglycoldiacrylat, Ditrimethylolpropantetraacrylat, Methylmethacrylat, Ethylmethacrylat, n-Butylmethacrylat, Isobutylmethacrylat, 2-Ethylhexylmethacrylat, Isodecylmethacrylat, n-Laurylmethacrylat, Tridecylmethacrylat, n-Stearylmethacrylat, Methoxydiethylenglycolmethacrylat, Methoxypolyethylenglycolmethacrylat, Cyclohexylmethacrylat, Tetrahydrofurfuralmethacrylat, Benzylmethacrylat, Phenoxyethylmethacrylat, Isobornylmethacrylat, 2-Hydroxyethylmethacrylat, 2-Hydroxypropylmethacrylat, 2-Hydroxybutylmethacrylat, 2-Methacryloyloxyethylsuccinsäure, 2-Methacroyloxyethylhexahydrophthalsäure, 2-Methacryloyloxyethyl2-hydroxypropylphthalat, Ethyleneglycoldimethacrylat, Diethyleneglycoldimethacrylat, 1,4-Butandioldimethacrylat, 1,3-Butandioldimethacrylat, 1,6-Hexandioldimethacrylat, 1,9-Nonandioldimethacrylat, Trimethylolpropantrimethacrylat, Glycerindimethacrylat, 2-Hydroxy-3-acryloyloxypropylmethacrylat, t-Butylmethacrylat, Isostearylmethacrylat, Methoxytriethylenglycolmethacrylat, n-Butoxyethylmethacrylat, 3-Chloro-2-hydroxypropylmethacrylat, Triethylenglycoldimethacrylat, Neopentylglycoldimethacrylat oder Mischungen hiervon verwendet.

Besonders bevorzugt wird mindestens ein Monomer mit mindestens zwei, besonders bevorzugt drei, reaktiven Doppelbindungen (Vernetzer) verwendet. Besonders bevorzugt enthält oder besteht das Monomer daher aus 1,6-Hexandioldiacrylat, 1,9-Nonandioldiacrylat, Dimethyloltricyclodecandiacrylat Neopentylglycoldimethacrylat Trimethylolpropantriacrylat, Trimethylolpropantrimethacrylat oder deren Mischungen.

Weiterhin kann die Acrylat-/Methacrylathaltige Kunststoffschicht zusätzlich Acrylsäure und/oder Methacrylsäure sowie weitere radikalisch polymerisierbare ungesättigte Verbindungen aufweisen.

Eine erhöhte UV-Stabilität ist dann erwünscht, wenn die erfindungsgemäßen kupferhaltigen Metallpigmente, insbesondere kupferhaltigen Metalleffektpigmente bei Außenanwendungen verwendet werden, wie beispielsweise in einem Automobillack, einem Fassadenlack, etc..

Gemäß einer weiteren erfindungsgemäßen Variante wird die Kunststoffschicht aus der Gruppe bestehend aus Polyamid, Polycarbonat, Polyvinylchlorid, Polyethylenterephthalat und Mischungen davon ausgewählt. Kupferhaltige Metallpigmente mit mindestens einer derartigen Kunststoffschicht zeichnen sich beispielsweise durch eine erhöhte Temperaturstabilität aus.

Gemäß einer bevorzugten Ausführungsform der Erfindung ist der Kunststoff bis zu einer Temperatur von wenigstens 180 °C, weiter bevorzugt von wenigstens 260 °C, noch weiter bevorzugt bis zu einer Temperatur von wenigstens 350 °C, temperaturstabil. Unter temperaturstabil wird verstanden, dass die Kunststoffbeschichtung der plättchenförmigen kupferhaltigen Metallpigmente bei der vorgenannten Temperatur nicht schmilzt und/oder zersetzt. Die Prüfung auf ein mögliches Schmelzen und/oder Zersetzen bei einer vorgegebenen Temperatur kann beispielsweise mittels dynamischer Differenzkalorimetrie erfolgen.

Gemäß einer weiteren bevorzugten Ausführungsform sind auf der umhüllenden chemisch nichtreaktiven Kunststoffschicht physikalisch gebundene Oberflächenmodifizierungsmittel aufgebracht.

## Metalloxidschicht:

Gemäß einer bevorzugten Variante der Erfindung wird die wenigstens eine Metalloxidschicht aus der Gruppe, bestehend aus Siliziumoxid, Aluminiumoxid, Boroxid, Zirkoniumoxid, Ceroxid, Eisenoxid, Titanoxid, Chromoxid, Zinnoxid, Molybdänoxid, deren Oxidhydraten, deren Hydroxiden und Mischungen davon, ausgewählt. Bei bestimmten bevorzugten Ausführungsformen ist die wenigstens eine Metalloxidschicht dadurch gekennzeichnet, dass die wenigstens eine Metalloxidschicht im wesentlichen aus Siliziumoxid besteht.

Im Rahmen dieser Erfindung wird unter dem Begriff "im wesentlichen bestehend aus Siliciumoxid" verstanden, dass die Schicht überwiegend aus Siliciumoxid, bevorzugt $SiO_2$, besteht, jedoch auch bis zu 20 Gew.-% Wasser, bezogen auf die Siliciumoxidschicht, enthalten kann. Ferner kann das Siliciumoxid, bei einer Sol-Gel-Synthese aus Tetraalkoxysilanen bis zu 5 Gew.-% Alkoxygruppen, die nicht hydrolysiert und kondensiert wurden, enthalten.

Vorzugsweise besteht(en) wenigstens eine, bevorzugt alle, der wenigstens einen umhüllenden Metalloxidschicht aus einer Siliziumoxidschicht bzw. Siliziumoxidschichten, vorzugsweise einer $SiO_2$-Schicht bzw. $SiO_2$-Schichten, und/oder Aluminiumoxidschicht bzw. Aluminiumoxidschichten, vorzugsweise eine $Al_2O_3$-Schicht bzw. $Al_2O_3$-Schichten. Bei bestimmten bevorzugten Ausführungsformen besteht(en) wenigstens eine, bevorzugt alle, der wenigstens einen umhüllenden Metalloxidschicht aus einer Siliziumoxidschicht(en), vorzugsweise einer $SiO_2$-Schicht. Bei weiteren bestimmten bevorzugten Ausführungsformen besteht bzw. bestehen wenigstens eine, bevorzugt alle, der wenigstens einen umhüllenden Metalloxidschicht aus einer Aluminiumoxidschicht bzw. Aluminiumoxidschichten, vorzugsweise einer $Al_2O_3$-Schicht bzw. $Al_2O_3$-Schichten. Bei bestimmten besonders bevorzugten Ausführungsformen ist die wenigstens eine umhüllende Metalloxidschicht eine (Zahl: 1) Siliziumoxidschicht, vorzugsweise eine (Zahl: 1) $SiO_2$-Schicht.

Es hat sich überraschenderweise gezeigt, dass die Verwendung einer bestimmten Mindestmenge an Metalloxidschicht vorteilhaft ist, um beispielsweise eine besonders hohe Oxidationstabilität zu erreichen. Bei bestimmten Ausführungsformen ist es daher bevorzugt, dass der Gewichtsanteil der wenigstens einen Metalloxidschicht mindestens 0,9 Gew.-%, vorzugsweise mindestens 1,0 Gew.-%, mehr bevorzugt mindestens 1,5 Gew.-%, noch mehr bevorzugt mindestens 2,0 Gew.-% und am meisten bevorzugt mindestens 2,5 Gew.-% beträgt, jeweils bezogen auf das Gewicht des unbeschichteten kupferhaltigen Metallpigmentes.

Gemäß einer weiteren bevorzugten Ausführungsform der Erfindung liegt der Gewichtsanteil der Metalloxidschicht in einem Bereich von 0,9 bis 12 Gew.-%, weiter bevorzugt von 1 bis 10 Gew.-% und besonders bevorzugt von 1,5 bis 9 Gew.-% und ganz besonders bevorzugt von 2 bis 8 Gew.-%, jeweils bezogen auf das Gewicht des unbeschichteten kupferhaltigen Metallpigmentes.

Überraschenderweise wurde zudem gefunden, dass bei plättchenförmigen kupferhaltigen Metallpigmenten, bei denen eine erfindungsgemäße Metalloxidschicht als oberste Schicht aufgebracht wurde, es besonders vorteilhaft ist, eine größere Menge der Metalloxidschicht aufzutragen. Bei bestimmten Ausführungsformen ist es daher bevorzugt, dass der Gewichtsanteil der wenigstens einen Metalloxidschicht bei plättchenförmigen kupferhaltigen Metallpigmenten, bei denen eine erfindungsgemäße Metalloxidschicht als oberste Schicht aufgetragen wurde, mindestens 0,9 Gew.-% beträgt, bezogen auf das Gewicht des unbeschichteten kupferhaltigen Metallpigmentes. Insbesondere ist es bei bestimmten Ausführungsformen bevorzugt, dass der Gewichtsanteil der wenigstens einen Metalloxidschicht bei plättchenförmigen kupferhaltigen Metallpigmenten, bei denen eine erfindungsgemäße Metalloxidschicht als oberste Schicht aufgetragen wurde, mindestens 1,0 Gew.-% und bei plättchenförmigen kupferhaltigen Metallpigmenten, bei denen eine erfindungsgemäße chemisch nichtreaktive Kunststoffschicht als oberste Schicht aufgebracht wurde, mindestens 0,9 Gew.-%; vorzugsweise bei plättchenförmigen kupferhaltigen Metallpigmenten, bei denen eine erfindungsgemäße Metalloxidschicht als oberste Schicht aufgetragen wurde, mindestens 1,5 Gew.-% und bei plättchenförmigen kupferhaltigen Metallpigmenten, bei denen eine erfindungsgemäße chemisch nichtreaktive Kunststoffschicht als oberste Schicht aufgebracht wurde, mindestens 1,0 Gew.-%; mehr bevorzugt bei plättchenförmigen kupferhaltigen Metallpigmenten, bei denen eine erfindungsgemäße Metalloxidschicht als oberste Schicht aufgetragen wurde, mindestens 2,0 Gew.-% und bei plättchenförmigen kupferhaltigen Metallpigmenten, bei denen eine erfindungsgemäße chemisch nichtreaktive Kunststoffschicht als oberste Schicht aufgebracht wurde, mindestens 1,5 Gew.-%; und noch mehr bevorzugt bei plättchenförmigen kupferhaltigen Metallpigmenten, bei denen eine erfindungsgemäße Metalloxidschicht als oberste Schicht aufgetragen wurde, mindestens 2,5 Gew.-% und bei plättchenförmigen kupferhaltigen Metallpigmenten, bei denen eine erfindungsgemäße chemisch nichtreaktive Kunststoffschicht als oberste Schicht aufgebracht wurde, mindestens 2,0 Gew.-% beträgt, jeweils bezogen auf das Gewicht des unbeschichteten kupferhaltigen Metallpigmentes. Ohne dass es als Einschränkung der vorliegenden Erfindung verstanden werden soll, ist es die Ansicht der Erfinder, dass eine größere Menge an Metalloxid erforderlich ist, um eine außen liegenden Metalloxidschicht zu bilden, die beispielsweise gegenüber den mechanischen Einwirkungen hinreichend widerstandsfähig ist und die gewünschte Stabilisierung des Metallpigmentes bietet. Insbesondere ist es bei bestimmten Ausführungsformen bevorzugt, dass der Gewichtsanteil der wenigstens einen umhüllenden Metalloxidschicht bei plättchenförmigen kupferhaltigen Metallpigmenten, bei denen eine chemisch nichtreaktive Kunststoffschicht die oberste Schicht der Beschichtung bildet, in einem Bereich von 0,9 bis 12 Gew.-% und bei plättchenförmigen kupferhaltigen Metallpigmenten, bei denen eine Metalloxidschicht die oberste Schicht der Beschichtung bildet, in einem Bereich von 1,0 bis 10 Gew.-%, vorzugsweise bei plättchenförmigen kupferhaltigen Metallpigmenten, bei denen eine chemisch nichtreaktive Kunststoffschicht, die oberste Schicht der Beschichtung bildet in einem Bereich von 1,0 bis 10 Gew.-% und bei plättchenförmigen kupferhaltigen Metallpigmenten, bei denen eine Metalloxidschicht die oberste Schicht der Beschichtung bildet, in einem Bereich von 1,5 bis 9 Gew.-%, mehr bevorzugt bei plättchenförmigen kupferhaltigen Metallpigmenten, bei denen eine chemisch nichtreaktive Kunststoffschicht die oberste Schicht der Beschichtung bildet, in einem Bereich von 1,5 bis 9 Gew.-% und bei plättchenförmigen kupferhaltigen Metallpigmenten, bei denen eine Metalloxidschicht die oberste Schicht der Beschichtung bildet, in einem Bereich von 2,0 bis 8 Gew.-% und noch mehr bevorzugt bei plättchenförmigen kupferhaltigen Metallpigmenten, bei denen eine chemisch nichtreaktive Kunststoffschicht die oberste Schicht der Beschichtung bildet, in einem Bereich von 2,0 bis 8 Gew.-% und bei plättchenförmigen kupferhaltigen Metallpigmenten, bei denen eine Metalloxidschicht die oberste Schicht der Beschichtung bildet, in einem Bereich von 2,5 bis 7 Gew.-% liegt, jeweils bezogen auf das Gewicht des unbeschichteten kupferhaltigen Metallpigmentes.

Die mittlere Dicke der Metalloxidschicht liegt vorzugsweise in einem Bereich von 2 nm bis 25 nm, weiter bevorzugt von 3 nm bis 20 nm. Als sehr geeignet hat sich auch eine mittlere Dicke in einem Bereich von 5 nm bis 10 nm erwiesen.

Überraschenderweise reicht bei dem erfindungsgemäßen plättchenförmigen kupferhaltigen Metallpigment bereits eine (Zahl: 1) dünne Metalloxidschicht in Verbindung mit einer (Zahl: 1) chemisch nichtreaktiven Kunststoffschicht aus, um die kupferhaltigen Metallpigmente zum Einen vor Umwelteinflüssen zu schützen und zum Anderen zu verhindern, dass Kupferionen von dem kupferhaltigen Metallpigment in die Umgebung, beispielsweise einen Lack, eine Farbe, ein Kosmetikum, etc. abgegeben werden.

Bei einer sehr bevorzugten Ausführungsform ist die Metalloxidschicht als erste Schicht direkt auf dem Metallsubstrat aufgebracht. Insbesonders bei dieser Ausführungsform wird vermutet, dass die Metalloxidschicht zunächst einmal eine relativ effektive Barriere gegenüber austretenden Kupfer- und/oder Zinkionen darstellt. Diejenigen Metallionen, die diese Barriere dennoch überwinden, werden offenbar effektiv in der Kunststoffschicht abgefangen.

**Synergistische Wirkung der beiden Schichten:**

Es wird vermutet, dass die umhüllende Metalloxidschicht und die umhüllende chemisch nichtreaktive Kunststoffschicht in einer synergistischen Weise zusammenwirken.

Zum Einen wird vermutet, dass die umhüllende chemisch nichtreaktive Kunststoffschicht, d.h. die im wesentlichen vollständig auspolymerisierte, vorzugsweise auspolymerisierte, und/oder ausgehärtete Kunststoffschicht eine so dichte Kunststoffmatrix bildet, dass etwaige durch die $SiO_2$-Schicht gelangenden Kupferionen von der dichten Kunststoffschicht zuverlässig eingeschlossen werden.

Zum Anderen wird vermutet, dass etwaige aus der Umgebung des erfindungsgemäßen kupferhaltigen Metallpigmentes durch die chemisch nichtreaktive Kunststoffschicht gelangenden korrodierenden Substanzen, wie bspw. $H^+$- oder $OH^-$- Ionen, zwischen der Metalloxidschicht und Kunststoffschicht ebenfalls eingeschlossen werden, so dass diese korrodierenden Substanzen nur geringfügig, vorzugsweise gar nicht, mit den kupferhaltigen Metallpigmenten in Kontakt kommen. Etwaig durch korrodierende Einflüsse gleichwohl herausgelöste Kupferionen werden sodann, wie bereits vorstehend ausgeführt, vermutlich zwischen der Metalloxidschicht und der chemisch nichtreaktiven Kunststoffschicht eingeschlossen und mithin nicht an die Umgebung, beispielsweise eine Farbe oder einen Lack abgegeben.

Somit wirkt der wenigstens zweischichtige Beschichtungsaufbau mit einer umhüllenden Metalloxidschicht und einer umhüllenden chemisch nichtreaktiven Kunststoffschicht synergistisch zusammen, so dass etwaige korrodierende Einflüsse aus der Umgebung, beispielsweise bei Verwendung der kupferhaltigen Pigmente in einem Automobillack, einem Fassadenlack, etc. nicht mit Kupfer in Kontakt kommen und, sollte dies passiert sein, etwaige freigesetzte Kupferionen nicht in die Umgebung gelangen.

Ein weiterer wichtiger Aspekt ist die Oxidation der plättchenförmigen kupferhaltigen Metallpigmente mit Luftsauerstoff bei Temperaturen oberhalb von ca. 80°C. Dies tritt beispielsweise sowohl bei der Aushärtung von Pulverlack (Einbrenntemperaturen ca. 200 °C) oder bei der Coil-Coating-Lackierung (Einbrenntemperaturen ca. 280°C) auf. Hierbei hat sich überraschenderweise gezeigt, dass bereits eine dünne Metalloxidschicht in Kombination mit der chemisch nichtreaktiven Kunststoffschicht ausreicht, um eine Oxidation des kupferhaltigen Metallpigments zu unterbinden. Die Kunststoffschicht ist zwar durchlässig für Sauerstoff, verleiht dem Produkt jedoch die erforderliche Chemikalienstabilität.

Bei bestimmten Ausführungsformen ist es insbesondere bevorzugt, dass die Summe des Gehalts der Polymerschicht und der Metalloxidschicht in einem Bereich von 13 bis 40 Gew.-%, vorzugsweise in einem Bereich von 14 bis 35 Gew.-%, mehr bevorzugt in einem Bereich von 15 bis 33 Gew.-%, noch mehr bevorzugt in einem Bereich von 16 bis 29 Gew.-%, jeweils bezogen auf das Gewicht des unbeschichteten Metallpigments, liegt.

Es ist erfindungsgemäß bevorzugt, die Summe des Gehalts der Polymerschicht und der Metalloxidschicht insgesamt möglichst niedrig zu halten. Auf diese Weise wird eine optimale Deckfähigkeit der Metalleffektpigmente gewährleistet. Zudem hat sich gezeigt, dass bei zu dicken Schichten die Metalleffektpigmente zur Agglomeration neigen können. Weiterhin nimmt bei dickeren Schichten, die oberhalb von 50 Gew.-%, bezogen auf das Gewicht des unbeschichteten Metallpigments, liegen, die Schutzwirkung überraschend ab. Es wird vermutet, dass dickere Schichten eine geringe Qualität hinsichtlich ihrer Schutzeigenschaften aufweisen, da sie eine größere Brüchigkeit aufweisen.

Erfindungswesentlich ist, dass die chemisch nichtreaktive Kunststoffschicht in einem deutlich höheren Gewichtsanteil im Verhältnis zur Metalloxidschicht aufgebracht wird.

Besonders bevorzugte Ausführungsformen weisen ein Gewichtsverhältnis der wenigstens einen Metalloxidschicht zur wenigstens einen chemisch nichtreaktiven Kunststoffschicht in einem Bereich von 1:2,2 bis 1:17, vorzugsweise in einem Bereich von 1:2,5 bis 1:15, weiter bevorzugt in einem Bereich von 1:2,7 bis 1:13 und noch weiter bevorzugt in einem Bereich von 1:3 bis 1:10 auf.

Ferner hat sich überraschenderweise gezeigt, dass es bei bestimmten Varianten vorteilhaft ist, einen engeren Bereich des Gewichtsverhältnisses der wenigstens einen Metalloxidschicht zur wenigstens einen chemisch nichtreaktiven Kunststoffschicht bei plättchenförmigen kupferhaltigen Metallpigmenten, bei denen eine erfindungsgemäße chemisch nichtreaktive Kunststoffschicht als erste Schicht aufgebracht wurde, zu wählen. Bei bestimmten Ausführungsformen ist es daher bevorzugt, dass das Gewichtsverhältnis der wenigstens einen Metalloxidschicht zur wenigstens einen chemisch nichtreaktiven Kunststoffschicht bei plättchenförmigen kupferhaltigen Metallpigmenten, bei denen eine erfindungsgemäße chemisch nichtreaktive Kunststoffschicht als erste Schicht aufgebracht wurde, in einem Bereich von 1:2,2 bis 1:17 und bei plättchenförmigen kupferhaltigen Metallpigmenten, bei denen eine erfindungsgemäße Metalloxidschicht als erste Schicht aufgebracht wurde, in einem Bereich von 1:2,0 bis 1:20; vorzugsweise bei plättchenförmigen kupferhaltigen Metallpigmenten, bei denen eine erfindungsgemäße chemisch nichtreaktive Kunststoffschicht als erste Schicht aufgebracht wurde, in einem Bereich von 1:2,5 bis 1:15 und bei plättchenförmigen kupferhaltigen Metallpigmenten, bei denen eine erfindungsgemäße Metalloxidschicht als erste Schicht aufgebracht wurde, in einem Bereich von 1:2,2 bis 1:17; weiter bevorzugt bei plättchenförmigen kupferhaltigen Metallpigmenten, bei denen eine erfindungsgemäße chemisch nichtreaktive Kunststoffschicht als erste Schicht aufgebracht wurde, in einem Bereich von 1:2,7 bis 1:13 und bei plättchenförmigen kupferhaltigen Metallpigmenten, bei denen eine erfindungsgemäße Metalloxidschicht als erste Schicht aufgebracht wurde, in einem Bereich von 1:2,5 bis 1:15; und noch weiter bevorzugt bei plättchenförmigen kupferhaltigen Metallpigmenten, bei denen eine erfindungsgemäße chemisch nichtreaktive Kunststoffschicht als erste Schicht aufgebracht wurde, in einem Bereich von 1:3 bis 1:10 und bei plättchenförmigen kupferhaltigen Metallpigmenten, bei denen eine erfindungsgemäße Metalloxidschicht als erste Schicht aufgebracht wurde, in einem Bereich von 1:2,7 bis 1:13 liegt. Ohne dass es als Einschränkung der Erfindung verstanden werden soll, ist es die Ansicht der Erfinder, dass eine zunächst aufgebrachte chemisch nichtreaktive Kunststoffschicht eine ungleichmäßigere Oberfläche für nachfolgende Beschichtungen bietet, so dass der Einsatz eines genauer spezifizierten Gewichtsverhältnisses der wenigstens einen Metalloxidschicht zur wenigstens einen chemisch nichtreaktiven Kunststoffschicht bei plättchenförmigen kupferhaltigen Metallpigmenten, bei denen eine erfindungsgemäße chemisch nichtreaktive Kunststoffschicht als erste Schicht aufgebracht wurde, sich als vorteilhaft erweist, um die erfindungsgemäßen Effekte in besonders ausgeprägter Form hervorzurufen.

Insbesondere sind bei bestimmten Varianten der Erfindung Bereiche bevorzugt, die durch eine Kunststoffmenge von 8 Gew.-% bis 40 Gew.-% und eine Metalloxidmenge von 0,9 Gew.-% bis 12 Gew.-%, vorzugsweise eine Kunststoffmenge von von 9 Gew.-% bis 35 Gew.-% und eine Metalloxidmenge von 1 Gew.-% bis 10 Gew.-%, weiter bevorzugt eine Kunststoffmenge von 10 Gew.-% bis 30 Gew.-% und eine Metalloxidmenge von 1,5 Gew.-% bis 9 Gew.-%, noch weiter bevorzugt eine Kunststoffmenge von 12 Gew.-% bis 25 Gew.-% und eine Metalloxidmenge von 2 Gew.-% bis 8 Gew.-%, gekennzeichnet sind, jeweils bezogen auf Gewicht des unbeschichteten kupferhaltigen Metallpigments.

Bei spezifischen bevorzugten Ausführungsformen besteht die wenigstens eine chemisch nichtreaktive Kunststoffschicht im wesentlichen aus Polyacrylat und/oder Polymethacrylat und die wenigstens eine Metalloxidschicht im wesentlichen aus Siliziumoxid, bevorzugt $SiO_2$, wobei das Gewichtsverhältnis der wenigstens einen Metalloxidschicht zur wenigstens einen chemisch nichtreaktiven Kunststoffschicht in einem Bereich von 1:2,2 bis 1:17 liegt und die Summe des Gehalts der wenigstens einen chemisch nichtreaktiven Kunststoffschicht und der wenigstens einen Metalloxidschicht in einem Bereich von 10 bis 50 Gew.-% , bezogen auf das Gewicht des unbeschichteten Metallpigments, liegt.

Bei weiteren spezifischen bevorzugten Ausführungsformen besteht die wenigstens eine chemisch nichtreaktive Kunststoffschicht im wesentlichen aus Polyacrylat und/oder Polymethacrylat und die wenigstens eine Metalloxidschicht im wesentlichen aus Siliziumoxid, bevorzugt $SiO_2$, wobei das Gewichtsverhältnis der wenigstens einen Metalloxidschicht zur wenigstens einen chemisch nichtreaktiven Kunststoffschicht in einem Bereich von 1:2,5 bis 1:15 liegt und die Summe des Gehalts der wenigstens einen chemisch nichtreaktiven Kunststoffschicht und der wenigstens einen Metalloxidschicht in einem Bereich von 13 bis 40 Gew.-%, bezogen auf das Gewicht des unbeschichteten Metallpigments, liegt.

Bei weiteren spezifischen bevorzugten Ausführungsform besteht die wenigstens eine chemisch nichtreaktive Kunststoffschicht im wesentlichen aus Polyacrylat und/oder Polymethacrylat und die wenigstens eine Metalloxidschicht im wesentlichen aus Siliziumoxid, bevorzugt $SiO_2$, wobei das Gewichtsverhältnis der wenigstens einen Metalloxidschicht zur wenigstens einen chemisch nichtreaktiven Kunststoffschicht in einem Bereich von 1:2,2 bis 1:17 liegt und die Summe des Gehalts der chemisch nichtreaktiven Kunststoffschicht und der Metalloxidschicht in einem Bereich von 13 bis 40 Gew.-% , bezogen auf das Gewicht des unbeschichteten Metallpigments, liegt. Bei bestimmten der vorgenannten spezifischen bevorzugten Ausführungsformen liegt das Gewichtsverhältnis der wenigstens einen Metalloxidschicht zur wenigstens einen Kunststoffschicht in einem Bereich von 1:2,5 bis 1:15.

Bei weiteren spezifischen bevorzugten Ausführungsformen liegt das Gewichtsverhältnis der wenigstens einen Metalloxidschicht zur wenigstens einen chemisch nichtreaktiven Kunststoffschicht in einem Bereich von 1:2,5 bis 1:15.

Bei weiter bevorzugten Ausführungsformen werden die oben genannten spezifischen bevorzugten Ausführungsformen dadurch ergänzt, dass der Gewichtsanteil der Siliziumoxid-, bevorzugt $SiO_2$-Schicht, in einem Bereich von 1,5 bis 9 Gew.-%, bezogen auf das Gewicht des unbeschichteten kupferhaltigen Metallpigmentes, liegt.

Bei weiter bevorzugten Ausführungsformen werden die oben genannten spezifischen bevorzugten Ausführungsformen dadurch ergänzt, dass der Gewichtsanteil der wenigstens einen chemisch nichtreaktiven Kunststoffschicht in einem Bereich von 10 bis 35 Gew.-%, bezogen auf das Gewicht des unbeschichteten kupferhaltigen Metallpigmentes, liegt.

Optional können zwischen der $SiO_2$-Schicht und der Kunststoffschicht ein oder mehrere organofunktionelle Silane aufgebracht sein, die mindestens eine radikalisch polymerisierbare Doppelbindung enthalten, bevorzugt mindestens eine Acrylat- und/oder Methacrylatgruppe.

Durch die relativ geringen Dicken der Metalloxidschicht und der verhältnismäßig hohen Kunststoffschichtdicke weisen die erfindungsgemäßen kupferhaltigen Metallpigmente zwei wesentliche Vorteile auf.

Zum Einen ist das Deckungsvermögen, d.h. die pro Gewichtseinheit an erfindungsgemäßem Pigment abgedeckte Fläche, immer noch sehr gut, verglichen mit der Abdeckung eines unbeschichteten Metallpigmentes. Je dicker die aufgebrachte transparente Umhüllung ist, desto schlechter wird das Deckungsvermögen, da pro Gramm Pigment immer weniger Metallteilchen vorhanden sind. Das Deckungsvermögen kann zusätzlich verschlechtert werden, wenn mit zunehmender Beschichtungsdicke mehr Feinanteil der Metallpigmente in die Beschichtung größerer Pigmente eingebaut wird. Dieser Feinanteil ist jedoch maßgeblich für ein gutes Deckungsvermögen verantwortlich.

Insoweit ist es von Vorteil, wenn die Beschichtung eine möglichst geringe Schichtdicke aufweist, da dann weniger Feinanteil in die Beschichtung größerer Pigmente eingebaut wird und dieser somit durch eine statistische Verteilung im Lack nach wie vor einen Beitrag zur Deckung beitragen kann. Um die mittlere Schichtdicke der Beschichtung gering halten zu können, ist Voraussetzung, dass die aufgebrachte umhüllende transparente Beschichtung das kupferhaltige Metallpigment wirksam vor korrosiven Umgebungseinflüssen schützt und auch verhindert, dass Kupferionen in die Umgebung abgegeben werden.

Eine Kombination einer dünnen Metalloxidschicht mit einer chemisch nichtreaktiven Kunststoffschicht erlaubt überraschenderweise zum Einen, das kupferhaltige Metallpigment vor korrosiven Umwelteinflüssen zu schützen und zugleich die Kupferionen wirksam einzukapseln, so dass diese nicht in die Umgebung abgegeben werden. Im Hinblick auf die transparente Beschichtung mit geringer Schichtdicke weisen die erfindungsgemäßen kupferhaltigen Metallpigmente mithin ein hervorragendes Deckungsvermögen auf.

Bei bestimmten Ausführungsformen wird die wenigstens eine Kunststoffschicht mittels einer Initiator-induzierten radikalischen Polymerisation erhalten. Es hat sich überraschenderweise gezeigt, dass im Fall einer durch einen Initiator gestarteten radikalischen Polymerisation eine raue chemisch nichtreaktive Kunststoffschicht erhalten wird. Dies ist bei Verwendung der erfindungsgemäßen plättchenförmigen kupferhaltigen Metallpigmente in einem Pulverlack von großem Vorteil. Es verhält sich offenbar so, dass eine rauere Oberflächenstruktur der chemisch nichtreaktiven Kunststoffschicht eine leichtere elektrostatische Aufladbarkeit der erfindungsgemäßen plättchenförmigen kupferhaltigen Metallpigmente bewirkt. Bei einer stärkeren elektrostatischen Aufladung lassen sich die erfindungsgemäßen plättchenförmigen kupferhaltigen Metallpigmente leichter auf ein zu lackierendes Werkstück applizieren.

Dies führt zum Einen zu einer effektiveren Lackierung, bei der der Anteil an nicht appliziertem Pulverlack, der so genannte Overspray, verringert wird. Auf der anderen Seite können zu lackierende Werkstücke innerhalb einer kürzeren Zeit mit einer qualitativ hochwertigeren Pulverlacklackierung versehen werden.

Im Gegensatz zu Anwendungen der erfindungsgemäßen Metalleffektpigmente im Pulverlack hat sich gezeigt, dass glatte Oberflächen, wie sie bei der thermischen Polymerisation entstehen, für Nasslackanwendungen wie Coil-Coating, von Vorteil sind. Durch die glatten Oberflächen gibt es einen gleichmäßigen Durchgang des Lichts durch die Grenzschicht Lack - Beschichtung des Pigments. Dadurch werden unerwünschte Streueffekte minimiert, so dass diese Pigmente brillanter als vergleichbare Metalleffektpigmente aus dem Stand der Technik erscheinen. Ein weiterer Vorteil ist, dass durch die kleinere Oberfläche, weniger Bindemittel zur Benetzung der Pigmente notwendig ist, so dass höhere Pigmentbeladungen möglich sind.

### Weitere Schichten:

Zwischen der wenigstens einen umhüllenden Metalloxidschicht und der wenigstens einen umhüllenden chemisch nichtreaktiven Kunststoffschicht können eine oder mehrere weitere Schichten angeordnet sein. Bei dieser einen oder mehreren zusätzlichen Schichten kann es sich beispielsweise ebenfalls um zusätzliche Metalloxidschichten handeln. Bei bestimmten Ausführungsformen ist es jedoch bevorzugt, dass die eventuell vorhandenen Schichten zwischen der wenigstens einen umhüllenden Metalloxidschicht und der wenigstens einen umhüllenden chemisch nichtreaktiven Kunststoffschicht keine Metalloxidschicht oder Kunststoffschicht im Sinne der vorliegenden Erfindung darstellen.

Bei einer bevorzugten Ausführungsform werden hier jedoch als Haftvermittler und/oder weitere Schichtkomponente organofunktionelle Silane, Titanate, Aluminate, Phosphonsäuren (z.B. VPS: Vinylphosphonsäure), Phorsphorsäureester und/oder Zirkonate eingesetzt, wobei organofunktionelle Silane besonders bevorzugt sind. Diese Verbindungen können aufgrund ihrer bekannten Hydrolyse- und Kondensationsreaktionen beispielsweise besonders gut auf der Metalloberfläche bzw. Metalloxidoberfläche anbinden. Die Verbindungen sollten mindestens eine chemisch polymerisierbare Gruppe aufweisen, welche bevorzugt der Kunststoffschicht angepasst ist.

Wenn die Kunststoffschicht beispielsweise aus Polyacrylaten und/oder Polymethacrylaten besteht, so weist das organofunktionelle Silan bevorzugt wenigstens eine funktionelle Gruppe auf, die mit einer Acrylatgruppe und/oder Methacrylatgruppe des Polyacrylats und/oder Polymethacrylats chemisch umgesetzt sein kann. Als sehr geeignet haben sich radikalisch polymerisierbare organische funktionelle Gruppen erwiesen. Vorzugsweise wird die wenigstens eine funktionelle Gruppe aus der Gruppe ausgewählt, die aus Acryl, Methacryl, Vinyl, Allyl, Ethinyl sowie weiteren organischen Gruppen mit ungesättigten Funktionen besteht.

Vorzugsweise weist das organofunktionelle Silan wenigstens eine Acrylat- und/oder Methacrylatgruppe auf, da diese mit den zur Erzeugung des Polyacrylates und/oder Polymethacrylates verwendeten Acrylat- bzw. Methacrylatverbindungen völlig problemlos unter Ausbildung einer homogenen Kunststoffschicht umgesetzt werden können.

Als organofunktionelle eine acrylat- und/oder methacrylathaltige Silane können erfindungsgemäß beispielsweise (Methacryloxymethyl)methyldimethoxysilan, Methacryloxymethyltrimethoxysilan, (Methacryloxymethyl)methyldiethoxysilan, Methacryloxymethyltriethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltrimethoxysilan, 3-Methacryloxypropyltriacetoxysilan, 3- Methacryloxypropymethyldimethoxysilan, Vinyltrichlorsilan, Vinyltrimethoxysilan Vinyldimethoxymethylsilan, Vinyltriethoxysilan, Vinyltris(2-methoxyethoxy)silan, Vinyltriacetoxysilan oder Mischungen davon verwendet werden.

Derartige Silane können als Haftvermittler zwischen Metalloxidschicht und Kunststoffschicht oder zwischen Metalloxidschicht, bevorzugt Siliciumoxidschicht und Kunststoffschicht wirken. Bei anderen Ausführungsformen können derartige Silane zumindest teilweise auch in die Kunststoffschicht einpolymerisiert werden, wie in der WO 2008/095697 A1 beschrieben, die hiermit unter Bezugnahme aufgenommen ist.

Im Rahmen dieser Erfindung wird unter einer chemisch nichtreaktiven Kunststoffschicht, die "im wesentlichen aus Polyacrylat und/oder Polymethacrylat besteht" verstanden, dass eine derartige Schicht durch acrylat- und/oder methacrylathaltige Silane modifiziert sein kann. Dabei entspricht der Mengenanteil der eingesetzten acrylat- und/oder methacrylathaltigen Silane maximal dem Mengenanteil der eingesetzten Acrylat- und/oder Methacrylatmonomere.

Vorzugsweise liegt das Molverhältnis von acrylat- und/oder methacrylathaltigen Silanen zu Acrylat- und/oder Methacrylatmonomere bei 1:2 bis 1:40, vorzugsweise bei 1:3 bis 1: 30.

Nicht Gegenstand dieser Erfindung sind jedoch Schichten, bei denen die acrylat- und/oder methacrylathaltige Silane in die wenigstens eine Metalloxidschicht, bevorzugt Siliciumoxidschicht, während eines Sol-Gel-Prozesses eingebaut werden. Derartige Schichten sind in der EP 1812519 B1 beschrieben. Es hat sich herausgestellt, dass durch die dort beschriebene Technologie die Schichten nicht zuverlässig reproduzierbar hergestellt werden können, um den kupferhaltigen plättchenförmigen Metallpigmenten die erforderlichen Stabilitäten zu verleihen.

Zwischen den kupferhaltigen Metallpigmenten und der wenigstens einen umhüllenden Metalloxidschicht sowie der wenigstens einen chemisch nichtreaktiven Kunststoffschicht können ebenfalls eine oder mehrere weitere Schichten angeordnet sein. Beispielsweise kann zwischen der umhüllenden Metalloxidschicht und den kupferhaltigen Metallpigmenten eine Schicht aus Kupferoxid angeordnet sein. Diese Kupferoxidschicht kann beispielsweise durch so genanntes Feuerfärben erhalten werden, bei dem kupferhaltige Pigmente in Gegenwart von Luftsauerstoff unter Ausbildung einer farbigen Kupferoxidschicht erhitzt werden. Diese Oxidation muss allerdings vor der Aufbringung der Metalloxidschicht erfolgen, da die Metalloxidschicht die Oxidation verhindert. Aufgrund der Eigenfarbe und von Interferenzeffekten ergeben diese Kupferoxidschichten in Abhängigkeit von ihren Schichtdicken bei plättchenförmigen Metalleffektpigmenten unterschiedlichste Farbtöne im gelb-roten Farbbereich.

Bei der sogenannten Feuerbehandlung von kupferhaltigen Metalleffektpigmenten wirkt Luftsauerstoff über einen definierten Zeitraum unter definierter Temperatur auf das kupferhaltige Metalleffektpigment ein, wodurch sich eine dünne Kupferoxidschicht auf dem kupferhaltigen Metallplättchen ausbildet. Durch Interferenzreflexion werden interessante Farbnuancen hervorgerufen. Feuereingefärbte kupferhaltige Metalleffektpigmente werden unter anderem in den Farbtönen Englischgrün, Zitron, Dukatengold und in Feuerrottönen kommerziell gehandelt. Die feuereingefärbten plättchenförmigen, kupferhaltigen Metallpigmente, die auch als kupferhaltige Metalleffektpigmente bezeichnet werden, sind aufgrund der durch die Wärmebehandlung in Gegenwart von Luftsauerstoff erzeugten Kupferoxidschicht gegenüber Korrosion oder korrodierenden Einflüssen in einem gewissen Umfang bereits geschützt.

Das kupferhaltige Metallpigment oder das mit einer Kupferoxidschicht durch Feuerfärbung versehene kupferhaltige Metallpigment weist sodann wenigstens eine umhüllende Metalloxidschicht auf, die von Kupferoxid verschieden ist. Mithin handelt es sich bei der wenigstens einen umhüllenden Metalloxidschicht, die synergistisch mit der chemisch nichtreaktiven Kunststoffschicht zusammenwirkt, im Sinne der Erfindung um keine Kupferoxidschicht.

Gemäß einer bevorzugten Variante der Erfindung ist die wenigstens eine Metalloxidschicht, die synergistisch mit der wenigstens einen chemisch nicht reaktiven Kunststoffschicht zusammenwirkt, nicht das Oxidationsprodukt des unbeschichteten kupferhaltigen Metallpigments. Vorzugsweise wird die Metalloxidschicht, die synergistisch mit der wenigstens einen chemisch nicht reaktiven Kunststoffschicht zusammenwirkt, in einem separaten Schritt aufgebracht. Bei dem separaten Schritt kann es sich beispielsweise um eine nasschemische Beschichtung oder um eine Gasphasenbeschichtung, beispielsweise mittels PVD oder CVD, handeln.

Bei bestimmten sehr bevorzugten Ausführungsformen der Erfindung folgen die umhüllende(n) Metalloxidschicht(en) und die umhüllend(n) chemisch nichtreaktive(n) Kunststoffschicht(en) unmittelbar aufeinander. Bei weiteren bestimmten, sehr bevorzugten Ausführungsformen ist es ferner bevorzugt, dass die umhüllende Metalloxidschicht bzw. die umhüllende chemisch nichtreaktive Kunststoffschicht unmittelbar auf der kupferhaltigen Metallpigmentoberfläche oder unmittelbar auf der Kupferoxidschicht aufgebracht sind.

Bei bestimmten bevorzugten Ausführungsformen weisen die kupferhaltigen Metallpigmente neben einer optionalen Kupferoxidschicht nur eine (Zahl: 1) umhüllende Metalloxidschicht und nur eine (Zahl: 1) umhüllende chemisch nichtreaktive Kunststoffschicht auf.

**Bestimmung Schichtdicken und Gehalt Kunststoffschicht und Metalloxidschicht**

Der Schichtdicken der Metalloxidschichten und der Kunststoffschichten auf den kupferhaltigen Metallpigmenten werden beispielsweise mittels REM-Aufnahmen an geeigneten Querschliffen ermittelt. Hierbei werden die Pigmente in einem Lack appliziert und dieser ausgehärtet. Hierbei ist auf eine möglichst gute Orientierung der Plättchen im Anwendungsmedium zu achten. Hierzu können die Metalleffektpigmente zuvor durch geeignete Additive vorbehandelt werden. Anschließend wird der ausgehärtete Lack angeschliffen und nach üblicher Probenpräparation der Querschliff im REM betrachtet. Für die Zählung werden nur Teilchen ausgewählt, die eine gute Orientierung aufweisen. Schlecht orientierte Plättchen ergeben bei dieser Methode einen hohen Fehler aufgrund des unbekannten Betrachtungswinkels. Die Beschichtungen weisen einen sehr guten Kontrast zum Metallkern auf. Sollte man die Schichtdicken der Metalloxid- und der Kunststoffschicht nicht gut unterscheiden können, so kann man örtlich aufgelöste EDX-Analysen verwenden, bevor die Schichtdicken vermessen werden. Der Begriff "mittlere Schichtdicke" im Sinne der Erfindung bezeichnet das arithmetische Mittel der Schichtdicken der Schichten von mindestens 20 Metallpigmenten. Sofern die Beschichtung ungleichmäßig ist, so wird das arithmetische Mittel der dünnsten und der dicksten Stelle der Beschichtung des jeweiligen Partikels gebildet. Einzelne gravierende Abweichungen, die beispielsweise auf dem Einschluss bereits beschichteter Feinteilpigmente in die Beschichtung beruhen, werden nicht bei die Berechnung der mittleren Schichtdicke berücksichtigt.

Der Gehalt an Metalloxid kann über eine Elementanalyse erfolgen. So kann beispielsweise im Fall einer $SiO_2$-Schicht der Si-Gehalt in Relation zum Gehalt an dem als Substrat verwendeten kupferhaltigen Metall bestimmen und dann auf $SiO_2$ hochrechnen.

**Herstellung Metalleffektpigmente:**

Vorzugsweise werden die unbeschichteten plättchenförmigen kupferhaltigen Metallpigmente durch Vermahlen von kupferhaltigem Grieß beispielsweise Kupfer- oder Messinggrieß erhalten.

Im Fall von Kupfergrieß wird vorzugsweise hochreines, elektrolytisch gewonnenes Kupfer eingesetzt. Sofern erforderlich, wird der Kupfergrieß klassiert, um eine gewünschte Größenverteilung zu erhalten.

Der kupferhaltige Grieß, beispielsweise Messinggrieß, kann eine Größenverteilung mit einem $D_{Grieß,50}$ von 1 bis 220 $\mu$m und einem $D_{Grieß,90}$ von 2 bis 470 $\mu$m aufweisen. Ein solcher Grieß wird vorzugsweise bei einer Trockenmahlung verwendet. Der kupferhaltige Grieß bspw. Messinggrieß kann auch durch Nassmahlung in plättchenförmige kupferhaltige Pigmente überführt werden. Nach der Vermahlung kann eine Klassierung erforderlich sein, um die gewünschte unbeschichtete plättchenförmige, kupferhaltige Metallpigmentfraktion zu erhalten.

Der kupferhaltige Metallgrieß kann neben Kupfer auch Zink und/oder Aluminium sowie weitere Metalle enthalten. Beispielsweise kann Messing 0,1 bis 2 Gew.-% Aluminium, bezogen auf das Gewicht des unbeschichteten kupferhaltigen Metallpigments, enthalten.

Im Fall von Messinggrieß wird vorzugsweise hochreines elektrolytisch gewonnenes Kupfer und Zink eingesetzt und vorzugsweise unter Zusatz von etwas Aluminium, wie vorstehend ausgeführt, als Reduktionsmittel legiert. Dazu werden Kupfer und Zink miteinander verschmolzen und die erzeugte Messingschmelze zu einem Messinggrieß verdüst oder zerstäubt. Der so erhaltene Messinggrieß kann dann klassiert werden, beispielsweise unter Verwendung eines Zyklons, um einen Ausgangsmessinggrieß mit einer gewünschten Größenverteilung zu erhalten.

Vorzugsweise weist der Messinggrieß eine Größenverteilung mit einem $D_{Grieß,50}$ im Bereich von 1 bis 220 $\mu$m, vorzugsweise von 2 $\mu$m bis 190 $\mu$m, mehr bevorzugt von 4 $\mu$m bis 150 $\mu$m und noch mehr bevorzugt von 6 $\mu$m bis 110 $\mu$m auf, und einen $D_{Grieß,90}$ von 2 bis 470 $\mu$m, vorzugsweise von 3 $\mu$m bis 410 $\mu$m, mehr bevorzugt von 6 bis 360 $\mu$m und noch mehr bevorzugt von 12 bis 310 $\mu$m auf.

Der kupferhaltige Metallgrieß mit der gewünschten Größenverteilung wird nachfolgend zu unbeschichteten plättchenförmigen kupferhaltigen Metalleffektpigmenten vermahlen.

Die Vermahlung von kupferhaltigem Metallgrieß, beispielsweise Kupfer- oder Messinggrieß, erfolgt überwiegend nach dem Hametagschen Trockenmahlverfahren. Hierbei wird der kupferhaltige Metallgrieß, beispielsweise Kupfer- oder Messinggrieß, in Kugelmühlen in mehreren Mahlstufen bei unterschiedlichen Mahlbedingungen, wie beispielsweise Mühlengröße, - durchmesser, -drehgeschwindigkeit, Kugelgröße, Mahldauer unter Zugabe von Schmiermittel, wie beispielsweise Stearin- oder Ölsäure, zur Verhinderung einer Kaltverschweißung der kupferhaltigen Metallpartikel, beispielsweise Kupfer- oder Messingpartikel, und mit Mahlkörpern, wie z. B. Stahlkugeln, vermahlen. Die unbeschichteten plättchenförmigen kupferhaltigen Metalleffektpigmente werden nach dem Vermahlen und optionalen Klassieren in verschiedenen Behältnissen gesammelt und anschließenden homogenisiert bzw. gemischt.

Bei einer Nassmahlung von kupferhaltigem Metallgrieß, beispielsweise Kupfer- oder Messinggrieß, wird dieser in Gegenwart von Schmier- und Lösemittel vermahlen. Eine Nassmahlung ist bevorzugt, da diese schonender als eine Trockenvermahlung ist.

Bei bestimmten bevorzugten Ausführungsformen weisen die unbeschichteten plättchenförmigen kupferhaltigen Metallpigmente gemäß einer Dickenauszählung mit Rasterelektronenmikroskopie (REM) einen $h_{50}$-Wert in einem Be-

reich von 10 bis 50 nm, bevorzugt von 15 bis 45 nm, besonders bevorzugt von 15 bis 40 nm und ganz besonders bevorzugt von 20 bis 35 nm,auf.

Weiterhin weisen die unbeschichteten plättchenförmigen kupferhaltigen Metalleffektpigmente bei bestimmten Ausführungsformen eine über Dickenauszählung mit Rasterelektronenmikroskopie (REM) ermittelte Dickenverteilung mit einem $h_{90}$-Wert von 20 bis 70 nm, bevorzugt von 20 bis 60 nm, weiter bevorzugt von 21 bis 50 nm und besonders bevorzugt von 22 bis 40 nm, auf.

Bei einer weiterhin bevorzugten Ausführungsform der Erfindung weisen die unbeschichteten plättchenförmigen kupferhaltigen Metalleffektpigmente einen $h_{10}$-Wert der Dickenverteilung im Bereich von 8 bis 25 nm und besonders bevorzugt von 10 bis 20 nm auf.

Weiterhin weisen die unbeschichteten plättchenförmigen kupferhaltigen Metalleffektpigmente bei bestimmten bevorzugten Ausführungsformen eine über Dickenauszählung mit Rasterelektronenmikroskopie (REM) ermittelte relative Breite der Dickenverteilung Δh, welche anhand der entsprechenden Summendurchgangskurve der relativen Häufigkeit nach der Formel

$$\Delta h = (h_{90}-h_{10}) \, / \, h_{50}$$

berechnet wird, von 0,3 bis 0,9, bevorzugt von 0,35 bis 0,85 und besonders bevorzugt von 0,4 bis 0,8, auf.

Ferner weisen die unbeschichteten plättchenförmigen kupferhaltigen Metalleffektpigmente in bestimmten bevorzugten Ausführungsformen Aspektverhältnis von 150 bis 3.000 auf. Bevorzugt sind die unbeschichteten plättchenförmigen kupferhaltigen Pigmente durch Aspektverhältnis von 250 bis 2.500, weiter bevorzugt von 300 bis 1.000 und besonders bevorzugt von 325 bis 600, gekennzeichnet.

Weitere Informationen zu einem hierbei einsetzbaren Vermahlungsverfahren finden sich in der WO 2009/152941 A2, auf deren Offenbarung hiermit in ihrer Gesamtheit Bezug genommen wird.

Bei einer weiteren bevorzugten Ausführungsform werden die kupferhaltigen Grießpartikel, beispielsweise Kupfer- oder Messingpartikel, in zwei Stufen vermahlen. Dabei werden in der ersten Stufe die kupferhaltigen Grießpartikel, beispielsweise Kupfer- oder Messingpartikel, vorverformt und in der zweiten Stufe bis zum Erhalt der vollständig flächig verformten unbeschichteten plättchenförmigen kupferhaltigen Metalleffektpigmente vermahlen.

Gemäß einer weiteren bevorzugten Variante der Erfindung können auch durch physikalische Dampfabscheidung erhaltene unbeschichtete Kupfereffektpigmente oder unbeschichtete Messingeffektpigmente verwendet werden, die im Folgenden auch als PVD-Kupfereffektpigmente bzw. PVD-Messingeffektpigmente bezeichnet werden. Derartige Effektpigmente sind beispielsweise in den EP 1 529 0784 B1 und EP 1 529 0785 B1 offenbart.

PVD-Kupfereffektpigmente bzw. PVD-Messingeffektpigmente weisen eine absolut plane Oberfläche auf. In diesem Fall kann durch Beschichtung mit Metalloxid und einer chemisch nichtreaktiven Kunststoffschicht keine raue Oberfläche erzeugt werden. Dieser unter Verwendung von PVD-Kupfereffektpigmenten bzw. PVD-Messingeffektpigmenten hergestellten erfindungsgemäßen unbeschichteten plättchenförmigen kupferhaltigen Metalleffektpigmente weisen nach Umhüllung mit der zweischichtigen Beschichtung gemäß der vorliegenden Erfindung gleichwohl eine hervorragende Beständigkeit gegenüber korrosiven Einflüssen aus der Umgebung auf und verhindern, dass Kupferionen in die Umgebung abgegeben werden.

Unter Verwendung von PVD-Kupfereffektpigmenten bzw. PVD-Messingeffektpigmenten bereitgestellte erfindungsgemäße plättchenförmige kupferhaltige Metallpigmente eignen sich im Hinblick auf die glatte Oberfläche insbesondere zur Verwendung in Farben, Druckfarben, Lacken und Kosmetika. Eine Verwendung der erfindungsgemäß beschichteten PVD-Kupfereffektpigmente bzw. PVD-Messingeffektpigmente in Pulverlacken ist weniger bevorzugt.

## Verwendungen

*Kosmetik*

In kosmetischen Formulierungen können die erfindungsgemäßen plättchenförmigen kupferhaltigen Metallpigmente mit für die jeweilige Anwendung geeigneten Roh-, Hilfs- und Wirkstoffen kombiniert werden. Die Konzentration der plättchenförmigen kupferhaltigen Metallpigmente in der Formulierung kann zwischen 0,001 Gew.% für Rinse-off-Produkte und 40,0 Gew.% für Leave-on-Produkte liegen.

Die erfindungsgemäßen plättchenförmigen kupferhaltigen Metallpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen plättchenförmigen kupferhaltigen Metallpigmente weitere Farbmittel und/oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige der Fa. Eckart), BiOCl-Plättchen, $TiO_2$-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen (wie z.B. die Produktgruppe MIRAGE der Fa. Eckart), $Al_2O_3$-, $SiO_2$- oder $TiO_2$-Plättchen verwendet werden. Außerdem können auch Metalleffektpigmente, wie z.B. die Produktgruppe Visionaire der Fa. Eckart, zugegeben werden. Die Farbmittel können aus anorganischen oder organischen Pigmenten ausgewählt sein.

*Beschichtungsmittel*

Die der Erfindung zugrundeliegende Aufgabe wird des Weiteren durch Bereitstellung eines Beschichtungsmittels, das erfindungsgemäße plättchenförmige kupferhaltige Metallpigmente enthält, gelöst.

Gemäß einer bevorzugten Variante der vorliegenden Erfindung ist das Beschichtungsmittel ein Lack, wie z.B. ein Coil-Coatinglack, ein Lackkonzentrat, eine Druckfarbe, ein Druckfarbenkonzentrat, eine Farbe, ein Farbkonzentrat, ein Pulverlack oder ein Pulverlackkonzentrat .

Bei den vorstehend genannten Beschichtungsmitteln ist es von großem Vorteil, dass die erfindungsgemäßen plättchenförmigen kupferhaltigen Metallpigmente keine merklichen Mengen an Kupferionen, vorzugsweise keine Kupferionen, an das Beschichtungsmittel abgeben.

Wie eingangs ausgeführt, besteht im Falle der Abgabe von Kupferionen an ein Beschichtungsmittel das Problem, dass es zum Einen zu Reduktionsreaktionen kommt, wenn Kupfer(I) in die Oxidationsstufe Kupfer(II) übergeht. Zum anderen besteht das Problem, dass Kupfer(II)-Ionen farbige Komplexe, beispielsweise stark blau gefärbte Komplexe mit Aminen, bilden.

Eine solche Verfärbung von Beschichtungsmitteln ist naturgemäß unerwünscht.

Die der Erfindung zugrundeliegende Aufgabe wird des Weiteren durch Bereitstellung eines beschichteten Gegenstandes gelöst, wobei der beschichtete Gegenstand erfindungsgemäße plättchenförmige kupferhaltige Pigmente oder ein erfindungsgemäßes Beschichtungsmittel enthält oder aufweist.

Bei den beschichteten Gegenständen kann es sich um Automobilkarosserien, Fassadenelemente, Drucksachen, wie beispielsweise bedruckte Folien, Papier, Kartonagen, Kunststoffformteile, etc. handeln.

Die der Erfindung zugrundeliegende Aufgabe wird des Weiteren durch die Verwendung der erfindungsgemäßen plättchenförmigen kupferhaltigen Metallpigmente in einem Beschichtungsmittel gelöst.

Die erfindungsgemäßen kupferhaltigen Metallpigmente erwiesen sich beispielsweise als sehr vorteilhaft in Farben und Lacken auf Basis organischer Lösungsmittel oder Wasser. Infolge ihrer herausragenden Beständigkeit erwiesen sie sich als besonders geeignet für Anwendungen, bei denen beispielsweise eine jahrelange oder jahrzentelange Farbstabilität unter belastenden Bedingungen erforderlich ist. Beispielsweise sind die erfindungsgemäßen Pigmente gut für Anwendungen, bei denen Hautkontakt besteht, und Außenanwendungen geeignet. So können beispielsweise Fassadenelemente oder Handyschalen hiermit eingefärbt werden.

Bei Farben und Lacken auf organischer Basis erwiesen sich die erfindungsgemäßen Pigmenten als sehr vorteilhaft, da hiermit beispielsweise eine ausgezeichnete Langzeitstabilität erzielt werden konnte. Dies scheint auf das zuverlässige Einschließen der Kupferionen zurückzuführen zu sein, wodurch ungewünschte Reaktionen der Lackbestandteile vermieden werden. Jedoch erwies sich auch der Einsatz in wasserbasierenden Farben und Lacken als sehr vorteilhaft, bei denen eine zuverlässige und dauerstabile Einfärbung erzielt wurde. Es wird vermutet, dass die besonders gute Wasserstabilität der erfindungsgemäßen kupferhaltigen Metallpigmente eine Reaktion des Kupfers mit dem Wasser und somit eine Farbveränderung langfristig verhindert. Die erfindungsgemäßen kupferhaltigen Metallpigmente sind daher beispielsweise besonders gut zur Verwendung in wässrigen Dispersionsfarben wie Wandfarben geeignet. Insbesondere Anwendungsformen wie die Applikation als Wandfarbe profitieren von der Langzeitstabilität auch in ihrer applizierten Form. So wird beispielsweise auch bei feuchten Wänden keine Verfärbung ins grünliche oder bläuliche infolge austretender Kupferionen beobachtet.

Bevorzugte die erfindungsgemäßen plättchenförmigen kupferhaltigen Metallpigmente enthaltende Beschichtungsmittel sind Wasserlacke, Pulverlacke, Nagellacke, Polymere und Coil-Coating-Formulierungen. Bei bestimmten bevorzugten Ausführungsformen ist das Beschichtungsmittel ein Pulverlack, eine Nagellackkomposition oder einem Lack zur Verwendung im Coil-Coating-Verfahren.

*Pulverlack*

Pulverlacke werden beispielsweise in der industriellen Serienfertigung zur Beschichtung von elektrisch leitfähigen und temperaturbeständigen Werkstoffen verwendet. Der zu applizierende Pulverlack liegt hierbei als festes und lösungsmittelfreies Pulver vor. Ferner sind die als Grundierung oder Einschichtdecklack eingesetzten Pulverlacke fast vollständig recycelbar. Die umweltfreundlichen und vielseitig einsetzbaren Pulverlacke enthalten Bindemittel, Pigmente, Füllstoffe und Vernetzer sowie optional Additive. Unter einem Bindemittel wird erfindungsgemäß die in der DIN 55 945 angegebene Definition verstanden. D.h. das Bindemittel umfaßt sowohl den Filmbildner wie auch nicht flüchtige Hilfsstoffe wie Weichmacher und Trockenstoffe. Eine Auftragung der feinpulvrigen Pulverlacke erfolgt in der Regel elektrostatisch, bevor sie durch Einbrennen oder durch Strahlungsenergie gehärtet werden.

Zur Pigmentierung der Pulverlacke können unter anderem Metalleffektpigmente eingesetzt werden. Bei mittels Mischverfahren hergestellten Pulverlacken kann es sich jedoch als problematisch erweisen, dass durch die beim Extrusions- und Vermahlungsprozess auf die Pigmentplättchen einwirkenden Scherkräfte eine Beschädigung oder Zerstörung der Pigmentplättchen auftreten kann. Hierdurch kann insbesondere der Glanz und somit auch die Optik derart pigmentierter Anwendungen negativ beeinträchtigt werden.

Aus diesem Grund werden beispielsweise im Dry-blend-Verfahren die Metalleffektpigmente dem Basispulverlack erst nach der Vermahlung zugemischt. Dies hat jedoch den Nachteil, dass während der Lackapplikation eine mögliche Separation von Pigment und Pulverlack durch das unterschiedliche Aufladeverhalten der einzelnen Lackbestandteile auftritt. Hieraus folgt ein unregelmäßiger optischer Effekt als der Ab- oder Anreicherung von Pigment während der Pulverlackapplikation. Ferner führt die Separierung von Pigment und Bindemittel zu einer veränderten Zusammensetzung des "Oversprays". Alternativ wird das sogenannte Bonding-Verfahren eingesetzt, bei dem das Pigment unter Erwärmen an den Partikeln des Basislackes fixiert wird. Die Herstellung derartiger Bonding-Pulverlacken ist jedoch verhältnismäßig kostenaufwendig. Die Herstellung der zurzeit kostengünstigsten Pulverlacke erfolgt mittels Mischverfahren. Hierbei werden die Pigmente zusammen mit allen anderen Rohstoffen vermischt, extrudiert und vermahlen.

Da Pulverlack-beschichtete Substrate nach der Pulverlackapplikation in einem Ofen Temperaturen von 200°C ausgesetzt werden, führt es dazu, dass kupferhaltige Metallpigmente oxidiert werden, welches sich durch eine unerwünschte Farbänderung bemerkbar macht. Es hat sich gezeigt, dass auch eine sehr dicke Kunststoffschicht nicht in der Lage ist die Oxidation zu unterbinden. Allerdings bewirkt eine derartige Kunststoffschicht eine gute Chemikalienstabilität. Ferner hat sich gezeigt, dass eine Metalloxidschicht wirksam die Oxidation verhindert. Jedoch ist die Chemikalienstabilität einer derartigen Schicht nicht ausreichend. Das Aufbringen beider Schichten in üblichen Mengenverhältnissen führt jedoch zu einer gravierenden Verschlechterung der optischen Eigenschaften. Überraschenderweise hat sich jedoch gezeigt, dass durch die erfindungsgemäß aufzubringende Beschichtung die gewünschten Stabilitäten erreicht werden, während die optischen Eigenschaften des Pigments nahezu oder vollständig erhalten bleiben.

*Coil-Coating*

Auch das Coil-Coating ist als sehr umweltfreundliches Beschichtungsverfahren bekannt. Beschichtung und Trocknung finden hierbei kontinuierlich in einem geschlossenen System statt, wobei im no-rinse Verfahren zudem das Abspülen von Chemikalienresten entfällt. Ferner kann durch eine optimierte Prozessführung ein Auftragswirkungsgrad von nahezu 100 % erreicht werden, während ansonsten bei den meisten Lackierverfahren beispielsweise größere Verluste durch das over spraying vorhanden sind. Da jedoch der Lack beim Coil-Coating bei Temperaturen von 240 bis 280°C eingebrannt wird, werden auch hier Oxidationsphänomene bei herkömmlichen kupferhaltigen Metallpigmenten analog zur Pulverbeschichtung beobachtet. Die oben diskutierten Probleme und Beobachtungen in Bezug auf den Pulverlack gelten daher auch für das Coil-coating.

*Nagellack*

Unbeschichtete kupferhaltige Pigmente setzten sehr leicht Kupferionen frei, welche z.B. in Nitrocelluloselacken zu einer Grünfärbung führen. Darüber hinaus setzen sich die Pigmente mit der Zeit am Boden ab und können schon nach wenigen Tagen nicht mehr aufgeschüttelt bzw. redispergiert werden. Somit kann der Nagellack nach wenigen Tagen nicht mehr verwendet werden. Eine Metalloxidschicht kann die Freisetzung von Kupferionen etwas eindämmen, allerdings können keine zufriedenstellenden Lagerstabilitäten erreicht werden. Überraschenderweise wurde jedoch beobachtet, dass plättchenförmige kupferhaltige Metallpigmente, die erfindungsgemäß mit wenigstens einer Kunststoffschicht und wenigstens einer Metalloxidschicht beschichtet sind, Lagerstabilitäten über 6 Monate aufweisen, ohne dass eine merkliche Verschlechterung der optischen Qualitäten, verglichen mit dem unbeschichteten Pigment, auftritt. Innerhalb der Lagerzeit, konnten die erfindungsgemäßen plättchenförmige kupferhaltige Pigmente, welche sich am Boden abgesetzt haben, immer wieder aufgeschüttelt bzw. leicht redispergiert werden. Außerdem zeigte sich während der Lagerzeit keine Grünfärbung.

*Polymere*

In Polymere eingearbeitete kupferhaltige Metallpigmente, insbesondere plättchenförmige kupferhaltige Metallpigmente, werden während der Verarbeitung oft oxidiert, wodurch sich beispielsweise die Farbe ändert. Dies kann auch durch Änderungen der Verfahrensbedingungen nur bedingt verhindert werden, wodurch beispielsweise die Reproduktion eines gewünschten Farbtons nahezu unmöglich wird. Überraschenderweise wurde gefunden, dass die erfindungsgemäßen plättchenförmigen kupferhaltigen Metallpigmente eine hinreichende Stabilität aufweisen, um unter Standardbedingungen eine Verarbeitung zu ermöglichen, so dass keine merkliche oder gar keine Veränderung des Farbtons der erfindungsgemäßen plättchenförmigen kupferhaltigen Metallpigmente beobachtet wird.

Die verwendeten Polymere umfassen hierbei vorzugsweise thermoplastische, duroplastische oder elastomere Polymere. Besonders bevorzugt sind hierbei thermoplastische Polymere.

Als thermoplastische Polymere kommen alle dem Fachmann bekannten Thermoplaste in Betracht. Geeignete thermoplastische Polymere werden beispielsweise im Kunststoff-Taschenbuch, Hrsg. Saechtling, 25. Ausgabe, Hanser-Verlag, München, 1992, insbesondere Kapitel 4 sowie darin zitierte Verweise, und im Kunststoff-Handbuch, Hrsg. G. Becker und D. Braun, Bände 1 bis 11, Hanser-Verlag, München, 1966 bis 1996, beschrieben.

Exemplarisch seien als geeignete Thermoplaste genannt: Polyoxyalkylene, Polycarbonate (PC), Polyester wie Polybutylenterephthalat (PBT) oder Polyethylenterephthalat (PET), Polyolefine wie Polyethylen oder Polypropylen (PP), Poly(meth)acrylate, Polyamide, vinylaromatische (Co)polymere wie Polystyrol, schlagzähmodifiziertes Polystyrol wie HI-PS, oder ASA-, ABS- oder AES-Polymerisate, Polyarylenether wie Polyphenylenether (PPE), Polysulfone, Polyurethane, Polylactide, halogenhaltige Polymerisate, imidgruppenhaltige Polymere, Celluloseester, Silicon-Polymere oder thermoplastische Elastomere. Es können auch Mischungen unterschiedlicher Thermoplaste als Materialien für die Polymerformteile eingesetzt werden. Bei diesen Mischungen kann es sich um ein- oder mehrphasige Polymerblends handeln.

Die Polymere können aus identischen oder verschiedenen Thermoplasten bzw. Thermoplast-Blends bestehen.

Polyoxyalkylenhomo- oder -copolymerisate, insbesondere (Co)polyoxymethylene (POM), und Verfahren zu deren Herstellung sind dem Fachmann an sich bekannt und in der Literatur beschrieben. Geeignete Materialien sind im Handel unter der Markenbezeichnung Ultraform® (BASF AG, Deutschland) erhältlich. Ganz allgemein weisen diese Polymere mindestens 50 Mol-% an wiederkehrenden Einheiten -CH$_2$O-in der Polymerhauptkette auf. Die Homopolymeren werden im Allgemeinen durch Polymerisation von Formaldehyd oder Trioxan hergestellt, vorzugsweise in der Gegenwart von geeigneten Katalysatoren. Bevorzugt sind Polyoxymethylencopolymere und Polyoxymethylenterpolymerisate. Die bevorzugten Polyoxymethylen(co)polymere haben Schmelzpunkte von mindestens 150°C und Molekulargewichte (Gewichtsmittelwert) M im Bereich von 5.000 bis 200.000, vorzugsweise von 7.000 bis 150.000 g/mol. Endgruppenstabilisierte Polyoxymethylenpolymerisate, die an den Kettenenden C-C-Bindungen aufweisen, werden besonders bevorzugt.

Geeignete Polycarbonate sind an sich bekannt und sind z.B. gemäß DE-B-1 300 266 durch Grenzflächenpolykondensation oder gemäß DE-A-14 95 730 durch Umsetzung von Biphenylcarbonat mit Bisphenolen erhältlich. Bevorzugtes Bisphenol ist 2,2-Di(4-hydroxyphenyl)propan, im Allgemeinen als Bisphenol A bezeichnet. Die relative Viskosität dieser Polycarbonate liegt im Allgemeinen im Bereich von 1,1 bis 1,5, insbesondere 1,28 bis 1,4 (gemessen bei 25°C in einer 0,5 Gew.-%igen Lösung in Dichlormethan). Geeignete Polycarbonate sind im Handel unter der Markenbezeichnung Lexan® (GE Plastics B.V., Holland) erhältlich.

Geeignete Polyester sind ebenfalls an sich bekannt und in der Literatur beschrieben. Sie enthalten einen aromatischen Ring in der Hauptkette, der von einer aromatischen Dicarbonsäure herrührt. Der aromatische Ring kann auch substituiert sein, z.B. durch Halogen wie Chlor und Brom oder durch C$_1$-C$_4$-Alkylgruppen wie Methyl-, Ethyl-, i- bzw. n-Propyl- und n-, i- bzw. tert.-Butylgruppen. Die Polyester können durch Umsetzung von aromatischen Dicarbonsäuren, deren Estern oder anderer esterbildender Derivate derselben mit aliphatischen Dihydroxyverbindungen in an sich bekannter Weise hergestellt werden. Als bevorzugte Dicarbonsäuren sind Naphthalindicarbonsäure, Terephthalsäure und Isophthalsäure oder deren Mischungen zu nennen. Bis zu 10 mol% der aromatischen Dicarbonsäuren können durch aliphatische oder cycloaliphatische Dicarbonsäuren wie Adipinsäure, Azelainsäure, Sebacinsäure, Dodecandisäuren und Cyclohexandicarbonsäuren ersetzt werden. Von den aliphatischen Dihydroxyverbindungen werden Diole mit 2 bis 6 Kohlenstoffatomen, insbesondere 1,2-Ethandiol, 1,4-Butandiol, 1,6-Hexandiol, 1,4-Hexandiol, 1,4-Cyclohexaridiol und Neopentylglykol oder deren Mischungen bevorzugt. Als besonders bevorzugte Polyester sind Polyalkylenterephthalate, die sich von Alkandiolen mit 2 bis 6 C-Atomen ableiten, zu nennen. Von diesen werden insbesondere Polyethylenterephthalat (PET), Polyethylennaphthalat und Polybutylenterephthalat (PBT) bevorzugt. Diese Produkte sind im Handel z.B. unter den Markenbezeichnungen Rynite® (PET; Fa. DuPont, USA) bzw. Ultradur® (PBT; BASF AG) erhältlich. Die Viskositätszahl der Polyester liegt im Allgemeinen im Bereich von 60 bis 200 ml/g (gemessen in einer 0,5 Gew.-%igen Lösung in einem Phenol/o-Dichlorbenzol-Gemisch (Gew.-Verh. 1:1 bei 25°C)).

Geeignete Polyolefine stellen ganz allgemein Polyethylen und Polypropylen sowie Copolymerisate auf der Basis von Ethylen oder Propylen, ggf. auch mit höheren α-Olefinen, dar. Entsprechende Produkte sind z.B. unter den Handelsnamen Lupolen® bzw. Novolen® erhältlich. Unter Polyolefinen sollen auch Ethylen-Propylen-Elastomere und Ethylen-Propylen-Terpolymere verstanden werden.

Unter den Poly(meth)acrylaten sind insbesondere Polymethylmethacrylat (PMMA) sowie Copolymere auf der Basis von Methylmethacrylat mit bis zu 40 Gew.-% weiterer copolymerisierbarer Monomeren, wie n-Butylacrylat, t-Butylacrylat oder 2-Ethylhexylacrylat, zu nennen, wie sie beispielsweise unter den Bezeichnungen Lucryl® (BASF AG) oder Plexiglas® (Röhm GmbH, Deutschland) erhältlich sind. Im Sinne der Erfindung sind hierunter auch schlagzähmodifizierte Poly(meth)acrylate sowie Mischungen aus Poly(meth)acrylaten und SAN-Polymerisaten, die mit Polyacrylatkautschuken schlagzäh modifiziert sind (z.B. das Handelsprodukt Terlux® der BASF AG), zu verstehen.

Als Polyamide sind solche geeignet mit aliphatischem teilkristallinen oder teilaromatischen oder amorphem Aufbau jeglicher Art und deren Blends, einschließlich Polyetheramiden wie Polyetherblockamiden. Unter Polyamiden sollen alle bekannten Polyamide verstanden werden. Geeignete Polyamide weisen im Allgemeinen eine Viskositätszahl von 90 bis 350, vorzugsweise 110 bis 240 ml/g auf (bestimmt in einer 0,5 Gew.-%-igen Lösung in 96 Gew.-%iger Schwefelsäure bei 25°C gemäß ISO 307). Halbkristalline oder amorphe Harze mit einem Molekulargewicht (Gewichtsmittelwert) von mindestens 5.000 g/mol, wie sie z.B. in den US Patentschriften 2 071 250, 2 071 251, 2 130 523, 2 130 948, 2 241 322, 2 312 966, 2 512 606 und 3 393 210 beschrieben werden, sind bevorzugt.

Beispiele hierfür sind Polyamide, die sich von Lactamen mit 7 bis 13 Ringgliedern ableiten, wie Polycaprolactam, Polycapryllactam und Polylaurinlactam, sowie Polyamide, die durch Umsetzung von Dicarbonsäuren mit Diaminen erhalten werden.

Als Dicarbonsäuren sind Alkandicarbonsäuren mit 6 bis 12, insbesondere 6 bis 10 Kohlenstoffatomen und aromatische Dicarbonsäuren einsetzbar. Hier seien Adipinsäure, Azelainsäure, Sebacinsäure, Dodecandisäure (= Decandicarbonsäure) und und/oder Isophthalsäure als Säuren genannt.

Als Diamine eignen sich besonders Alkandiamine mit 6 bis 12, insbesondere 6 bis 8 Kohlenstoffatomen sowie m-Xylylendiamin, Di-(4-aminophenyl)methan, Di-(4-aminocyclohexyl)-methan, 2,2-Di-(4-aminophenyl)-propan oder 2,2-Di- (4-aminocyclohexyl)-propan.

Bevorzugte Polyamide sind Polyhexamethylenadipinsäureamid (PA 66), z.B. das Handelsprodukt Ultramid® A (BASF AG), und Polyhexamethylensebacinsäureamid (PA 610), z.B. das Handelsprodukt Nylon® 610 (Fa. DuPont), Polycaprolactam (PA 6), z.B. das Handelsprodukt Ultramid® B (BASF AG) sowie Copolyamide 6/66, insbesondere mit einem Anteil von 5 bis 95 Gew.-% an Caprolactam-Einheiten, z.B. das Handelsprodukt Ultramid® C (BASF AG). PA 6, PA 66 und Copolyamide 6/66 sind besonders bevorzugt.

Außerdem können auch Polyamide verwendet werden, die z.B. durch Kondensation von 1,4-Diaminobutan mit Adipinsäure unter erhöhter Temperatur erhältlich sind (Polyamid-4,6). Herstellungsverfahren für Polyamide dieser Struktur sind z.B. in den EP-A 38 094, EPA 38 582 und EP-A 39 524 beschrieben.

Weitere Beispiele sind Polyamide, die durch Copolymerisation zweier oder mehrerer der vorgenannten Monomeren erhältlich sind, oder Mischungen mehrerer Polyamide, wobei das Mischungsverhältnis beliebig ist.

Weiterhin haben sich solche teilaromatischen Copolyamide wie PA 6/6T und PA 66/6T als besonders vorteilhaft erwiesen, deren Triamingehalt weniger als 0,5, vorzugsweise weniger als 0,3 Gew.-% beträgt (siehe EP-A 299 444). Die Herstellung der teilaromatischen Copolyamide mit niedrigem Triamingehalt kann nach den in den EP-A 129 195 und 129 196 beschriebenen Verfahren erfolgen.

Weitere geeignete thermoplastische Materialien stellen vinylaromatische (Co)polymere dar. Das Molekulargewicht dieser an sich bekannten und im Handel erhältlichen Polymeren liegt im allgemeinen im Bereich von 1.500 bis 2.000.000, vorzugsweise im Bereich von 70.000 bis 1.000.000 g/mol.

Beispielhaft werden vinylaromatische (Co)polymere aus Styrol, Chlorstyrol, $\alpha$-Methylstyrol und p-Methylstyrol genannt; in untergeordneten Anteilen, vorzugsweise nicht mehr als 20 Gew.-%, insbesondere nicht mehr als 8 Gew.-%, können auch Comonomere wie (Meth)acrylnitril oder (Meth)acrylsäureester am Aufbau beteiligt sein. Besonders bevorzugte vinylaromatische (Co)polymere sind Polystyrol, Styrol-Acrytnitril-Copolymere (SAN) und schlagzähmodifiziertes Polystyrol (HIPS = High Impact Polystyrene). Es versteht sich, dass auch Mischungen dieser Polymeren eingesetzt werden können. Die Herstellung erfolgt vorzugsweise nach dem in EP-A-302 485 beschriebenen Verfahren.

Weiterhin sind ASA-, ABS- und AES-Polymerisate (ASA = Acrylnitril-Styrol-Acrylester, ABS = Acrylnitril-Butadien-Styrol, AES = Acrylnitril-EPDM-Kautschuk-Styrol) besonderes bevorzugt. Diese schlagzähen vinylaromatischen Polymere enthalten mindestens ein kautschukelastisches Pfropfpolymerisat und ein thermoplastisches Polymerisat (Matrixpolymerisat). Als Matrixmaterial wird im Allgemeinen auf ein Styrol/Acrylnitril-Polymerisat (SAN) zurückgegriffen. Bevorzugt werden Pfropfpolymerisate verwendet, die als Kautschuk

- einen Dienkautschuk auf Basis von Dienen, wie z.B. Butadien oder Isopren, (ABS);
- einen Alkylacrylatkautschuk auf Basis von Alkylestern der Acrylsäure, wie n-Butylacrylat und 2-Ethylhexylacrylat, (ASA);
- einen EPDM-Kautschuk auf Basis von Ethylen, Propylen und einem Dien, (AES);

oder Mischungen dieser Kautschuke bzw. Kautschukmonomeren enthalten.

Die Herstellung von geeigneten ABS-Polymerisaten findet sich z.B. in der deutschen Patentanmeldung DE-A 19728629 eingehend beschrieben. Für die Herstellung von ASA-Polymerisaten kann z.B. auf die EP-A 99 532 zurückgegriffen werden. Angaben über die Herstellung von AES-Polymerisaten sind beispielsweise in der US 3,055,859 oder in der US 4,224,419 offenbart. Auf die in diesem Absatz genannten Patentschriften wird hiermit ausdrücklich Bezug genommen.

Unter Polyarylenethern sind bevorzugt sowohl Polyarylenether an sich, Polyarylenethersulfide, Polyarylenethersulfone oder Polyarylenetherketone zu verstehen. Deren Arylengruppen können gleich oder verschieden sein und unabhängig voneinander einen aromatischen Rest mit 6 bis 18 C-Atomen bedeuten. Beispiele geeigneter Arylenreste sind Phenylen, Bisphenylen, Terphenylen, 1,5-Naphthylen, 1,6-Naphthylen, 1,5-Anthrylen, 9,10-Anthrylen oder 2,6-Anthrylen. Darunter werden 1,4-Phenylen und 4,4'-Biphenylen bevorzugt. Vorzugsweise sind diese aromatischen Reste nicht substituiert. Sie können jedoch einen oder mehrere Substituenten tragen. Geeignete Polyphenylenether sind unter der Bezeichnung Noryl® (GE Plastics B.V., Holland) kommerziell erhältlich.

Im Allgemeinen weisen die Polyarylenether mittlere Molekulargewichte M (Zahlenmittel) im Bereich von 10.000 bis 60.000 g/mol und Viskositätszahlen von 30 bis 150 ml/g auf. Die Viskositätszahlen werden je nach Löslichkeit der Polyarylenether entweder in 1 Gew.-%iger N-Methylpyrrolidon-Lösung, in Mischungen aus Phenol und o-Dichlorbenzol oder in 96%-iger Schwefelsäure bei jeweils 20°C bzw. 25°C gemessen.

Die Polyarylenether sind an sich bekannt oder können nach an sich bekannten Methoden hergestellt werden.

Bevorzugte Verfahrensbedingungen zur Synthese von Polyarylenethersulfonen oder -ketonen sind beispielsweise in der EP-A 113 112 und EP-A 135 130 beschrieben. Polyarylenethersulfone weisen in der Regel einen Schmelzpunkt von mindestens 320°C, Polyarylenetherketone von mindestens 370°C auf. Geeignete Polyphenylenethersulfone sind z.B. unter der Bezeichnung Ultrason® E (BASF AG), geeignete Polyphenylenetherketone unter der Bezeichnung Victrex® im Handel erhältlich.

Des Weiteren sind Polyurethane, Polyisocyanurate und Polyharnstoffe zur Einfärbung mit erfindungsgemäßen plättchenförmigen kupferhaltigen Metallpigmenten geeignet. Weiche, halbharte oder harte, thermoplastische oder vernetze Polyisocyanat-Polyadditionsprodukte, beispielsweise Polyurethane, Polyisocyanurate und/oder Polyharnstoffe, insbesondere Polyurethane, sind allgemein bekannt und im Handel u. a. unter der Bezeichnung Elastolan® (Elastogran GmbH, Deutschland) erhältlich. Ihre Herstellung ist vielfältig beschrieben und erfolgt üblicherweise durch Umsetzung von Isocyanaten mit gegenüber Isocyanaten reaktiven Verbindungen bei allgemein bekannten Bedingungen. Bevorzugt wird die Umsetzung in Gegenwart von Katalysatoren und/oder Hilfsstoffen durchgeführt. Wenn es sich um geschäumte Polyisocyanat-Polyadditionsprodukte handelt, so werden diese in Gegenwart von üblichen Treibmitteln hergestellt.

Als Isocyanate kommen die an sich bekannten aromatischen, arylaliphatischen, aliphatischen und/oder cycloaliphatischen organischen Isocyanate, bevorzugt Diisocyanate in Frage.

Als gegenüber Isocyanaten reaktive Verbindungen können beispielsweise allgemein bekannte Verbindungen mit einem Molekulargewicht von 60 bis 10.000 g/mol und einer Funktionalität gegenüber Isocyanaten von 1 bis 8, bevorzugt 2 bis 6 eingesetzt werden (im Falle von thermoplastischen Polyurethanen TPU Funktionalität ca. 2), beispielsweise Polyole mit einem Molekulargewicht von 500 bis 10.000 g/mol, z.B. Polyetherpolyole, Polyesterpolyole, Polyetherpolyesterpolyole, und/oder Diole, Triole und/oder Polyole mit Molekulargewichten kleiner 500 g/mol.

Polylactide, also Polymere der Milchsäure, sind an sich bekannt oder können nach an sich bekannten Verfahren hergestellt und ebenfalls in Verbindung mit dem erfindungsgemäßen plättchenförmigen kupferhaltigen Metallpigmenten verwendet werden. Neben Polylactid können auch Co- oder Blockcopolymere auf der Basis von Milchsäure und weiteren Monomeren verwendet werden. Meist werden lineare Polylactide eingesetzt. Es können aber auch verzweigte Milchsäurepolymerisate verwendet werden. Als Verzweiger können z.B. mehrfunktionelle Säuren oder Alkohole dienen.

Als geeignete halogenhaltige Polymerisate sind insbesondere Polymerisate des Vinylchlorids zu nennen, insbesondere Polyvinylchlorid (PVC) wie Hart-PVC und Weich-PVC, und Copolymerisate des Vinylchlorids wie PVC-U-Formmassen.

Weiterhin kommen fluorhaltige Polymere in Betracht, insbesondere Polytetrafluorethylen (PTFE), Tetrafluorethylen-PerfluorpropylenCopolymere (FEP), Copolymere des Tetrafluorethylens mit Perfluoralkylvinylether, Ethylen-Tetrafluorethylen-Copolymere (ETFE); Polyvinylidenfluorid (PVDF), Polyvinylfluorid (PVF), Polychlortrifluorethylen (PCTFE), und Ethylen-Chlortrifluorethylen-Copolymere (ECTFE).

Imidgruppenhaltige Polymere sind insbesondere Polyimide, Polyetherimide, und Polyamidimide.

Geeignete Celluloseester sind etwa Celluloseacetat, Celluloseacetobutyrat, und Cellulosepropionat.

Daneben kommen auch Silicon-Polymere als Thermoplaste in Betracht. Geeignet sind insbesondere Siliconkautschuke. Dabei handelt es sich üblicherweise um Polyorganosiloxane, die zu Vernetzungsreaktionen fähige Gruppen aufweisen.

Derartige Polymere werden beispielsweise in Römpp Chemie Lexikon, CD-ROM Version 1.0, Thieme Verlag Stuttgart 1995, beschrieben.

Die erfindungsgemäßen plättchenförmigen kupferhaltigen Metallpigmente können ebenfalls in thermoplastische Elastomere (TPE) eingebracht werden. TPE lassen sich wie Thermoplaste verarbeiten, haben jedoch kautschukelastische Eigenschaften. Es sind TPE-Blockpolymere, TPE-Pfropfpolymere und segmentierte TPE-Copolymere aus zwei oder mehr Monomerbausteinen geeignet. Besonders geeignete TPE sind thermoplastische Polyurethanelastomere (TPE-U oder TPU), Styrol-Oligoblock-Copolymere (TPE-S) wie SBS (Styrol-Butadien-Styrol-oxyBlockcopolymer) und SEES (Styrol-Ethylen-Butylen-Styrol-Blockcopolymer, erhältlich durch Hydrieren von SBS), thermoplastische Polyolefin-Elastomere (TPE-O), thermoplastische Polyester-Elastomere (TPE-E), thermoplastische Polyamid-Elastomere (TPE-A) und insbesondere thermoplastische Vulkanisate (TPE-V). Einzelheiten zu TPE findet der Fachmann in G. Holden et al., Thermoplastic Elastomers, 2. Auflage, Hanser Verlag, München 1996.

Zusätzlich zu den erfindungsgemäßen plättchenförmigen kupferhaltigen Metalleffektpigmenten können in den Polymeren weiterhin übliche Zusatzstoffe enthalten sein. Diese Zusatzstoffe können beispielsweise aus der Gruppe, die aus Füllstoffen, Additiven, Weichmachern, Gleit- oder Entformungsmitteln, Schlagzähigkeitsverbesserern, Farbpigmenten, Farbstoffen, Flammschutzmitteln, Antistatika, optischen Aufhellern, Antioxidantien, antimikrobiell wirksame Biostabilisatoren, chemischen Treibmitteln oder organischen Vernetzungsmitteln sowie andere Zusatzstoffen und Mischungen davon besteht, ausgewählt werden.

Es wurde gefunden, dass der farbgebende Effekt unterhalb von 0,1 Gew.-% der erfindungsgemäßen plättchenförmigen kupferhaltigen Metallpigmente, bezogen auf das Gesamtgewicht des Metallpigment enthaltenden Polymers, deutlich schwächer ausgeprägt ist. Ferner wurde gefunden, dass die mechanische Festigkeit oberhalb von 10 Gew.-%, bezogen auf das Gesamtgewicht des Metallpigment enthaltenden Polymers, abnimmt. Gemäß bestimmten bevorzugten Ausführungsformen beträgt der Anteil der erfindungsgemäßen plättchenförmigen kupferhaltigen Metallpigmente im Polymer 0,01 bis 10 Gew.-%, vorzugsweise 0,1 bis 8 Gew.-%, jeweils bezogen auf das Gesamtgewicht des Metallpigment enthaltenden Polymers. Weiterhin ist bevorzugt, dass der Anteil der erfindungsgemäßen plättchenförmigen kupferhaltigen Metallpigmente in dem Polymer bei 0,25 bis 5 Gew.-%, noch weiter bevorzugt 0,5 bis 2,5 Gew.-%, jeweils bezogen auf das Gesamtgewicht des Metallpigment enthaltenden Polymers beträgt.

Die das erfindungsgemäße plättchenförmige kupferhaltige Metallpigment enthaltenden Kunststoffe können auch bei der Lasermarkierung verwendet werden. Der Laserstrahl erwärmt bei Bestrahlung die getroffenen erfindungsgemäßen plättchenförmigen kupferhaltigen Metallpigmente, die sodann zu einer sichtbaren Veränderung des die Metallpigmente umgebenden Kunststoffs führen.

Dabei wurde festgestellt, dass die Möglichkeit der Lasermarkierung unterhalb von 0,0005 Gew.-%, bezogen auf das Gesamtgewicht des das erfindungsgemäße plättchenförmige kupferhaltige Metallpigment enthaltenden Polymers, stark nachlässt. Andererseits werden die optischen Eigenschaften des Polymers bei einer Konzentration von mehr als 0,7 Gew.-%, bezogen auf das Gesamtgewicht des das erfindungsgemäße plättchenförmige kupferhaltige Metallpigment enthaltenden Polymers, bereits stark beeinflusst. Gemäß bestimmten bevorzugten Ausführungsformen beträgt der Anteil der plättchenförmigen kupferhaltigen Metallpigmente im Polymer daher 0,0005 bis 0,7 Gew.-%, vorzugsweise 0,001 bis 0,5 Gew.-%, jeweils bezogen auf das Gesamtgewicht des Metallpigment enthaltenden Polymers. Weiterhin ist es bei bestimmten Ausführungsformen bevorzugt, dass der Anteil der erfindungsgemäßen plättchenförmigen kupferhaltigen Metallpigmente 0,005 bis 0,5 Gew.-%, noch weiter bevorzugt 0,01 bis 0,1 Gew.-%, jeweils bezogen auf das Gesamtgewicht des Metallpigment enthaltenden Polymers, beträgt.

## Verfahren:

Die Aufgabe der Erfindung wird des Weiteren durch Bereitstellung eines Verfahrens zur Herstellung eines erfindungsgemäßen kupferhaltigen Pigmentes gelöst, wobei das Verfahren die folgenden Schritte umfasst:

(1a) Beschichten von plättchenförmigen kupferhaltigen Metallpigmenten mit Metalloxid,

(1b) Beschichten der in Schritt (1a) erhaltenen metalloxidbeschichteten plättchenförmigen kupferhaltigen Metallpigmenten mit dem (den) Edukt(en) der chemisch nichtreaktiven Kunststoffschicht,

(1c) Aushärten oder Auspolymerisieren der in Schritt (1b) mit dem (den) Edukt(en) der chemisch nichtreaktiven Kunststoffschicht beschichteten kupferhaltigen Metallpigmente

oder

(2a) Beschichten von plättchenförmigen kupferhaltigen Metallpigmenten mit dem (den) Edukt(en) der chemisch nichtreaktiven Kunststoffschicht

(2b) Aushärten oder Auspolymerisieren der in Schritt (2a) mit dem (den) Edukt(en) der chemisch nichtreaktiven Kunststoffschicht beschichteten plättchenförmigen kupferhaltigen Metallpigmente

(2c) Beschichten der in Schritt (2b) erhaltenen mit chemisch nichtreaktiven Kunststoffschicht beschichteten plättchenförmigen kupferhaltigen Metallpigmente mit Metalloxid.

Die in Schritt (1c) oder Schritt (2b) erhaltenen plättchenförmigen kupferhaltigen Metallpigmenten weisen dann eine chemisch nichtreaktive Kunststoffschicht auf.

Bei bestimmten bevorzugten Ausführungsformen werden die vorgenannten Beschichtungen mit Metalloxid und/oder der chemisch nichtreaktiven Kunststoffschicht wiederholt oder mit anderen Metalloxiden bzw. Edukten einer chemisch nichtreaktiven Kunststoffschicht ausgeführt, um eine Beschichtungen mit mehreren Metalloxidschichten und/oder chemisch nichtreaktiven Kunststoffschichten bereitzustellen.

Das Beschichten der plättchenförmigen kupferhaltigen Metallpigmente mit Metalloxid kann auf herkömmliche Art und Weise vorgenommen werden. Beispielsweise können die Metalloxide unter Hydrolyse entsprechender Metallsalze, wie beispielsweise Metallhalogenide, insbesondere der Metallchloride, aufgebracht werden.

Vorzugsweise werden die Metalloxidschichten mittels Sol-Gel-Verfahren aufgebracht. Hierbei werden die entsprechenden Metallalkoxide unter Zugabe von Wasser, sowie vorzugsweise Säuren oder Basen als Katalysatoren hydrolysiert, wobei die entsprechenden Metalloxide und/oder Metalloxidhydrate auf die kupferhaltigen Metallpigmente auffallen und diese ummanteln.

Bei den Alkoxygruppen handelt es sich vorzugsweise um Methoxy-, Ethoxy-, Propoxy-, Butoxy- und/oder Pentoxygruppen. Äußerst bevorzugt handelt es sich bei den Alkoxygruppen um Methoxy- und/oder Ethoxygruppen.

Die Beschichtung mit Metalloxid mittels Sol-Gel-Verfahren erfolgt üblicherweise in organischem Lösungsmittel in Gegenwart von geringen Mengen an Wasser, wie beispielsweise 1 bis 10 Vol.-%, vorzugsweise 2 bis 5 Vol.-%, Wasser, bezogen auf das Gesamtvolumen des wasserhaltigen organischen Lösungsmittels.

Als organische Lösemittel werden vorzugsweise Alkohole, Glykole, Ester, Ketone sowie Mischungen dieser Lösemittel verwendet. Als besonders geeignet ist die Verwendung von Alkoholen, Glykolen oder deren Mischungen. Besonders bevorzugt werden Alkohole verwendet.

Der Alkohol wird vorzugsweise aus der Gruppe, die aus Methanol, Ethanol, Isopropanol, n-Propanol, t-Butanol, n-Butanol, Isobutylalkohol, Pentanol, Hexanol und deren Mischungen besteht, ausgewählt. Als sehr geeignet haben sich Ethanol und/oder Isopropanol erwiesen.

Als Glykol werden vorzugsweise Butylglykol, Propylglykol, Ethylenglykol oder deren Mischungen davon verwendet.

Die plättchenförmigen, kupferhaltigen Metallpigmente werden in dem organischen Lösungsmittel unter optionaler Zugabe von Wasser dispergiert. Zu dieser Suspension wird entweder Säure oder Base als Katalysator zugegeben.

Vorzugsweise wird die Dispersion erwärmt. Das zur Hydrolyse erforderliche Wasser kann bereits in dem organischen Lösemittel enthalten sein oder zu einem späteren Zeitpunkt zugegeben werden.

Bei der Säure kann es sich um organische und/oder anorganische Säure handeln. Die organische Säure wird vorzugsweise aus der Gruppe, die aus Ameisensäure, Essigsäure, Propionsäure, Oxalsäure, Malonsäure, Maleinsäure, Bernsteinsäure, Anhydriden der genannten Säuren und Mischungen davon besteht, ausgewählt. Vorzugsweise werden Ameisensäure, Essigsäure, Oxalsäure oder deren Mischungen verwendet.

Die anorganische Säure kann aus der Gruppe ausgewählt werden, die aus Salpetersäure, Schwefelsäure, Phosphorsäure, Salzsäure, Borsäure, Flusssäure und deren Mischungen besteht, ausgewählt werden. Vorzugsweise werden Salpetersäure und/oder Flusssäure verwendet.

Gemäß einer bevorzugten Variante handelt es sich bei dem basischen Katalysator um ein Amin. Hierbei kann es sich um primäre, sekundäre oder tertiäre Amine handeln.

Gemäß einer bevorzugten Ausführungsform wird das Amin aus der Gruppe, die aus Dimethylethanolamin (DMEA), Monoethanolamin (MEA), Diethanolamin (DEA), Triethanolamin (TEA), Ethylendiamin (EDA), t-Butylamin, Monomethylamin, Dimethylamin, Trimethylamin, Monoethylamin, Diethylamin, Triethylamin, Diisopropylethylamin, Pyrridin, Pyrridinderivate, Anilin, Anilinderivate, Cholin, Cholinderivate, Harnstoff, Harnstoffderivat, Hydrazinderivate und Mischungen davon besteht, ausgewählt.

Als sehr geeignet haben sich als basischer aminischer Katalysator Ethylendiamin, Monoethylamin, Diethylamin, Monomethylamin, Dimethylamin, Triethylamin oder Mischungen davon erwiesen.

Selbstverständlich können auch anorganische Basen, wie Ammoniak, Hydrazin, Natriumhydroxid, Kaliumhydroxid, Ammoniumhydroxid, Ammoniumcarbonat, Ammoniumhydrogencarbonat, Natriumcarbonat, Natriumhydrogencarbonat, Kaliumcarbonat, Natriumhydrogencarbonat oder Mischungen davon verwendet werden. Als sehr geeignet haben sich Ammoniak und/oder Hydrazin erwiesen.

Gemäß einer äußerst bevorzugten Ausführungsform wird als Metallalkoxid Tetraalkoxysilan verwendet. Als Tetraalkoxysilan werden vorzugsweise Tetramethoxysilan, Tetraethoxysilan, Tetraisopropoxysilan oder Kondensate davon oder deren Mischungen davon verwendet. Als sehr geeignet haben sich Tetraethoxysilan und/oder Oligomere des Tetraethoxysilans erwiesen.

Vorzugsweise werden nach Aufbringen der Metalloxidschicht die mit Metalloxid beschichteten plättchenförmigen kupferhaltigen Metallpigmente abgetrennt und die chemisch nichtreaktive Kunststoffschicht aufgebracht. Die chemisch nichtreaktive Kunststoffschicht kann durch Polymerisation geeigneter Monomere aufgebaut werden. Die Monomere können dabei Funktionalitäten aufweisen, die aus der Gruppe, die aus Amino-, Hydroxy-, Thiol-, Epoxy-, Acrylat-, Methacrylat-, Vinyl-, Allyl-, Alkenyl-, Alkinyl-, Carboxy-, Carboxylanhydrid-, Isocyanat-, Cyanat-, Ureido-, Carbamat-, Estergruppen und Mischungen davon besteht, ausgewählt werden.

Als Edukte der Kunststoffschicht sind als Monomere oder reaktive Oligomere oder Polymere insbesondere vernetzende, d.h. mehrfunktionelle (Meth)acrylate geeignet. Beispiele für solche Verbindungen sind:
Allylmethacrylat, Bisphenol-A-dimethacrylat, 1,3-Butandioldimethacrylat, 1,4-Butandioldimethacrylat, Ethylenglykoldimethacrylat, 1,6-Hexandioldiacrylat, 1,6-Hexandioldimethacrylat, Diethylenglykoldimethacrylat, Diurethandimethacrylat, Dipropylenglykoldiacrylat, 1,12-Dodecandioldimethacrylat, Ethylenglykoldimethacrylat, Methacrylsäureanhydrid, N,N-Methylen-bis-methacrylamid, Neopentylglykoldimethacrylat, Polyethylenglykoldimethacrylat, Polyethylenglykol-200-diacrylat, Polyethylenglykol-400-diacrylat, Polyethylenglykol-400-dimethacrylat, Tetraethylenglykoldiacrylat, Tetraethylenglykoldimethacrylat, Tricyclodecandimethanoldiacrylat, Tripropylenglykoldiacrylat, Triethylenglykoldimethacrylat, Pentaerythritoltriacrylat, Trimethylohpropantriacrylat, Trimethylolpropan-trimethacrylat, Tris-(2-hydroxyethyl)isocyanurattriacrylat, Penta¬erythritoltetraacrylat, Dipentaerythritolpentaacrylat oder Mischungen davon.

Bei bestimmten Ausführungsformen sind tri- und höherfunktionelle (Meth)acrylate, insbesondere trifunktionelle (Meth)acrylate bevorzugt. Der Begriff "(Meth)acrylat" im Sinne der vorliegenden Erfindung umfasst Methacrylate und Acrylate.

Das Aushärten oder Auspolymerisieren von Vinyl- und/oder (Meth)acrylat-funktionellen Monomeren beim Erzeugen der chemisch nichtreaktiven Kunststoffschicht kann bei bevorzugten Ausführungsformen thermisch erfolgen.

Bei weiteren bevorzugten Ausführungsformen erfolgt das Aushärten oder Auspolymerisieren durch radikalische Polymerisation unter Verwendung von Polymerisationsstartern, vorzugsweise von Radikalinitiatoren. Dabei handelt es sich um handelsübliche in der Regel organische oder anorganische Peroxide oder Diazoniumverbindungen. Beispiele solcher Verbindungen sind: Acetyl-cyclohexan-sulfonylperoxid, Bis(2,4-dichlorbenzoyl)peroxid, Diisononanylperoxid, Dioctanoylperoxid, Diacetyl- und Dibenzoylperoxid; Peroxidicarbonate (z.B. Diisopropyl-peroxydicarbonat, Di-n-butylperoxydicarbonat, Di-2-ethylhexyl-peroxydi-carbonat, Dicyclohexyl-peroxydicarbonat), Alkylperester (z.B. Cumylperneodecanoat, t-Butyl-perneodecanoat, t-Amyl-perpivalat, t-Butyl-per-2-ethylhexanoat, t-Butylperiso-butyrat, t-Butylperbenzoat), Dialkylperoxide (z.B. Dicumylperoxid, t-Butylcumyl-peroxid, 2,5-Dimethylhexan-2,5-di-t-butylperoxid, Di(t-butylperoxyisopropyl)benzol, Di-t-butyl-peroxid, oder 2,5-Dimethylhexin-3-2,5-di-t-butylperoxid), Perketale (z.B. 1,1'-Bis-(t-butylperoxy)-3,3,5-trimethylcyclohexanonperoxid, Methylisobutylketon-peroxid, Methylethylketonperoxid, Acetylacetonperoxid), Alkylhydroperoxide (z.B. Pinanhydroperoxid, Cumolhydroperoxid, 2,5-Dimethylhexan-2,5-dihydroperoxid oder t-Butylhydroperoxid), Azoverbindungen (z.B. 4-4'-Azo-bis(4-cyanvaleriansäure), 1,1'-Azo-bis(cyclohexan-carbonsäurenitril), 1,1'-Azo-bis(isobutyrosäureamidin)dihydro-chlorid, 2,2,-Azo-bis(iso-butyronitril), Dimethyl 2,2'-azobis(2-methylpropionat) oder Persulfate wie Natriumperoxodisulfat und Kaliumperoxodisulfat. Bevorzugt ist 2,2'-Azo-bis(isobutyronitril) und Dimethyl 2,2'-azobis(2-methylpropionate). Diese Verbindungen sind kommerziell erhältlich bei Aldrich Chemie, D-89552, Steinheim oder Wako Chemicals GmbH, Fuggerstraße 12, 41468 Neuss.

Bei den Edukten der Kunststoffschicht, beispielsweise reaktiven Oligomeren und/oder Polymeren, kann es sich auch um reaktive Polymere, die aus der Gruppe, die aus Polyacrylaten, Poly(meth)acrylaten, Polyethern, Polyestern, Polyaminen, Polyamiden, Polyolen, Polyurethanen, Polyolefinen und Mischungen davon besteht, ausgewählt werden.

Die mit Metalloxid beschichteten plättchenförmigen kupferhaltigen Metallpigmente, werden gemäß einer Variante der Erfindung in einem, vorzugsweise organischen, Lösemittel dispergiert und die Suspension auf Reaktionstemperatur gebracht. Dann werden die Edukte der Kunststoffschicht, beispielsweise in Form von organischen Monomeren und/oder reaktiven Oligo-/Polymeren, sowie gegebenenfalls Polymerisationsinitiatoren zugegeben, beispielsweise durch Zutropfen, wodurch die chemisch nichtreaktive Kunststoffschicht (organische Polymerschicht) auf den metalloxidbeschichteten kupferhaltigen Pigmenten gebildet wird. Vorzugsweise wird die Dispersion während des Aufbringens der Kunststoffschicht gerührt oder bewegt.

Selbstverständlich kann die chemisch nichtreaktive Kunststoffschicht auch durch Aufsprühen der Edukte der Kunststoffschicht, beispielsweise der organischen Monomere und/oder reaktiven organischen Oligomere und/oder reaktiven organischen Polymeren, sowie optional von Polymerisationsinitiatoren in einer Wirbelschicht, in der die metalloxidbeschichteten kupferhaltigen Metallpigmente verwirbelt werden, aufgebracht werden.

Gemäß einer bevorzugten Variante der Erfindung erfolgt die Beschichtung in einer Flüssigphase.

Gemäß einer weiteren Variante der Erfindung erfolgt die Aufbringung der chemisch nichtreaktiven Kunststoffschicht in dem gleichen Lösungsmittel, in dem die Metalloxidschicht aufgebracht wurde. Diese Verfahrensvariante ist im Unterschied zu der oben beschriebenen zweistufigen Verfahrensvariante einstufig.

Nach Aufbringung der chemisch nichtreaktiven Kunststoffschicht werden diese aus der Suspension vorzugsweise abfiltriert.

Gemäß einer weiteren Variante der Erfindung erfolgt die Aufbringung der chemisch nichtreaktiven Kunststoffschicht auf der Metalloxidschicht in Form einer thermischen Polymerisation. Es wurde beobachtet, dass bei dieser thermischen Polymerisation, die ohne Zugabe eines Initiators durchgeführt wird, glatte Oberflächen resultieren.

Gemäß einer weiteren Variante der Erfindung erfolgt die Aufbringung der Schichten in umgekehrter Reihenfolge, d.h. es wird zunächst die chemisch nichtreaktive Kunststoffschicht aufgebracht und anschließend die Metalloxidschicht.

Die erfindungsgemäßen plättchenförmigen kupferhaltigen Metallpigmente werden vorzugsweise pelletiert, granuliert, stranggranuliert, stranggepresst, brikettiert, tablettiert und liegen mithin in einer im wesentlichen staubarmen, vorzugsweise staubfreien, kompaktierten Form vor. In diesen Darreichungsformen können die erfindungsgemäßen kupferhaltigen Metallpigmente leicht gehandhabt und in Beschichtungsmittel, wie beispielsweise Lacke, Farben, Druckfarben, Pulverlacken, Kunststoffe, Kosmetika, etc. einfach eingearbeitet werden.

Gemäß einer bevorzugten Variante werden die erfindungsgemäßen plättchenförmigen kupferhaltigen Metallpigmente in Pulverlack eingearbeitet.

Gemäß einer bevorzugten Variante der Erfindung ist die chemisch nichtreaktive Kunststoffschicht der plättchenförmigen kupferhaltigen Metallpigmente, kompatibel mit dem Bindemittel oder Bindemittelsystem des Pulverlacks.

Die Erfindung wird nachfolgend anhand von Beispielen näher erläutert, ohne darauf beschränkt zu sein.

### Beispiele

### Beispiel 1

*Beispiel 1a: Metalloxidschicht*

Es wurden 250 g Reichbleichgold G900 (Eckart GmbH, Hartenstein, Deutschland) in 500 g Ethanol dispergiert. Nach Erhitzen auf 50 °C wurden 26 g Tetraethoxysilan (TEOS) zugegeben. Anschließend wurden 80 ml einer 3 %-igen Ammoniak-Lösung über 3 h zudosiert. Danach wurde eine weitere Stunde gerührt, die Reaktionsmischung abfiltriert und das Produkt als Paste erhalten.

*Beispiel 1b: Kunststoffschicht (Initiatorvariante)*

Es wurden 139 g der in Beispiel 1a gewonnenen Paste (metalloxidverkapseltes Reichbleichgold G 900) (entspricht 100 g Metallpigment) in 486 g Ethanol dispergiert, so dass eine 16 Gew.-%-ige Dispersion entstand. Anschließend wurde 1 g Methacryloxypropyltrimethoxysilan (MEMO) zugegeben und für 30 min bei 25 °C und 1 h bei 75 °C gerührt. Danach wurden 250 ml einer Lösung von 1,5 g Dimethyl 2,2'-azobis(2-methylpropionat) (Handelsname V 601; erhältlich bei WAKO Chemicals GmbH, Fuggerstraße 12, 41468 Neuss), 4,75 g Methacryloxypropyltrimethoxysilan (MEMO) und 17,5 g Trimethylolpropantrimethacrylat (TMPTMA) in Testbenzin über 1 h zur Reaktionsmischung zudosiert. Nachfolgend wurde weitere 15 h bei 75 °C gerührt, die Reaktionsmischung abfiltriert und als Paste isoliert.

*Beispiel 1c.- Kunststoffschicht (thermische Variante)*

Es wurden 139 g der in Beispiel 1a gewonnenen Paste (metalloxidverkapseltes Reichbleichgold G900) (entspricht 100 g Metallpigment) in 486 g Testbenzin D100 dispergiert, so dass eine 16 Gew.-%-ige Dispersion entstand. Anschließend wurde 1 g Methacryloxypropyltrimethoxysilan (MEMO) zugegeben und für 15 min bei 25 °C und 3 h bei 50 °C gerührt. Danach wurden bei 130 °C über 2 h 240 ml einer Lösung von 5,8 g Methacryloxypropyltrimethoxysilan (MEMO) und 17,5 g Trimethylolpropantrimethacrylat (TMPTMA) in Testbenzin D100 zugegeben. Nach 15 h Rühren bei 130 °C wurde die Reaktionsmischung abfiltriert, mit 800 ml Testbenzin D100 gewaschen und als Paste isoliert.

### Beispiel 2

Es wurden 100 g Bleichgold Dorolan L 900 (Eckart GmbH, Hartenstein, Deutschland) in 525 g Testbenzin D100 dispergiert, so dass eine 16 Gew.-%-ige Dispersion entstand. Anschließend wurde 1 g Methacryloxypropyltrimethoxysilan (MEMO) zugegeben und für 1 h bei 25 °C und für 3 h bei 50 °C gerührt. Danach wurden bei 130 °C 240 ml einer Lösung von 4,6 g Methacryloxypropyltrimethoxysilan (MEMO) und 13,75 g Trimethylolpropantrimethacrylat (TMPTMA) in Testbenzin D100 über 2 h zudosiert. Nachfolgend wurde weitere 15 h bei 130 °C gerührt, die Reaktionsmischung abfiltriert und als Paste isoliert.

### Beispiel 3

Es wurden 100 g Feuerrot Dorolan L900 (Eckart GmbH, Hartenstein, Deutschland) in 525 g Testbenzin D100 dispergiert, so dass eine 16 Gew.-%-ige Dispersion entstand. Anschließend wurde 1 g Methacryloxypropyltrimethoxysilan (MEMO) zugegeben und für 1 h bei 25 °C und 3 h bei 50 °C gerührt. Danach wurden bei 130 °C 240 ml einer Lösung von 19,35 g Trimethylolpropantrimethacrylat (TMPTMA) in Testbenzin D100 über 2 h zudosiert. Nachfolgend wurde weitere 15 h bei 130 °C gerührt, die Reaktionsmischung abfiltriert und als Paste isoliert.

**Beispiel 4**

Analog zu Beispiel 3 wurde die gleiche Beschichtung mit Kupfer Dorolan L900 durchgeführt.

**Beispiel 5**

Es wurden 100 g Kupfer Dorolan L900 (Eckart GmbH, Hartenstein, Deutschland) in 525 g Testbenzin D100 dispergiert, so dass eine 16 Gew.-%ige Dispersion entstand. Anschließend wurde 1 g Methacryloxypropyltrimethoxysilan (MEMO) zugegeben und für 1 h bei 25 °C und 3 h bei 50 °C gerührt. Danach wurden bei 130 °C 240 ml einer Lösung von 18,35 g Trimethylolpropantrimethacrylat (TMPTMA) in Testbenzin D100 über 3 h zudosiert. Nach dem Ende der Dosierung wurde weitere 15 h bei 130 °C gerührt, die Reaktionsmischung abfiltriert und als Paste isoliert.

**Beispiel 6**

Es wurden 100 g Feuerrot Dorolan L 900 (Eckart GmbH, Hartenstein, Deutschland) in 525 g Testbenzin D100 dispergiert, so dass eine 16 Gew.-%ige Dispersion entstand. Anschließend wurde 1 g Methacryloxypropyltrimethoxysilan (MEMO) zugegeben und für 1 h bei 25 °C und 3 h bei 50 °C gerührt. Danach wurden bei 130 °C 240 ml einer Lösung von 18,35 g Trimethylolpropantrimethacrylat (TMPTMA) in Testbenzin D100 über 3 h zudosiert. Nach dem Ende der Dosierung wurde weitere 15 h bei 130 °C gerührt, die Reaktionsmischung abfiltriert und als Paste isoliert.

**Beispiel 7**

Es wurden 100 g Feuerrot Dorolan L 900 (Eckart GmbH, Hartenstein, Deutschland) in 525 g Testbenzin dispergiert, so dass eine 16 Gew.-%ige Dispersion entstand. Anschließend wurde 1 g Methacryloxypropyltrimethoxysilan (MEMO) zugegeben und für 1 h bei 25 °C und 3 h bei 50 °C gerührt. Danach wurden bei 130 °C 240 ml einer Lösung von 12,85 g Trimethylolpropantrimethacrylat (TMPTMA) in Testbenzin D00 über 3 h zudosiert. Nach dem Ende der Dosierung wurde weitere 15 h bei 130 °C gerührt, die Reaktionsmischung abfiltriert und als Paste isoliert.

**Beispiel 8**

*Beispiel 8a: Kunststoffschicht*

Es wurden 200 g Reichbleichgold RBIG G900 (Eckart GmbH, Hartenstein, Deutschland) in 1050 g Ethanol dispergiert, so dass eine 16 Gew.-%ige Dispersion entstand. Anschließend wurden 1,3 g Methacryloxypropyltrimethoxysilan (MEMO) und 4 g Trimethylolpropantrimethacrylat (TMPTMA) zugegeben und für 1 h bei 25 °C und 3 h bei 50 °C gerührt. Im Anschluss wurden bei 75 °C 100 ml einer Lösung von 42 g Trimethylolpropantrimethacrylat (TMPTMA) und 1 g Dimethyl 2,2'-azobis(2-methylpropionat) (Handelsname V 601; erhältlich bei WAKO Chemicals GmbH, Fuggerstraße 12, 41468 Neuss) in Ethanol über 3 h zudosiert. Nachfolgend wurde 15 h bei 75 °C gerührt, die Reaktionsmischung abfiltriert und als Paste isoliert.

*Beispiel 8b: Metalloxidschicht*

Es wurden 104 g der in Beispiel 8 gewonnenen Paste (entspricht 70 g Pigment) in 316 g Ethanol dispergiert, so dass ein Feststoffgehalt von 16,7 Gew.-% resultiert. Danach wurden 23,3 g Tetraethoxysilan (TEOS) zugegeben und die Reaktionsmischung wurde auf 75 °C erhitzt. Anschließend wurden bei 75 °C 100 ml einer Lösung von 2 g Ethylendiamin (EDA) und 20 g Wasser in Ethanol über 3 h zugegeben, Nachfolgend wurde 15 h bei 75 °C gerührt, die Reaktionsmischung abfiltriert und das Produkt als Paste erhalten.

**Beispiel 9**

*Beispiel 9a: Kunststoffschicht*

Es wurden 200 g Reichbleichgold G900 (Eckart GmbH, Hartenstein, Deutschland) in 1050 g Ethanol dispergiert, so dass eine 16 Gew.-%ige Dispersion entstand. Anschließend wurden 1,3 g Methacryloxypropyltrimethoxysilan (MEMO) zugegeben und für 1 h bei 25 °C und 3 h bei 75 °C gerührt. Nachfolgend wurden bei 75 °C 100 ml einer Lösung von 28 g Trimethylolpropantrimethacrylat (TMPTMA) und 0,9 g Dimethyl-2,2'-azobis(2-methylpropionat) (Handelsname V 601; erhältlich bei WAKO Chemicals GmbH, Fuggerstraße 12, 41468 Neuss) in Ethanol über 3 h zudosiert. Nachfolgend 15 h bei 75 °C gerührt, die Reaktionsmischung abfiltriert und als Paste isoliert.

*Beispiel 9b: Metalloxidschicht*

Es wurden 103 g der in Beispiel 9a gewonnenen Paste (entspricht 70 g Pigment) in 333 g Ethanol dispergiert, so dass ein Feststoffgehalt von 16 Gew.-% resultierte. Danach wurden bei 75 °C 7,56 g Tetraethoxysilan (TEOS) zugegeben. Anschließend wurden bei 75 °C 100 ml einer Lösung von 0,65 g Ethylendiamin (EDA) und 21 g Wasser in Ethanol zugegeben. Nachfolgend wurde 15 h bei 75 °C gerührt, die Reaktionsmischung abfiltriert und das Produkt als Paste erhalten.

**Beispiel 10**

Analog zu den unter Beispiel 8 und/oder 9 beschriebenen Bedingungen wurden verschiedene beschichtete plättchenförmige kupferhaltige Metallpigmente hergestellt.

Tabelle 1: Eduktmengen der Beispiele 10-1 bis 10-11

| | Durchführung analog | MEMO | TMPTMA 1. Zugabe | TMPTMA 2. Zugabe | TEOS |
|---|---|---|---|---|---|
| VB 10-1 | 8a,9b | 1,3 g | 4,0 g | 8 g | 8,1 g |
| VB 10-2 | 8a,9b | 1,3 g | 4,0 g | 8 g | 13,1 g |
| VB 10-3 | 8a,9b | 1,3 g | 4,0 g | 8 g | 18,8 g |
| Beispiel 10-4 | 8a,9b | 1,3 g | 4,0 g | 24 g | 3,8 g |
| Beispiel 10-5 | 9a,9b | 1,3 g | 4,0 g | 24 g | 8,0 g |
| Beispiel 10-6 | 8a,9b | 1,3 g | 4,0 g | 42 g | 2,5 g |
| Beispiel 10-7 | 8a,9b | 1,3 g | 4,0 g | 42 g | 7,5 g |
| Beispiel 10-8 | 8a,9b | 1,3 g | 4,0 g | 42 g | 23,3 g |
| VB 10-9 | 8a | 1,3 g | 4,0 g | 87 g | - |
| VB 10-10 | 8a,9b | 1,3 g | 4,0 g | 60 g | 2 g |
| VB 10-11 | 8a,9b | 1,3 g | 4,0 g | 6 g | 9,3 g |
| VB: Vergleichsbeispiel | | | | | |

**Beispiel 11**

Analog zu den unter den Beispielen 1a und 5 beschriebenen Bedingungen wurden verschiedene beschichtete plättchenförmige kupferhaltige Metallpigmente hergestellt. Als Ausgangsmaterial diente Reichbleichgold G900.

Tabelle 2: Eduktmengen der Beispiele 11-1 bis 11-12

| | Durchführung analog | TEOS | MEMO | TMPTMA |
|---|---|---|---|---|
| Beispiel 11-1 | 1a, 5 | 17,5 g | 1 g | 16,8 g |
| Beispiel 11-2 | 1a, 5 | 21,8 g | 1 g | 16,8 g |
| Beispiel 11-3 | 1a, 5 | 26,0 g | 1 g | 16,8 g |
| Beispiel 11-4 | 1a, 5 | 32,8 g | 1 g | 16,8 g |
| Beispiel 11-5 | 1a, 5 | 62,0 g | 1 g | 16,8 g |
| Beispiel 11-6 | 1a, 5 | 32,8 g | 1 g | 29 g |
| Beispiel 11-7 | 1a, 5 | 62,0 g | 1 g | 29 g |
| Beispiel 11-8 | 1a, 5 | 77,5 g | 1 g | 29 g |
| VB 11-9 | 1a, 5 | 32,8 g | 1 g | 4 g |
| VB 11-10 | 1a, 5 | 62 g | 1 g | 11 g |
| VB 11-11 | 1a, 5 | 5 g | 1 g | 16,8 g |

(fortgesetzt)

|  | Durchführung analog | TEOS | MEMO | TMPTMA |
|---|---|---|---|---|
| VB 11-12 | 1a, 5 | 5 g | 1 g | 29 g |
| VB: Vergleichsbeispiel | | | | |

**Beispiel 12: Anwendungsbeispiel Pulverlack und Chemikalientest**

Die erhaltenen Pasten wurden unter Vakuum mit leichtem Inertgasstrom bei 100 °C getrocknet und anschließend mit 71 $\mu$m Maschenweite gesiebt. Das jeweilige Metalleffektpigment wurde zusammen mit dem Pulverlack AL 96 sowie mit 0,2 % Aeroxide Alu C (Fa. Evonik) mittels ThermoMix für 4 Minuten auf Stufe 4 eingearbeitet. Die Pigmentierungshöhe betrug 5,0 Gew.-%, da bei höherer Pigmentierung ein verifizierbares Applikationsverhalten erreichbar ist. Demzufolge wurde der Pulverlack zu 95,0 Gew.-% eingewogen. Die Gesamtmenge an Pulverlack im Mischer betrug 300 g plus 0,6 g Aeroxid Alu C. Der ThermoMix ist ein handelsüblicher Küchenmixer (Fa. Vorwerk). Bei dem Zusatz Aeroxid Alu C handelt es sich um $Al_2O_3$-Partikel, welches in diesem Anwendungsfall die Funktion eines Rieselmittels übernimmt. Die Pulverlacke wurden mit der OptiSelect (Fa. ITWGema) in einer handelsüblichen Pulverkabine appliziert. Zur Beurteilung der Applikationseigenschaften wurde für 20 Sekunden gemäß den in Tabelle 1 angegebenen Parametern in die Pulverkabine gesprüht, anschließend die Beschichtung des Substrats durchgeführt und dann die Anhaftungen an den Elektroden und am Prallteller vergleichend beurteilt. Dieses Verfahren lässt einen Schluss auf das Langzeitverhalten der Pigmente während der praxisgerechten Lackierung zu.
Weiterhin wurde anhand der eingebrannten Pulverlackierung das Spritzbild bewertet. Das Augenmerk wurde vor allem auf den Verlauf, also der Glätte der Oberflächenstruktur, sowie auf schwarze, mikroskopisch kleine Fehlstellen, sogenannte Black Spots geworfen. Als Black Spots werden Bereiche auf der Pulverlackoberfläche bezeichnet, die durch eine inhomogen Verteilung der Metalleffektpigmente hervorgerufen werden. Da diese Erscheinungen im makroskopischen Bereich liegen ist zur Beurteilung der Erscheinung ein lacktechnisch geschulter Blick vonnöten. Bevorzugt sind insbesondere sehr glatte Strukturen mit sehr glattem Verlauf ohne Erscheinungen von Black Spots.
Das Applikationsverhalten, das Vorhandensein von Black Spots und die Struktur bzw. der Verlauf der Pulverlacke wurden visuell beurteilt.

*Chemikalientest*

Das beschichtete Prüfblech wurde in eine waagrechte Lage gebracht. Es wurden 5 Tropfen 10 %iger HCl mit den Einwirkzeiten 180, 150, 120, 90, und 60 min aufgebracht. Weiterhin wurden 5 Tropfen 1 M NaOH mit den Einwirkzeiten 180, 120, 60, 30 und 15 min aufgebracht.
Danach wurden die Tropfen mit Wasser entfernt und die ehemals bedeckten Flächen mit den unbedeckten Flächen optisch verglichen. Hierbei wurde eine Bewertungsskala von 0-3 (für jeden einzelnen Punkt) herangezogen (0 = kein Angriff, 3 maximaler Abbau von Pigmenten). Die ermittelten Punke wurden anschließend summiert.

Tabelle 3: Chemikalientest Pulverlackbeschichtung

| Probe | SiO$_2$-Gehalt in Gew.-% | Kunststoffgehalt in Gew.-% | Gew.verhältnis SiO$_2$ zu Kunststoff | Chemikalientest |
|---|---|---|---|---|
| VB: Reichbleichgold G900 | - | - | - | 18 |
| VB: Beispiel 1a | 3,1 | - | - | 18 |
| Beispiel 1b | 3,1 | 17,6 | 1 : 5,7 | 0 |
| Beispiel 1c | 3,1 | 22,3 | 1 : 7,2 | 0 |
| VB: Bleichgold Dorolan L900 | 3,8 | - | - | 11 Pkt |
| Beispiel 2, Bleichgold Dorolan L900 + Kunststoffschicht | 3,8 | 22,5 | 1 : 5,9 | 0 Pkt |
| VB: Kupfer Dorolan L 900 | 3,1 | - | - | 11 pkt |
| Beispiel 5: Kupfer Dorolan L 900 + Kunststoffschicht | 3,1 | 19,1 | 1 : 6,2 | 0 Pkt |
| VB: Feuerrot Dorolan L 900 | 3,7 | - | - | 20 pkt |

(fortgesetzt)

| Probe | SiO$_2$-Gehalt in Gew.-% | Kunststoffgehalt in Gew.-% | Gew.verhältnis SiO$_2$ zu Kunststoff | Chemikalientest |
|---|---|---|---|---|
| Beispiel 6: Feuerrot Dorolan L 900 + Kunststoffschicht | 3,7 | 19 | 1 : 5,1 | 0 Pkt |
| Beispiel 7: Feuerrot Dorolan L 900 + Kunststoffschicht | 3,7 | 13,6 | 1 : 3,7 | 0 Pkt |
| VB: Reichbleichgold RBIG G900 | - | - | - | 18 |
| VB 8a: Reichbleichgold RBIG G900 + Kunststoffschicht | - | 23,6 | - | 3 |
| Beispiel 8b: Reichbleichgold RBIG G900 + Kunststoffschicht + SiO$_2$ | 9,8 | 23,6 | 1 : 2,4 | 0 |
| VB: Reichbleichgold G900 | - | - |  | 18 |
| VB 9a: Reichbleichgold G900 + Kunststoffschicht | - | 14,7 |  | 8 |
| Beispiel 9b: Reichbleichgold G900 + Kunststoffschicht + SiO$_2$ | 3,3 | 14,7 | 1 : 4,5 | 0 |
| VB 10-1 | 3,3 | 5,7 | 1 : 1,7 | 14 |
| VB 10-2 | 4,6 | 5,7 | 1 : 1,2 | 12 |
| VB 10-3 | 6,0 | 5,7 | 1 : 1,0 | 13 |
| Beispiel 10-4 | 1,7* | 13 | 1 : 7,6 | 0 |
| Beispiel 10-5 | 3,3 | 14,7 | 1 : 4,5 | 0 |
| Beispiel 10-6 | 1,2* | 23,6 | 1 : 19,7 | 0 |
| Beispiel 10-7 | 3,0 | 23,6 | 1 : 7,9 | 0 |
| Beispiel 10-8 | 9,8 | 23,6 | 1 : 2,4 | 0 |
| VB 10-9 | - | 46 | - | 0 |
| VB 10-10 | 0,7* | 30,2 | 1 : 43 | 0 |
| VB 10-11 | 3,7 | 5 | 1 : 1,4 | 4 |
| Beispiel 11-1 | 2,0 | 16,2 | 1: 8,1 | 0 |
| Beispiel 11-2 | 2,5 | 15,7 | 1 : 6,3 | 0 |
| Beispiel 11-3 | 3,0 | 16,7 | 1 : 5,6 | 0 |
| Beispiel 11-4 | 3,6 | 16,6 | 1 : 4,6 | 0 |
| Beispiel 11-5 | 6,7 | 16,2 | 1 :2,4 | 0 |
| Beispiel 11-6 | 3,6 | 27,3 | 1 :7,6 | 0 |
| Beispiel 11-7 | 6,6 | 26,7 | 1 :4,0 | 0 |
| Beispiel 11-8 | 8,5 | 28,2 | 1 :3,3 | 0 |
| VB 11-9 | 3,6 | 4,6 | 1 :1,3 | 5 |
| VB 11-10 | 7,2 | 11 | 1 :1,5 | 2 |
| VB 11-11 | 0,8 | 16,7 | 1 :20,9 | 4 |
| VB: Vergleichsbeispiel<br>n.b. = nicht bestimmt<br>* theor. SiO$_2$-Gehalt<br>Gew.-%: jeweils bezogen auf das unbeschichtete kupferhaltige Metallpigment | | | | |

**Beispiel 13: Anwendungsbeispiel Coil Coating und Chemikalientest**

Die in den vorgenannten Versuchen erhaltenen Pasten wurden direkt im Coil Coating-Verfahren eingesetzt. Es wurden 8,0 g Aluminiumpaste und 8,0 g Solvesso 150 mit einem Spatel gut dispergiert bis die Mischung stippenfrei war. Anschließend wurden 84,0 g PE-Lack 42-00001 zugeben und gerührt, bevor mit 5,0 g Solvesso 150 verdünnt wurde. Anschließend wurde 3 Minuten mit dem Zahnkranzrührer bei 500 U/min gerührt. Die Viskosität betrug 100"± 10 im DIN4 - Becher

Dieser Lackansatz wurde auf ein Alcan - Aluminiumblech DIN A4 (Nr. 11) mit einer Spiralrakel abgezogen. Das Blech wurde sofort für 55 sec. in einen 280° heißen Ofen überführt. Danach wurde das Blech im Wasserbad (RT) abgeschreckt. Nach frühestens 24 h wurde dann der Chemikalientest durchgeführt.

Das beschichtete Prüfblech wurde in eine waagrechte Lage gebracht. Es wurde je ein Tropfen Salzsäure (HCl) 5 %-ig und ein Tropfen Natronlauge (NaOH) 5 %-ig auf das Blech aufgebracht. Die Tropfengröße sollte 20 bis 25 mm im Durchmesser betragen. Anschließend wurden die Tropfen mit einem Uhrglas abgedeckt und für 48 h stehen gelassen. Danach wurden die Tropfen mit Wasser entfernt und die ehemals bedeckten Flächen mit den unbedeckten Flächen optisch verglichen. Hierbei wurde eine Bewertungsskala von 0-3 herangezogen (0 = kein Angriff, 3 maximaler Abbau von Pigmenten).

Tabelle 4: Chemikalientest Coil Coating-Beschichtung

| | $SiO_2$-Gehalt in Gew.-% | Kunststoffgehalt in Gew.-% | Gewichtsverhältnis $SiO_2$ zu Kunststoff | Chemikalientest |
|---|---|---|---|---|
| VB: Reichbleichgold G900 | - | - | - | 25 |
| VB: 1a | 3,1 | - | - | n.b. |
| Beispiel 1b | 3,1 | 17,6 | 1 : 5,7 | 22 |
| Beispiel 1c | 3,1 | 22,3 | 1 : 7,2 | 3 |
| VB: Vergleichsbeispiel Gew.-%: jeweils bezogen auf das unbeschichtete kupferhaltige Metallpigment | | | | |

**Beispiel 14: Anwendungsbeispiel Pulverlack und Oxidationstest**

Von verschiedenen Beispielen/Vergleichsbeispielen wurden Pulverlackbleche angefertigt und für 12 und gegebenenfalls auch für 60 min im Ofen bei 200°C vernetzt. Es zeigte sich, dass lediglich im Falle der Beispiele 8b, 9b, 10-5, 10-7 und 10-8 keine Farbveränderung infolge einer Oxidation der Metalleffektpigmente auftrat.

Tabelle 5: Oxidationstest Pulverlackbeschichtungen

| Probe | Kunststoffgehalt in Gew.-% | SiO$_2$-Gehalt in Gew.-% | Gew.-Verhältnis SiO$_2$ zu Kunststoff | Oxidation | Farbton nach 12 min | Farbton nach 60 min |
|---|---|---|---|---|---|---|
| Ausgangsfarbe der Metallpigmente: reichbleichgold | | | | | | |
| VB: Reichbleichgold RBIG G900 | - | - | - | ja | dunkel | n.b. |
| VB 8a: Reichbleichgold RBIG G900 + Kunststoffschicht | 23,6 | - | - | ja | orangegold | n.b. |
| Beispiel 8b: Reichbleichgold RBIG G900 + Kunststoffschicht + SiO$_2$ | 23,6 | 9,8 | 1 : 2,4 | nein | reichbleichgold | n.b. |
| VB: Reichbleichgold G900 | - | - | | ja | dunkel | n.b. |
| VB 9a: Reichbleichgold G900 + Kunststoffschicht | 14,7 | - | | ja | orangegold | n.b. |
| Beispiel 9b: Reichbleichgold G900 + Kunststoffschicht + SiO$_2$ | 14,7 | 3,3 | 1 : 4,5 | nein | reichbleichgold | n.b. |
| Ausgangsfarbe der Metallpigmente: bleichgold | | | | | | |
| VB: 8a | - | 23,6 | - | ja | orange-gold | dunkel-gold |
| Beispiel: 10-5 | 14,7 | 3,3 | 1 : 4,5 | nein | bleichgold | n.b. |
| Beispiel: 10-7 | 23,6 | 3,0 | 1 : 7,9 | nein | bleichgold | n.b. |
| Beispiel: 10-8 | 23,6 | 9,8 | 1 : 2,4 | nein | bleichgold | bleichgold |
| VB: 10-9 | 46,0 | - | - | ja | orange-gold | Dunkel-gold |
| VB: 10-10 | 30,2 | 0,7* | 1 : 43 | ja | orange-gold | Dunkel-gold |
| VB: 10-11 | 5 | 3,7 | 1 : 1,4 | ja | etw. dunkler | n.b. |

VB: Vergleichsbeispiel n.b.: nicht bestimmt

* theor. SiO$_2$ Gehalt

Gew.-%: jeweils bezogen auf das unbeschichtete kupferhaltige Metallpigment

Weiterhin wurden Versuche durchgeführt, bei denen der Zustand der Oxidation quantitativ mittels Farbmessung bestimmt wurde. Hierbei wurde der Pulverlack auf Bleche aufgebracht und für 10 min im Ofen bei 200°C vernetzt. Von den erhaltenen Blechen wurde der Helligkeitswert (L-Wert) mittels eines Minolta Spectrophotometer CM-508i bestimmt.

Ferner wurde bei verschiedenen der vorgenannten Pulverlacke der Farbunterschied (ΔL) infolge einer um 60 min längeren Behandlung bei 200 °C bestimmt. Die erfindungsgemäßen Beispiele zeigten hierbei eine Helligkeitsabweichung von weniger als 3 Einheiten, während die Vergleichsbeispiele eine Helligkeitsabweichung von mehr als 8 Einheiten und somit eine starke Oxidation zeigten.

Tabelle 6: Oxidationstest Pulverlackbeschichtungen Restimmunn mittels Farbmessung

| Probe | Kunststoffgehalt in Gew.-% | $SiO_2$-Gehalt in Gew.-% | Gew.-Verhältnis $SiO_2$ zu Kunststoff | Oxidation | L-Wert | ΔL |
|---|---|---|---|---|---|---|
| Beispiel 11-1 | 2,0 | 16,2 | 1 : 8,1 | nein | 62,7 | -2,4 |
| Beispiel 11-2 | 2,5 | 15,7 | 1 : 6,3 | nein | 62,6 | -2,6 |
| Beispiel 11-3 | 3,0 | 16,7 | 1 : 5,6 | nein | 63,7 | n.b. |
| Beispiel 11-4 | 3,6 | 16,6 | 1 : 4,6 | nein | 64,1 | -2,5 |
| Beispiel 11-5 | 6,7 | 16,2 | 1 :2,4 | nein | 64,4 | n.b. |
| Beispiel 11-6 | 3,6 | 27,3 | 1 :7,6 | nein | 63,3 | n.b. |
| Beispiel 11-7 | 6,6 | 26,7 | 1 :4,0 | nein | 65,3 | n.b. |
| Beispiel 11-8 | 8,5 | 28,2 | 1 :3,3 | nein | 64,2 | n.b. |
| VB 11-11 | 0,8 | 16,7 | 1 :20,9 | ja | 61,4 | -8,1 |
| VB 11-12 | 0,7 | 27,9 | 1 :39,9 | ja | 61,9 | -8,4 |
| VB: Vergleichsbeispiel<br>n.b.: nicht bestimmt<br>Gew.-%: jeweils bezogen auf das unbeschichtete kupferhaltige Metallpigment | | | | | | |

**Beispiel 15: Anwendungsbeispiel - Nagellack**

Die Metalleffektpigmente wurden mit einer Pigmentierung von 4 Gew.-% in der Base 359 (von International Lacquers) mit einem Pinsel eingerührt und anschließend in handelsübliche Nagellackfläschchen überführt. Danach wurden die Nagellackgläschen a) bei RT und b) in einem Ofen mit einer Temperatur von 40 °C für 6 Monate gelagert. Während der Lagerzeit wurde optisch beurteilt, ob eine Grünfärbung eingetreten ist. Außerdem wurde getestet, ob sich die Pigmente, welche sich am Boden abgesetzt haben, wieder aufschütteln, bzw. redispergieren ließen. Trat entweder Grünfärbung auf, oder ließ sich das Pigment nicht mehr aufschütteln bzw. redispergieren, galt der Lagertest als nicht bestanden.

Tabelle 7: Lagerstabilitätstest Nagellack

| | Kunststoffgehalt in Gew.-% | $SiO_2$-Gehalt in Gew.-% | Gew.-Verhältnis $SiO_2$ zu Kunststoff | Lagerstabilität bei RT | Lagerstabilität bei 40 °C |
|---|---|---|---|---|---|
| VB: Bleichgold Dorolan L900 | - | 3,8 | - | 10 Tage | 3 Tage |

(fortgesetzt)

|  | Kunststoffgehalt in Gew.-% | SiO$_2$-Gehalt in Gew.-% | Gew.-Verhältnis SiO$_2$ zu Kunststoff | Lagerstabilität bei RT | Lagerstabilität bei 40 °C |
|---|---|---|---|---|---|
| Beispiel 2: Bleichgold Dorolan L900 + Kunststoffschicht | 22,5 | 3,8 | 1 : 5,9 | > 6 Monate | > 6 Monate |
| VB: Feurrot Dorolan L900 | - | 3,7 | - | 7 Tage | 6 Tage |
| Beispiel 3: Feuerrot Dorolan L900 + Kunststoffschicht | 19,0 | 3,7 | 1 : 5,1 | > 6 Monate | > 6 Monate |
| VB: Kupfer Dorolan L900 | - | 3,1 | - | 6 Tage | 6 Tage |
| Beispiel 4: Kupfer Dorolan L900 + Kunststoffschicht | 18,9 | 3,1 | 1 : 6,1 | > 6 Monate | > 6 Monate |
| VB: Vergleichsbeispiel Gew.-%: jeweils bezogen auf das unbeschichtete kupferhaltige Metallpigment | | | | | |

Es zeigte sich, dass die SiO$_2$-Beschichtung alleine nicht in der Lage ist eine Lagerzeit von > 6 Monaten zu gewährleisten. Erst durch die zusätzliche Kunststoffschicht, konnte eine Lagerstabilität von > 6 Monaten erreicht werden.

**Beispiel 16: Anwendungsbeispiel - Polymer**

Verschiedene kupferhaltige Metalleffektpigmente wurden in Mischung mit thermoplastischem Polypropylen (PP) (R 771 - 10; Fa. DOW, Deutschland, Wesseling) im Spritzgussverfahren zu Platten (Fläche 42 x 60 mm, Dicke 2 mm) verarbeitet.

Zur Herstellung einer 1 Gew.-%igen Mischung wurde wie folgt verfahren:

495 g Polypropylen-Granulat (PP) und 4,95 g des kupferhaltigen Pigments wurden in einem Taumelmischer vermischt und nachfolgend in einem Doppelschneckenextruder (Fa. Bersdorff, Deutschland, Durchmesser 25 mm, 28UD) ohne Zugabe weiterer Additive bei einer Verarbeitungstemperatur von ca. 230 °C zu einem Granulat verarbeitet. Dieses Granulat wurde anschließend mittels Spritzgießmaschine (Arburg Allrounder 221-55-250) bei 260 °C zu den Muster-Plättchen mit den oben genannten Abmessungen verarbeitet. Die Prüfung auf Oxidation des Metallpigments erfolgte durch Vergleich des Farbtons der vorgenannten Plättchen mit Referenzplättchen, deren Verarbeitung (Herstellung Granulat und Spritzguss) bei 190 °C durchgeführt wurde. Hierzu wurde ein Bykmac der Firma Byk-Gardner eingesetzt, wobei ein ΔE<3 die Oxidationsstabilität belegte.

Tabelle 8: Anwendungsbeispiel - Oxidationstest Polymer

| Probe | Kunststoffgehalt in Gew.-% | SiO$_2$-Gehalt in Gew.-% | Gew.-Verhältnis SiO$_2$ zu Kunststoff | Oxidation |
|---|---|---|---|---|
| VB: Reichbleichgold G900 | - | - | - | ja |
| VB: Beispiel 1a | - | 3,1 | - | ja |
| Beispiel 1b | 17,6 | 3,1 | 1 : 5,7 | nein |
| VB: Reichbleichgold RBIG G900 | | | | ja |
| VB 8a: Reichbleichgold RBIG G900 + Kunststoffschicht | 23,6 | - | - | ja |

(fortgesetzt)

| Probe | Kunststoffgehalt in Gew.-% | SiO$_2$-Gehalt in Gew.-% | Gew.-Verhältnis SiO$_2$ zu Kunststoff | Oxidation |
|---|---|---|---|---|
| Beispiel 8b: Reichbleichgold RBIG G900 + Kunststoffschicht + SiO$_2$ | 23,6 | 9,8 | 1 : 2,4 | nein |
| VB: Vergleichsbeispiel Gew.-%: jeweils bezogen auf das unbeschichtete kupferhaltige Metallpigment | | | | |

**Beispiel 17: Anwendungsbeispiel - Körper Lotion Wasser-in-Silikon**

Tabelle 9: Anwendungsbeispiel - Körper Lotion Wasser-in-Silikon

| INCI Name | Produktname | Gew.-% | Hersteller/ Lieferant |
|---|---|---|---|
| *Phase A* | | | |
| Cyclopentasiloxane (and) Dimethiconol | Dow Corning 1501 | 11,20 | Dow Corning |
| Cyclopentasiloxane | Xiameter PMX-0245 Cyclosiloxane | 5,75 | Dow Corning |
| Cyclopentasiloxane (and) PEG/PPG-18/18 Dimethicone | Dow Corning 5225 C | 13,80 | Dow Corning |
| C 30-45 Alkyl Methicone | Dow Corning Cosmetic Wax AMS-C30 | 3,45 | Dow Corning |
| | kupferhaltiges Metallpigment gemäß Beispiel 2 | 1,00 | |
| *Phase B* | | | |
| Polysorbate 20 | Tween 20 | 0,60 | Croda |
| Phenoxyethanol (and) Methylparaben (and) Ethylparaben (and) Butylparaben (and) Probylparaben (and) Isobutylparaben | Uniphen P-23 | 0,35 | Induchem |
| Sodium Chloride | Natriumchlorid | 0,75 | VWR |
| Aqua | Wasser | 63,10 | |

Das kupferhaltige Metalleffektpigment kann in einem Bereich von 0,2 bis 2,5 Gew.-%, bezogen auf das Gesamtgewicht der Formulierung, eingesetzt werden. Der Ausgleich kann mit Wasser erfolgen.

Phase A wurde gemischt und auf 75°C erhitzt, Phase B nach dem Mischen auf 70°C erhitzt, anschließend wurde Phase B langsam unter Homogenisierung zu Phase A hinzugefügt. Unter Rühren wurde die Emulsion abgekühlt und in ein angemessenes Behältnis abgefüllt.

**Beispiel 18: Anwendungsbeispiel - Creme Lidschatten**

Tabelle 10: Anwendungsbeispiel - Creme Lidschatten

| INCI Name | Produktname | Gew.-% | Hersteller/ Lieferant |
|---|---|---|---|
| *Phase A* | | | |
| Castor Oil | Rizinusöl | 43,70 | Honeywell Riedel-de Haen |
| Ethylhexyl Palmitate | Cegesoft C24 | 6,00 | Cognis |
| Cocos Nucifera (Coconut) Oil | Lipovol C-76 | 7,00 | Lipo Chemicals |
| Cera Alba | Ewacera 12 | 6,00 | H. Erhard Wagner |
| Isopropyl Lanolate | Ewalan IP | 5,00 | H. Erhard Wagner |
| Persea Gratissima (Avocado) Oil and Hydrogenated Vegetable Oil | Avocado Butter | 7,00 | Impag |
| Magnesium Stearate | Magnesiumstearat | 3,00 | Sigma-Aldrich |
| Bis-Hydroxyethoxypropyl Dimethicone | Dow Corning 5562 Carbinol Fluid | 7,00 | Dow Corning |
| Dimethicone/ Vinyl Dimethicone Crosspolymer and Silica | Dow Corning 9701 Cosmetic Powder | 5,00 | Dow Corning |
| Phenoxyethanol (and) Methylparaben (and) Ethylparaben (and) Butylparaben (and) Probylparaben (and) Isobutylparaben | Uniphen P-23 | 0,30 | Induchem |
| *Phase B* | | | |
| | kupferhaltiges Metallpigment gemäß Beispiel 3 | 10,00 | |

Das Pigment kann in einem Bereich von 5 bis 22,0 Gew.-%, bezogen auf das Gesamtgewicht der Formulierung, eingesetzt werden. Der Ausgleich kann mit Castor Oil erfolgen.

Phase A wurde gemischt und auf 85 °C erhitzt, Phase B wurde anschließend unter Rühren zu Phase A hinzugegeben. Nach Abfüllen in ein entsprechendes Behältnis wird die Mischung auf Raumtemperatur abgekühlt.

**Beispiel 19: Anwendungsbeispiel - Duschgel**

Tabelle 11: Anwendungsbeispiel - Duschgel

| INCI Name | Produktname | Gew.-% | Hersteller/Lieferant |
|---|---|---|---|
| *Phase A* | | | |
| | kupferhaltiges Metallpigment gemäß Beispiel 3 | 0,50 | |
| Aqua | Wasser | 58,10 | |
| Acrylates Copolymer | Carbopol Aqua SF-1 | 5,50 | Lubrizol |

(fortgesetzt)

| | | | |
|---|---|---|---|
| *Phase B* | | | |
| Sodium Hydroxide | NaOH (10 Gew.-%) | 1,50 | |
| *Phase C* | | | |
| Sodium Laureth Sulfate | Texapon NSO | 22,00 | Cognis |
| Cocamidopropyl Betaine | Tego Betain F 50 | 6,00 | Evonik |
| PEG-7 Glyceryl Cocoate | Emanon HE | 2,00 | Kao Corp. |
| Disodium Laureth Sulfosuccinate | Sectacin 103 Spezial | 2,00 | Zschimmmer & Schwarz |
| *Phase D* | | | |
| Phenoxyethanol (and) Piroctone Olamine | Nipaguard PO 5 | 0,60 | Clariant |
| Fragrance | Water Lily OA | 0,20 | Bell Flavors and Fragrances |
| Sodium Chloride | Natriumchlorid | 1,60 | VWR |

Das Pigment kann in einem Bereich von 0,01 bis 1,0 Gew.-%, bezogen auf das Gesamtgewicht der Formulierung, eingesetzt werden. Der Ausgleich kann mit Wasser erfolgen.

Phase A wurde gemischt und gerührt. Danach wurde Phase B zugegeben und gerührt bis ein homogenes Erscheinungsbild erreicht wurde. Phase C wurde separat eingewogen, vermischt und zu Phase AB hinzugefügt. Anschließend erneut rühren lassen und Phase D einzeln zugeben.

**Beispiel 20: Anwendungsbeispiel - Gepresster Lidschatten**

Tabelle 12: Anwendungsbeispiel - Gepresster Lidschatten

| **INCI Name** | **Produktname** | **Gew.-%** | **Hersteller/ Lieferant** |
|---|---|---|---|
| *Phase A* | | | |
| Mica | Silk Mica | 17,00 | VWR |
| Boron Nitride | Softouch CCS 102 | 2,50 | Momentive |
| Zinc Stearate | Zinkstearat | 7,00 | VWR |
| Talc | Talkumpuder | 43,50 | Sigma-Aldrich |
| | kupferhaltiges Metallpigment gemäß Beispiel 3 | 20,00 | |
| *Phase B* | | | |
| Dimethicone | Xiameter PMX-200 Silicone Fluid 5cs | 5,00 | Dow Corning |
| Cyclopentasiloxane (and) Dimethicone Crosspolymer | Dow Corning 9040 Elastomer | 5,00 | Dow Corning |

Das Pigment kann in einem Bereich von 5,0 bis 40,0 Gew.-%, bezogen auf das Gesamtgewicht der Formulierung, eingesetzt werden. Der Ausgleich kann mit Talc erfolgen.

Phase A wurde für 30s bei 2500rpm in einem Hochgeschwindigkeitsmixer gemischt. Anschließend wurde Phase B zugegeben und die Mischung für 60s bei 3000rpm im gleichen Mixer gemischt. Zuletzt wird die Pulvermischung mittels einer Lidschattenpresse bei 150 bar für 30s in Form gepresst.

**Beispiel 21: Anwendungsbeispiel - Haar Mascara**

Tabelle 13: Anwendungsbeispiel - Haar Mascara

| INCI Name | Produktname | Gew.-% | Hersteller/ Lieferant |
|---|---|---|---|
| *Phase A* | | | |
| Polyquaternium-16 | Luviquat FC 905 (Luviquat Exellence) | 2,70 | BASF |
| Propylene Glycol | 1,2-Propandiol | 1,80 | VWR |
| Methylparaben | Methyl-4-hydroxybenzoat | 0,20 | Sigma-Aldrich |
| Aqua | Wasser | 64,45 | |
| *Phase B* | | | |
| Cetearyl Alcohol | Lanette O | 5,00 | Cognis |
| Dimethicone | Xiameter PMX-200 Silicone Fluid 350cs | 1,00 | Dow Corning |
| Ceteareth-25 | Cremophor A 25 | 2,00 | BASF |
| Propylparaben | Propyl-4-hydroxybenzoat | 0,10 | Sigma-Aldrich |
| *Phase C* | | | |
| Hydroxypropylcellulose | Klucel G | 0,50 | Ashland |
| Magnesium Aluminium Silicate | Veegum HV | 0,50 | R. T. Vanderbilt |
| Aqua | Wasser | 19,00 | |
| *Phase D* | | | |
| | kupferhaltiges Metallpigment gemäß Beispiel 3 | 2,50 | |
| Phenoxyethanol (and) Methylparaben (and) Butylparaben (and) Ethylparaben (and) Propylparaben (and) Isobutylparaben | Phenonip | 0,20 | Clariant |
| Fragrance | Blue Shadow ÖKO | 0,05 | Bell Flavors and Fragrances |

Das Pigment kann in einem Bereich von 1,0 bis 10,0 Gew.-%, bezogen auf das Gesamtgewicht der Formulierung, eingesetzt werden. Der Ausgleich kann mit dem Wasser aus Phase A erfolgen.

Phase A und Phase B wurden getrennt auf 80°C erhitzt, danach wurde Phase B langsam zu Phase A hinzugefügt. In einem separaten Gefäß wurden Klucel und Veegum dem Wasser von Phase C hinzugegeben. Anschließend wurde Phase AB auf 40°C abgekühlt und während des Abkühlens Phasen C und D unter Rühren hinzugefügt.

**Beispiel 22: Anwendungsbeispiel - Haargel**

Tabelle 14: Anwendungsbeispiel - Haargel

| INCI Name | Produktname | Gew.-% | Hersteller/ Lieferant |
|---|---|---|---|
| *Phase A* | | | |
| | kupferhaltiges Metallpigment gemäß Beispiel 2 | 0,10 | |
| Ammonium Acryloyldimehtyltaurate/ VP Copolymer | Aristoflex AVC | 1,40 | Clariant |
| Citric Acid | Zitronensäure | 0,10 | VWR |
| Aqua | Wasser | 55,10 | |
| *Phase B* | | | |
| PVP | Luviskol K 30 Powder | 1,50 | BASF |
| Propylene Glycol, Diazolidinyl, Urea, Methylparaben, Propylparaben | Germaben II | 0,20 | International Speciality Products |
| Triethanolamine | Triethanolamin | 1,20 | VWR |
| Wasser | Wasser | 40,40 | |

Das Pigment kann in einem Bereich von 0,01 bis 2,0 Gew.-%, bezogen auf das Gesamtgewicht der Formulierung, eingesetzt werden. Der Ausgleich kann mit Wasser erfolgen.

Das Pigment wurde mit Wasser aus Phase A verrührt, Aristoflex AVC und Citric Acid wurden unter Rühren hinzugegeben und bei einer Geschwindigkeit von 800 rpm für 15 Minuten gemischt. Phase B wurde gelöst bis eine homogene Lösung entstand, anschließend wurde Phase B zu Phase A gegeben und gemischt.

**Beispiel 23: Anwendungsbeispiel - Körperpuder**

Tabelle 15: Anwendungsbeispiel - Körperpuder

| INCI Name | Produktname | Gew.-% | Hersteller/ Lieferant |
|---|---|---|---|
| *Phase A* | | | |
| Mica | Silk Mica | 58,70 | VWR |
| Talc | Talkumpuder | 18,00 | Sigma-Aldrich |
| Boron Nitride | Softouch CCS 102 | 5,00 | Advanced Ceramics |
| Nylon-12 | Orgasol 2002 D/Nat | 8,00 | Arkema |
| Magnesium Stearate | Magnesiumstearat | 6,00 | Sigma-Aldrich |
| Methylparaben, Propylparaben | Rokonsal SSH-1 | 0,30 | ISP Biochema |
| | kupferhaltiges Metallpigment gemäß Beispiel 2 | 2,00 | |

(fortgesetzt)

| Phase B | | | |
|---|---|---|---|
| Tridecyl Stearate (and) Tridecyl Trimellitate (and) Dipentaerythrityl Hexacaprylate/Hexacaprate | Lipovol MOS-130 | 2,00 | Lipo Chemicals |

Das Pigment kann in einem Bereich von 0,2 bis 5,0 Gew.-%, bezogen auf das Gesamtgewicht der Formulierung, eingesetzt werden. Der Ausgleich kann mit Silk Mica erfolgen.

Phase A wurde gemischt, anschließend wurde Phase B zu Phase A zugegeben und anschließend in ein geeignetes Gefäß abgefüllt.

**Beispiel 24: Anwendungsbeispiel - Lipgloss**

Tabelle 16: Anwendungsbeispiel - Lipgloss

| INCI Name | Produktname | Gew.-% | Hersteller/ Lieferant |
|---|---|---|---|
| Phase A | | | |
| Hydrogenated Polyisobutene (and) Ethylene/Propylene/Styrene Copolymer (and) Butylene/Ethylene/Styrene Copolymer | Versagel ME 750 | 79,00 | Calumet Penreco |
| Simmondsia Chinensis (Jojoba) Seed Oil | Jojoba Oil - Natural/Golden | 2,00 | BioChemica |
| Caprylyl Trimethicone | Silcare Silicone 31M50 | 7,00 | Clariant |
| Stearyl Dimethicone | Silcare Silicone 41M65 | 3,20 | Clariant |
| Hydrogenated Polydecene | Nexbase 2002 | 4,00 | Jan Dekker |
| Isopropyl Myristate | Isopropylmyristat | 4,50 | VWR |
| Phase B | | | |
| | kupferhaltiges Metallpigment gemäß Beispiel 2 | 0,10 | |
| Propylparaben | Propyl-4-hydroxybenzoat | 0,20 | Sigma-Aldrich |

Das Pigment kann in einem Bereich von 0,10 bis 8,00 Gew.-%, bezogen auf das Gesamtgewicht der Formulierung, eingesetzt werden. Der Ausgleich kann mit Versagel ME 750 erfolgen.

Phase A wurde auf 85°C erhitzt, anschließend wurden die Inhaltstoffe der Phase B einzeln zu Phase A hinzugegeben, gerührt bis eine gleichmäßige Konsistenz entstand und dann in ein Lip Gloss Gefäß abgefüllt.

**Beispiel 25: Anwendungsbeispiel - Lippenkonturenstift**

Tabelle 17: Anwendungsbeispiel - Lippenkonturenstift

| INCI Name | Produktname | Gew.-% | Hersteller/ Lieferant |
|---|---|---|---|
| Phase A | | | |
| Hydrogenated Coco-Glycerides | Softisan 100 | 12,35 | Sasol Wax |

(fortgesetzt)

| INCI Name | Produktname | Gew.-% | Hersteller/ Lieferant |
|---|---|---|---|
| *Phase A* | | | |
| Candelilla Cera | Ewacera 42 | 14,00 | H. Erhard Wagner |
| Magnesium Stearate | Magnesiumstearat | 6,00 | Sigma-Aldrich |
| Stearic Acid | Kortacid 1895 | 8,50 | Akzo Nobel |
| Hydrogenated Coconut Oil | Lipex 401 | 8,00 | Aarhus Karlshamn |
| Cetyl Palmitate | Kahlwax 7157 | 7,00 | Kahl |
| Caprylic/ Capric Triglyceride | Liponate GC-K | 3,60 | Lipo Chemicals |
| Soybean Glycerides (and) Butyrospermum Parkii | Lipex L'sens | 15,00 | Aarhus Karlshamn |
| Tocopheryl Acetate | dl-alpha-Tocopherylacetat | 0,25 | Jan Dekker |
| Methylparaben; Propylparaben | Rokonsal SSH-1 | 0,30 | ISP Biochema |
| *Phase B* | | | |
| | kupferhaltiges Metallpigment gemäß Beispiel 3 | 25,00 | |

Das Pigment kann in einem Bereich von 15 bis 25 Gew.-%, bezogen auf das Gesamtgewicht der Formulierung, eingesetzt werden. Alternativ können zusätzlich zu dem Pigment weitere Farb- und/ oder Effektpigmente zugegeben werden. Die maximale Pigmentierungshöhe sollte jedoch nicht überschritten werden.

Phase A wurde auf 85°C erhitzt und anschließend die Phase B der Phase A unter Rühren hinzugefügt bis sich eine gleichförmige Masse ergab. Danach wurde die Mischung heiß in eine Stiftform eingefüllt.

**Beispiel 26: Anwendungsbeispiel - Lippenstift**

Tabelle 18: Anwendungsbeispiel - Lippenstift

| INCI Name | Produktname | Gew.-% | Hersteller/Lieferant |
|---|---|---|---|
| *Phase A* | | | |
| Carnauba Wax | Ewacera 34 | 4,50 | H. Erhard Wagner |
| Cera Alba | Ewacera 12 | 3,50 | H. Erhard Wagner |
| Candelilla Cera Extract | Ewacera 42 | 4,00 | H. Erhard Wagner |
| Microcrystalline Wax | TeCero-Wax 1030 K | 7,20 | TH.C. Tromm |
| Cetyl Palmitate | Kahlwax 7157 | 2,00 | Kahl |
| Hydrogenated Coco-Glycerides | Softisan 100 | 5,00 | Sasol Wax |
| Petrolatum | Penreco Blond | 5,80 | Calumet Penreco |
| Cetearyl Ethylhexanoate | Luvitol EHO | 10,70 | BASF |
| Tocopheryl Acetate | dl-alpha-Tocopherylacetat | 0,50 | Jan Dekker |
| Castor Oil | Rizinusöl | 46,60 | Honeywell Riedel-de Haen |

(fortgesetzt)

| Phase B | | | |
|---|---|---|---|
| | kupferhaltiges Metallpigment gemäß Beispiel 2 | 10,00 | |
| Methylparaben, Propylparaben | Rokonsal SSH-1 | 0,20 | ISP Biochema |

Das Pigment kann in einem Bereich von 0,5 bis 21,0 Gew.-%, bezogen auf das Gesamtgewicht der Formulierung, eingesetzt werden. Der Ausgleich kann mit Castor Oil erfolgen.

Phase A wurde auf 85°C erhitzt, danach wurde Phase B zu Phase A gegeben und gemischt. Anschließend wurde diese Mischung bei einer Temperatur von 75 °C in einem Lippenstiftform abgefüllt.

**Beispiel 27: Anwendungsbeispiel - Flüssig Lidstrich**

Tabelle 19: Anwendungsbeispiel - Flüssig Lidstrich

| INCI Name | Produktname | Gew.-% | Hersteller/ Lieferant |
|---|---|---|---|
| Phase A | | | |
| Aqua | Wasser | 66,70 | |
| Water/ carbon black dispersion | MBD 201 | 3,00 | Geotech |
| Acrylates Copolymer | Covacryl E14 | 10,00 | LCW |
| Magnesium Aluminium Silicate | Veegum HV | 1,00 | C. H. Erbslöh |
| Phase B | | | |
| Propylene Glycol | 1,2 Propandiol | 3,00 | VWR |
| Triethanolamine | Triethanolamin | 1,40 | VWR |
| Phase C | | | |
| Xanthan Gum | Keltrol CG-T | 0,30 | CP Kelco |
| Phase D | | | |
| | kupferhaltiges Metallpigment gemäß Beispiel 3 | 3,00 | |
| Mica | Silk Mica | 2,00 | VWR |
| Phase E | | | |
| Stearic Acid | Kortacid 1895 | 2,80 | Akzo Nobel |
| Glyceryl Stearate | Aldo MS K FG | 0,80 | Lonza |
| Oleyl Alcohol | HD-Ocenol 90/95 V | 0,50 | Cognis |
| Phenoxyethanol (and) Methylparaben (and) Ethylparaben (and) Butylparaben (and) Probylparaben (and) Isobutylparaben | Uniphen P-23 | 0,50 | Induchem |
| Phase F | | | |
| Dimethicone (and) Trisiloxane | Xiameter PMX-1184 Silicone Fluid | 5,00 | Dow Corning |

Das Pigment kann in einem Bereich von 0,5 bis 8,0 Gew.-%, bezogen auf das Gesamtgewicht der Formulierung

eingesetzt werden. Der Ausgleich kann mit Wasser erfolgen.

Veegum wurde in Phase A dispergiert und 15 Minuten gerührt, danach wurde Phase B zu Phase A hinzugefügt, anschließend Phase C zu Phase AB und erneut 10 Minuten verrührt. Anschließend wurde Phase D zu Phase ABC hinzugefügt und auf 75°C erhitzt. Nun wurde Phase E ebenfalls auf 75°C erwärmt und zu Phase ABCD hinzugefügt. Nach dem Abkühlen auf 60°C wurde Phase F zugefügt und in ein geeignetes Gefäß abgefüllt.

**Beispiel 28: Anwendungsbeispiel - Mousse**

Tabelle 20: Anwendungsbeispiel - Mousse

| INCI Name | Produktname | Gew.-% | Hersteller/ Lieferant |
|---|---|---|---|
| *Phase A* | | | |
| Cyclopentasiloxane | Xiameter PMX-0245 Cyclosiloxane | 8,60 | Dow Corning |
| Hydrogenated Polyisobutene | MC 30 | 4,00 | www.sophim.com |
| Dimethicone (and) Dimethicone Crosspolymer | Dow Corning 9041 Silicone Elastomer Blend | 37,14 | Dow Corning |
| Squalane | Squalane | 5,74 | Impag |
| Isononyl Isononanoate | Dermol 99 | 10,16 | Alzo International |
| Hydrogenated Jojoba Oil | Jojoba Butter LM | 2,15 | Desert Whale |
| Hydrogenated Jojaba Oi | Jojoba Butter HM | 1,00 | Desert Whale |
| C30-45 Alkyl Methicone (and) C30-45 Olefin | Dow Corning AMS-C30 Cosmetic Wax | 1,15 | Dow Corning |
| Stearyl Dimethicone | Dow Corning 2503 Cosmetic Wax | 0,47 | Dow Corning |
| Cyclopentasiloxane (and) Polypropylsilsesquioxane | Dow Corning 670 Fluid | 5,00 | Dow Corning |
| *Phase B* | | | |
| Dimethicone/ Vinyl Dimethicone Crosspolymer | Dow Corning 9506 Powder | 16,02 | Dow Corning |
| Silica Dimethyl Silylate | Covasilic 15 | 0,17 | LCW |
| Talc | Talkumpuder | 5,00 | Sigma-Aldrich |
| | kupferhaltiges Metallpigment gemäß Beispiel 2 | 3,00 | |
| *Phase D* | | | |
| Propylene Glycol, Diazolidinyl Urea, Methylparaben, Propylparaben | Germaben II | 0,40 | International Speciality Products |

Das Pigment kann in einem Bereich von 0,1 bis 8,0 Gew.-%, bezogen auf das Gesamtgewicht der Formulierung, eingesetzt werden. Der Ausgleich kann mit Dow Corning 9041 Elastomer erfolgen.

Phase A wurde gemischt und solange erhitzt bis alles aufgeschmolzen war. Phase B wurden separat eingewogen und mit einem Hochgeschwindigkeitsmixer für 60s bei 2400 rpm gemischt. Die Hälfte der geschmolzenen Phase A wurde zur Phase B zugegeben und wieder im Mixer bei 2400 rpm für 30s gemischt. Anschließend wurde der übrige Teil der Phase B ebenfalls zur Phase A zugegeben und wieder bei 2400 rpm für 30s gemischt. Zuletzt wird Phase C zu Phase AB gegeben und wieder bei 2400 rpm für 30s im Hochgeschwindigkeitsmixer gemischt.

**Beispiel 29: Anwendungsbeispiel - Nagellack**

Tabelle 21: Anwendungsbeispiel - Nagellack

| INCI Name | Produktname | Gew.-% | Hersteller/ Lieferant |
|---|---|---|---|
| *Phase A* | | | |
| | kupferhaltiges Metallpigment gemäß Beispiel 2 | 2,00 | |
| *Phase B* | | | |
| Butylacetat (und) Ethylacetat (und) Nitrocellulose (und) Isopropyl Alcohol | International Lacquers Nailpolish & Care Base 359 | 98,00 | International Lacquers |

Das Pigment kann in einem Bereich von 0,1 bis 10,0 Gew.-%, bezogen auf das Gesamtgewicht der Formulierung, eingesetzt werden. Der Ausgleich kann mit International Lacquers Nailpolish erfolgen.

Phase A und Phase B wurden gemischt und anschließend in ein angemessenes Behältnis abgefüllt.

**Beispiel 30: Anwendungsbeispiel - Nagellack mit "soft touch"-Effekt**

Tabelle 22: Anwendungsbeispiel - Nagellack mit "soft touch"-Effekt

| INCI Name | Produktname | Gew.-% | Hersteller/Lieferant |
|---|---|---|---|
| *Phase A* | | | |
| | kupferhaltiges Metallpigment gemäß Beispiel 2 | 2,00 | |
| | Ceraflour 913 | 5,00 | Byk Chemie |
| *Phase B* | | | |
| Butylacetat (und) Ethylacetat (und) Nitrocellulose (und) Isopropyl Alcohol | International Lacquers Nailpolish & Care Base 359 | 93,00 | International Lacquers |

Das Pigment kann in einem Bereich von 0,1 bis 10,0 Gew.-%, bezogen auf das Gesamtgewicht der Formulierung, eingesetzt werden. Der Ausgleich kann mit International Lacquers Nailpolish erfolgen.

**Beispiel 31: Anwendungsbeispiel - wässriger Nagellack**

Das Pigment kann in einem wässrigen Nagellack gemäß WO 2007/115675 A2 Beispiel 1 eingesetzt werden. Die Pigmentierungshöhe beträgt hierbei 0,1 bis 10,0 Gew.-%, beispielsweise 1,5 Gew.-%, bezogen auf das Gesamtgewicht der Formulierung.

**Beispiel 32: Anwendungsbeispiel - Flüssig Lidschatten**

Tabelle 23: Anwendungsbeispiel - Flüssig Lidschatten

| INCI Name | Produktname | Gew.-% | Hersteller/Lieferant |
|---|---|---|---|
| *Phase A* | | | |
| Wasser | Wasser | 70,10 | |
| Glycerin | Pricerine 9090 | 6,00 | Croda |
| *Phase B* | | | |
| PEG-800 | Polyglycol 35000 S | 0,60 | Clariant |
| Allantoin | Allantoin | 0,30 | 3V |
| Ammonium Acryloyldimehtyltaurate/ VP Copolymer | Aristoflex AVC | 0,80 | Clariant |
| Acrylates Copolymer | Worlee Micromer CEK 20/50 | 5,00 | Worlee |
| *Phase C* | | | |
| | kupferhaltiges Metallpigment gemäß Beispiel 2 | 10,00 | |
| Divinyldimethicone/ Dimethicone Copolymer C12-C13 Pareth-3, C12-C13 Pareth-23 | Dow Corning HMW 2220 Non-Ionic Emulsion | 6,00 | Dow Corning |
| Fragrance | Water Lily OA | 0,20 | Bell Flavors and Fragrances |
| Phenoxyethanol (und) Methylparaben (und) Butylparaben (und) Ethylparaben (und) Propylparaben (und) Isobutylparaben | Phenonip | 1,00 | Clariant |

Das Pigment kann in einem Bereich von 0,10 bis 17,00 Gew.-% eingesetzt werden. Der Ausgleich kann mit Wasser erfolgen.

Phase A wurde gerührt, anschließend wurden die Inhaltstoffe der Phase B einzeln zu Phase A hinzugegeben und gerührt bis eine gleichmäßige Konsistenz entstand. Danach wurden die Inhaltstoffe der Phase C einzeln zu Phase AB gegeben und gerührt bis wieder eine gleichmäßige Konsistenz entstand.

**Patentansprüche**

1. Plättchenförmige kupferhaltige Metallpigmente, welche einen Gehalt an elementarem Kupfer von wenigstens 50 Gew.-%, bezogen auf das Gewicht des unbeschichteten kupferhaltigen Metallpigmentes aufweisen, **dadurch gekennzeichnet, dass** die kupferhaltigen Metallpigmente wenigstens eine umhüllende Metalloxidschicht und wenigstens eine umhüllende chemisch nichtreaktive Kunststoffschicht aufweisen, wobei die Summe der Gehalte der wenigstens einen chemisch nichtreaktiven Kunststoffschicht und der wenigstens einen Metalloxidschicht in einem Bereich von 10 bis 50 Gew.-%, bezogen auf das Gewicht des unbeschichteten Metallpigments, liegt und das Gewichtsverhältnis der wenigstens einen Metalloxidschicht zur wenigstens einen chemisch nichtreaktiven Kunststoffschicht in einem Bereich von 1:2 bis 1:20 liegt.

2. Plättchenförmige kupferhaltige Metallpigmente gemäß Anspruch 1, **dadurch gekennzeichnet, dass** die wenigstens eine umhüllende Metalloxidschicht zwischen kupferhaltigem Metallpigment und der wenigstens einen chemisch nichtreaktiven Kunststoffschicht angeordnet ist.

3. Plättchenförmige kupferhaltige Metallpigmente nach einem der vorhergehenden Ansprüche,
**dadurch gekennzeichnet,**
**dass** der Gewichtsanteil der wenigstens einen umhüllenden Metalloxidschicht in einem Bereich von 0,9 bis 12 Gew.-%, bezogen auf das Gewicht des unbeschichteten kupferhaltigen Metallpigmentes, liegt.

4. Plättchenförmige kupferhaltige Metallpigmente nach einem der vorhergehenden Ansprüche,
**dadurch gekennzeichnet,**
**dass** der Gewichtsanteil der wenigstens einen umhüllenden Metalloxidschicht bei plättchenförmigen kupferhaltigen Metallpigmenten, bei denen eine chemisch nichtreaktive Kunststoffschicht die oberste Schicht der Beschichtung bildet, in einem Bereich von 0,9 bis 12 Gew.-% und bei plättchenförmigen kupferhaltigen Metallpigmenten, bei denen eine Metalloxidschicht die oberste Schicht der Beschichtung bildet, in einem Bereich von 1,0 bis 10 Gew.-% liegt.

5. Plättchenförmige kupferhaltige Metallpigmente nach einem der vorhergehenden Ansprüche,
**dadurch gekennzeichnet,**
**dass** der Gewichtsanteil der wenigstens einen umhüllenden Kunststoffschicht in einem Bereich von 8 bis 40 Gew.-%, bezogen auf das Gewicht des unbeschichteten kupferhaltigen Metallpigmentes, liegt.

6. Plättchenförmige kupferhaltige Metallpigmente nach einem der vorhergehenden Ansprüche,
**dadurch gekennzeichnet,**
**dass** die kupferhaltigen Metallpigmente aus der Gruppe, bestehend aus Kupferpigmenten, Messingpigmenten, oxidierten Kupferpigmenten, oxidierten Messingpigmenten und Mischungen davon, ausgewählt werden.

7. Plättchenförmige kupferhaltige Metallpigmente nach einem der vorhergehenden Ansprüche,
**dadurch gekennzeichnet,**
**dass** die wenigstens eine Metalloxidschicht im wesentlichen aus der Gruppe, bestehend aus Siliziumoxid, Aluminiumoxid, Boroxid, Zirkoniumoxid, Ceroxid, Eisenoxid, Titanoxid, Chromoxid, Zinnoxid, Molybdänoxid, deren Oxidhydraten, deren Hydroxiden sowie Mischungen davon, ausgewählt wird.

8. Plättchenförmige kupferhaltige Metallpigmente nach einem der vorhergehenden Ansprüche,
**dadurch gekennzeichnet,**
**dass** die wenigstens eine Kunststoffschicht im wesentlichen aus einem Kunststoff besteht, der aus der Gruppe, bestehend aus Polyacrylat, Polymethacrylat, Polyacrylamid, Polyacrylnitril, Polyvinylchlorid, Polyvinylacetat, Polyamid, Polyalken, Polydien, Polyalkin, Polyalkylenglykol, Epoxidharz, Polyester, Polyether, Polyol, Polyurethan, Polycarbonat, Polyethylenterephthalat und Mischungen davon, ausgewählt wird.

9. Plättchenförmige kupferhaltige Metallpigmente nach einem der vorhergehenden Ansprüche,
**dadurch gekennzeichnet,**
**dass** die wenigstens eine Kunststoffschicht im wesentlichen aus Polyacrylat und/oder Polymethacrylat besteht und dass die wenigstens eine Metalloxidschicht im wesentlichen aus Siliziumoxid besteht, wobei das Gewichtsverhältnis der wenigstens einen Metalloxidschicht zur wenigstens einen chemisch nichtreaktiven Kunststoffschicht in einem Bereich von 1:2,2 bis 1:17 liegt.

10. Plättchenförmige kupferhaltige Metallpigmente nach Anspruch 9,
**dadurch gekennzeichnet,**
**dass** das Gewichtsverhältnis der wenigstens einen Metalloxidschicht zur wenigstens einen chemisch nichtreaktiven Kunststoffschicht in einem Bereich von 1:2,5 bis 1:15 liegt.

11. Plättchenförmige kupferhaltige Metallpigmente nach einem der Ansprüche 9 oder 10
**dadurch gekennzeichnet,**
**dass** der Gewichtsanteil der wenigstens einen Metalloxidschicht in einem Bereich von 1,5 bis 9 Gew.-%, bezogen auf das Gewicht des unbeschichteten kupferhaltigen Metallpigmentes, liegt.

12. Plättchenförmige kupferhaltige Metallpigmente nach einem der Ansprüche 9 bis 11,
**dadurch gekennzeichnet,**
**dass** der Gewichtsanteil der chemisch nicht reaktiven Kunststoffschicht in einem Bereich von 10 bis 35 Gew.-%, bezogen auf das Gewicht des unbeschichteten kupferhaltigen Metallpigmentes, liegt.

13. Verwendung von plättchenförmigen kupferhaltigen Metallpigmenten nach einem der Ansprüche 1 bis 12 in einem Beschichtungsmittel.

14. Verwendung von plättchenförmigen kupferhaltigen Metallpigmenten nach einem der Ansprüche 1 bis 12 in einem Kosmetikum, das aus der Gruppe, die aus Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen besteht, ausgewählt wird.

15. Verfahren zur Herstellung eines plättchenförmigen kupferhaltigen Metallpigmentes nach einem der Ansprüche 1 bis 12, das die folgenden Schritte umfasst:

(1a) Beschichten von plättchenförmigen kupferhaltigen Metallpigmenten mit Metalloxid,
(1b) Beschichten der in Schritt (1a) erhaltenen metalloxidbeschichteten plättchenförmigen kupferhaltigen Metallpigmenten mit dem (den) Edukt(en) der chemisch nichtreaktiven Kunststoffschicht,
(1c) Aushärten oder Auspolymerisieren der in Schritt (1b) mit dem (den) Edukt(en) der chemisch nichtreaktiven Kunststoffschicht beschichteten kupferhaltigen Metallpigmente,
oder
(2a) Beschichten von plättchenförmigen kupferhaltigen Metallpigmenten mit dem (den) Edukt(en) der chemisch nichtreaktiven Kunststoffschicht,
(2b) Aushärten oder Auspolymerisieren der in Schritt (2a) mit dem (den) Edukt(en) der chemisch nichtreaktiven Kunststoffschicht beschichteten plättchenförmigen kupferhaltigen Metallpigmente,
(2c) Beschichten der in Schritt (2b) erhaltenen mit chemisch nichtreaktiven Kunststoffschicht beschichteten plättchenförmigen kupferhaltigen Metallpigmente mit Metalloxid.

## Claims

1. Platelet-shaped, copper-containing metal pigments having an elemental copper content of at least 50 % by weight relative to the weight of the uncoated copper-containing metal pigment,
**characterised in that**
the copper-containing metal pigments comprise at least one enveloping metal oxide layer and at least one enveloping chemically non-reactive plastic layer, the sum of the contents of the at least one chemically non-reactive plastic layer and the at least one metal oxide layer being within a range of 10 to 50 % by weight relative to the weight of the uncoated metal pigment, and the weight ratio of the at least one metal oxide layer to the at least one chemically non-reactive layer is within a range of 1:2 to 1:20.

2. Platelet-shaped, copper-containing metal pigments as claimed in claim 1,
**characterised in that**
the at least one enveloping metal oxide layer is disposed between the copper-containing metal pigment and the at least one chemically non-reactive plastic layer.

3. Platelet-shaped, copper-containing metal pigments as claimed in one of the preceding claims,
**characterised in that**
the proportion by weight of the at least one enveloping metal oxide layer is in a range of 0.9 to 12 % by weight relative to the weight of the uncoated copper-containing metal pigment.

4. Platelet-shaped, copper-containing metal pigments as claimed in one of the preceding claims,
**characterised in that**
the proportion by weight of the at least one enveloping metal oxide layer in platelet-shaped copper-containing metal pigments in which a chemically non-reactive plastic layer forms the uppermost layer of the coating is in a range of 0.9 to 12 % by weight and in platelet-shaped copper-containing metal pigments in which a metal oxide layer forms the uppermost layer of the coating is in a range of 1.0 to 10 % be weight.

5. Platelet-shaped, copper-containing metal pigments as claimed in one of the preceding claims,
**characterised in that**

the proportion by weight of the at least one enveloping plastic layer is in a range of 8 to 40 % by weight relative to the weight of the uncoated copper-containing metal pigment.

6. Platelet-shaped, copper-containing metal pigments as claimed in one of the preceding claims,
   **characterised in that**
   the copper-containing metal pigments are selected from the group comprising copper pigments, brass pigments, oxidised copper pigments, oxidised brass pigments and mixtures thereof.

7. Platelet-shaped, copper-containing metal pigments as claimed in one of the preceding claims,
   **characterised in that**
   the at least one metal oxide layer is essentially selected from the group comprising silicon oxide, aluminium oxide, boron oxide, zirconium oxide, cerium oxide, iron oxide, titanium oxide, chromium oxide, tin oxide, molybdenum oxide, oxide hydrates thereof, hydroxides thereof and mixtures thereof.

8. Platelet-shaped, copper-containing metal pigments as claimed in one of the preceding claims,
   **characterised in that**
   the at least one plastic layer essentially comprises a plastic selected from the group comprising polyacrylate, polymethacrylate, polyacrylamide, polyacrylonitrile, polyvinyl chloride, polyvinyl acetate, polyamide, polyalkene, polydiene, polyalkyne, polyalkylene glycol, epoxy resin, polyester, polyether, polyol, polyurethane, polycarbonate, polyethylene terephthalate and mixtures thereof.

9. Platelet-shaped, copper-containing metal pigments as claimed in one of the preceding claims,
   **characterised in that**
   the at least one plastic layer substantially comprises polyacrylate and/or polymethacrylate and the at least one metal oxide layer substantially comprises silicon oxide, the weight ratio of the at least one metal oxide layer to the at least one chemically non-reactive plastic layer being in a range of 1:2.2 and 1:17.

10. Platelet-shaped, copper-containing metal pigments as claimed in claim 9,
    **characterised in that**
    the weight ratio of the at least one metal oxide layer to the at least one chemically non-reactive plastic layer is in a range of 1:2.5 and 1:15.

11. Platelet-shaped, copper-containing metal pigments as claimed in one of claims 9 or 10,
    **characterised in that**
    the proportion by weight of the at least one metal oxide layer is in a range of 1.5 to 9 % by weight relative to the weight of the uncoated copper-containing metal pigment.

12. Platelet-shaped, copper-containing metal pigments as claimed in one of claims 9 to 11,
    **characterised in that**
    the proportion by weight of the chemically non-reactive plastic layer is in a range of 10 to 35 % by weight relative to the weight of the uncoated copper-containing metal pigment.

13. Use of platelet-shaped copper-containing metal pigments as claimed in one of claims 1 to 12 in a coating agent.

14. Use of platelet-shaped copper-containing metal pigments as claimed in one of claims 1 to 12 in a cosmetic selected from the group comprising body powder, face powder, pressed and loose powder, face makeup, powder cream, cream makeup, emulsion makeup, wax makeup, foundation, mousse makeup, blusher, eye makeup such as eyeshadow, mascara, eyeliner, liquid eyeliner, eyebrow pencil, lip balm, lipstick, lip gloss, lip liner, hair styling compositions such as hair spray, hair mousse, hair gel, hair wax, hair mascara, permanent or semi-permanent hair dyes, temporary hair dyes, skin care compositions such as lotions, gels, emulsions and nail polish compositions.

15. Method for producing a platelet-shaped copper-containing metal pigment as claimed in one of claims 1 to 12, comprising the following steps:

    (1a) coating platelet-shaped copper-containing metal pigments with metal oxide,
    (1b) coating the platelet-shaped copper-containing metal pigments coated with metal oxide obtained in step (1a) with the educt(s) of the chemically non-reactive plastic layer,
    (1c) curing or polymerising the copper-containing metal pigments coated with the educt(s) of the chemically non-reactive plastic layer obtained in step (1b), or

(2a) coating platelet-shaped copper-containing metal pigments with the educt(s) of the chemically non-reactive plastic layer,

(2b) curing or polymerising the platelet-shaped copper-containing metal pigments coated with the educt(s) of the chemically non-reactive plastic layer in step (2a)

(2c) coating the platelet-shaped copper-containing metal pigments coated with the chemically non-reactive plastic layer obtained in step

(2b) with metal oxide.

**Revendications**

1. Pigments métalliques lamellaires contenant du cuivre, qui présentent une teneur en cuivre élémentaire d'au moins 50 % en poids par rapport au poids du pigment métallique contenant du cuivre, non revêtu, **caractérisés en ce que** les pigments métalliques contenant du cuivre présentent au moins une couche d'oxyde métallique formant enveloppe et au moins une couche de matériau plastique chimiquement non réactif formant enveloppe, la somme des teneurs en l'au moins une couche de matériau plastique chimiquement non réactif et de l'au moins une couche d'oxyde métallique étant comprise dans la plage de 10 à 50 % en poids par rapport au poids du pigment métallique non revêtu, et le rapport en poids de l'au moins une couche d'oxyde métallique à l'au moins une couche de matériau plastique chimiquement non réactif étant compris dans la plage de 1:2 à 1:20.

2. Pigments métalliques lamellaires contenant du cuivre selon la revendication 1, **caractérisés en ce que** l'au moins une couche d'oxyde métallique formant enveloppe est disposée entre le pigment métallique contenant du cuivre et l'au moins une couche de matériau plastique chimiquement non réactif.

3. Pigments métalliques lamellaires contenant du cuivre selon l'une des revendications précédentes, **caractérisés en ce que** la proportion pondérale de l'au moins une couche d'oxyde métallique formant enveloppe est comprise dans la plage de 0,9 à 12 % en poids par rapport au poids du pigment métallique contenant du cuivre, non revêtu.

4. Pigments métalliques lamellaires contenant du cuivre selon l'une des revendications précédentes, **caractérisé en ce que** la proportion en poids de l'au moins une couche d'oxyde métallique formant enveloppe, dans le cas des pigments métalliques lamellaires contenant du cuivre dans lesquels c'est une couche de matériau plastique chimiquement non réactif qui forme la couche la plus supérieure du revêtement, est comprise dans la plage de 0,9 à 12 % en poids et, dans le cas des pigments métalliques lamellaires contenant du cuivre, dans lesquels c'est une couche d'oxyde métallique qui forme la couche la plus supérieure du revêtement, est comprise dans la plage de 1,0 à 10 % en poids.

5. Pigments métalliques lamellaires contenant du cuivre selon l'une des revendications précédentes, **caractérisés en ce que** la proportion en poids de l'au moins une couche de matériau plastique formant enveloppe est comprise dans la plage de 8 à 40 % en poids par rapport au poids du pigment métallique contenant du cuivre, non revêtu.

6. Pigments métalliques lamellaires contenant du cuivre selon l'une des revendications précédentes, **caractérisés en ce que** les pigments métalliques contenant du cuivre sont choisis dans le groupe consistant en les pigments de cuivre, les pigments de laiton, les pigments de cuivre oxydés, les pigments de laiton oxydés et les mélanges de ceux-ci.

7. Pigments métalliques lamellaires contenant du cuivre selon l'une des revendications précédentes, **caractérisés en ce que** l'au moins une couche d'oxyde métallique est pour l'essentiel choisie dans le groupe consistant en l'oxyde de silicium, l'oxyde d'aluminium, l'oxyde de bore, l'oxyde de zirconium, l'oxyde de cérium, l'oxyde de fer, l'oxyde de titane, l'oxyde de chrome, l'oxyde d'étain, l'oxyde de molybdène, les oxyhydroxydes de ceux-ci, les hydroxydes de ceux-ci, ainsi que les mélanges de ceux-ci.

8. Pigments métalliques lamellaires contenant du cuivre selon l'une des revendications précédentes, **caractérisés en ce que** l'au moins une couche de matériau plastique est pour l'essentiel constituée d'un matériau plastique qui est choisi dans le groupe consistant en les polyacrylates, les polyméthacrylates, le polyacrylamide, le polyacrylonitrile, le poly(chlorure de vinyle), le poly(acétate de vinyle), les polyamides, les polyalcènes, les polydiènes, les polyalcynes, les polyalkylèneglycols, les résines époxydes, les polyesters, les polyéthers, les polyols, le polyuréthanne, les polycarbonates, le poly(téréphtalate d'éthylène) et les mélanges de ceux-ci.

9. Pigments métalliques lamellaires contenant du cuivre selon l'une des revendications précédentes, **caractérisés en ce que** l'au moins une couche de matériau plastique est pour l'essentiel constituée de polyacrylates et/ou de polyméthacrylates, et que l'au moins une couche d'oxyde métallique est pour l'essentiel constituée d'oxyde de silicium, le rapport en poids de l'au moins une couche d'oxyde métallique à l'au moins une couche de matériau plastique chimiquement non réactif étant compris dans la plage de 1:2,2 à 1:17.

10. Pigments métalliques lamellaires contenant du cuivre selon la revendication 9, **caractérisés en ce que** le rapport en poids de l'au moins une couche d'oxyde métallique à l'au moins une couche de matériau plastique chimiquement non réactif est compris dans la plage de 1:2,5 à 1:15.

11. Pigments métalliques lamellaires contenant du cuivre selon l'une des revendications 9 ou 10, **caractérisés en ce que** la proportion en poids de l'au moins une couche d'oxyde métallique est comprise dans la plage de 1,5 à 9 % en poids par rapport au poids du pigment métallique contenant du cuivre, non revêtu.

12. Pigments métalliques lamellaires contenant du cuivre selon l'une des revendications 9 à 11, **caractérisés en ce que** la proportion en poids de la couche de matériau plastique chimiquement non réactif est comprise dans la plage de 10 à 35 % en poids par rapport au poids du pigment métallique contenant du cuivre, non revêtu.

13. Utilisation de pigments métalliques lamellaires contenant du cuivre selon l'une des revendications 1 à 12 dans une composition de revêtement.

14. Utilisation de pigments métalliques lamellaires contenant du cuivre selon l'une des revendications 1 à 12 dans un produit cosmétique qui est choisi dans le groupe consistant en les poudres pour le corps, les poudres pour le visage, les poudres compactes et libres, les produits de maquillage du visage, les poudres crèmes, les maquillages crèmes, les maquillages émulsions, les cires de maquillage, les fonds de teint, les maquillages mousses, les rouges à joues, les produits de maquillage des yeux tels que les ombres à paupières, le mascara, les contours des yeux, les contours des yeux liquides, les crayons à sourcils, les bâtons pour les soins des lèvres, les bâtons à lèvres, les brillants à lèvres, les crayons contour des lèvres, les compositions pour coiffure telles que les laques, les mousses pour cheveux, les gels pour cheveux, les cires pour cheveux, le mascara capillaire, les colorations capillaires permanentes ou semi-permanentes, les colorations capillaires provisoires, les compositions pour les soins de la peau telles que les lotions, les gels, les émulsions, ainsi que les compositions de vernis à ongles.

15. Procédé de fabrication d'un pigment métallique lamellaire contenant du cuivre selon l'une des revendications 1 à 12, qui comprend les étapes suivantes :

(1a) revêtement de pigments métalliques lamellaires contenant du cuivre, avec un oxyde métallique,
(1b) revêtement des pigments métalliques lamellaires contenant du cuivre, revêtus d'un oxyde métallique, obtenus dans l'étape (1a), avec la ou les matières premières de la couche de matériau plastique chimiquement non réactif,
(1c) durcissement ou polymérisation des pigments métalliques contenant du cuivre, revêtus dans l'étape (1b) avec la ou les matières premières de la couche de matériau plastique chimiquement non réactif, ou
(2a) revêtement des pigments métalliques lamellaires non revêtus avec la ou les matières premières de la couche de matériau plastique chimiquement non réactif,
(2b) durcissement ou polymérisation des pigments métalliques lamellaires contenant du cuivre, revêtus dans l'étape (2a) avec la ou les matières premières de la couche de matériau plastique chimiquement non réactif,
(2c) revêtement des pigments métalliques lamellaires contenant du cuivre, revêtus d'une couche de matériau plastique chimiquement non réactif, obtenus dans l'étape (2b), avec un oxyde métallique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10238090 A1
- WO 2009149834 A2
- WO 2007017195 A2
- WO 2005063897 A2
- WO 2005063897 A
- DE 19820112 A1
- DE 4030727 A1
- US 20090117281 A1
- WO 2008095697 A1
- WO 2009135784 A1
- EP 1812519 B1
- WO 2009152941 A2
- EP 15290784 B1
- EP 15290785 B1
- DE 1300266 B
- DE 1495730 A
- US 2071250 A
- US 2071251 A
- US 2130523 A
- US 2130948 A
- US 2241322 A
- US 2312966 A
- US 2512606 A
- US 3393210 A
- EP 38094 A
- EP 38582 A
- EP 39524 A
- EP 299444 A
- EP 129195 A
- EP 129196 A
- EP 302485 A
- DE 19728629 A
- EP 99532 A
- US 3055859 A
- US 4224419 A
- EP 113112 A
- EP 135130 A
- WO 2007115675 A2

**In der Beschreibung aufgeführte Nicht-Patentliteratur**

- Kunststoff-Taschenbuch. Hanser-Verlag, 1992
- Kunststoff-Handbuch. Hanser-Verlag, 1966, vol. 1 bis 11
- Römpp Chemie Lexikon, CD-ROM. Thieme Verlag, 1995
- **G. HOLDEN et al.** Thermoplastic Elastomers. Hanser Verlag, 1996